# EUROPEAN PATENT APPLICATION

(11) **EP 4 467 552 A1**
(43) Date of publication of application: **27.11.2024**
(21) Application number: 23742952.7
(22) Date of filing: 19.01.2023
(51) Int. Cl.: C07F 9/6561, A61K 31/662, A61P 35/00, A61P 31/00

(54) **PHOSPHONIC ACID COMPOUND AND PRODRUG THEREOF, AND PREPARATION METHODS FOR AND USES OF PHOSPHONIC ACID COMPOUND AND PRODRUG THEREOF**

(30) Priority: 21.01.2022 CN 202210076676
(71) Applicant: Haihe Biopharma Co., Ltd., Shanghai 201203 (CN)
(72) Inventor: GAO, Shanyun, Shanghai 201203 (CN); HOU, Yingjie, Shanghai 201203 (CN); LI, Jingjing, Shanghai 201203 (CN); ZHANG, Chaobo, Shanghai 201203 (CN); XU, Yanxiao, Shanghai 201203 (CN); TU, Wangyang, Shanghai 201203 (CN); YU, Bing, Shanghai 201203 (CN); ZHANG, Yixiang, Shanghai 201203 (CN); LI, Leping, Shanghai 201203 (CN)
(74) Representative: Kador & Partner Part mbB
(86) International application number: PCT/CN2023/073033
(87) International publication number: WO 2023/138637

(57) **Abstract**

The present invention provides a phosphonic acid compound and a prodrug thereof, andpreparation methods for and uses of the phosphonic acid compound and the prodrug thereof. Specifically, the present invention provides a compound of formula (I) or formula (I-A), wherein the compound of formula (I-A) is a prodrug form of the compound of formula (I). Upon experimental experimental verification, the phosphonic acid compound of the present invention has good ENPP1 kinase inhibition activity, and can be used as a therapeutic agent for ENPP1-related diseases, and the phosphonic acid compound has good metabolic stability *in-vivo.* In addition, the phosphonate or amide prodrug compound provided by the present invention has relatively high oral bioavailability, and can overcome the defect that a phosphonic acid original drug compound cannot be taken orally.

## Description

### TECHNICAL FIELD

The present invention belongs to the field of pharmaceutical chemistry and particularly relates to a phosphonic acid compound and a prodrug thereof, and a preparation method therefor and use thereof as an ENPP1 inhibitor.

### BACKGROUND

As the first line of the body to defend against pathogens, the human immune system plays an undeniable role in combating tumor cells. STING (Stimulator of Interferon Gene) is an interferon gene stimulator composed of 378 amino acids, which plays an important role in the immune response to tumor cells. STING can be activated by binding to cGAMP (cyclic GMP-AMP synthase), which in turn recruits TANK-binding kinase 1 (TBK1), leading to phosphorylation of interferon regulatory factor 3 (IRF3). The phosphorylated IRF3 will produce type I interferon (IFN) and other cytokines, which together with IFN trigger the immune response of human adaptive immune system to tumor cells and infectious diseases.

Ectonucleotide pyrophosphatase/phosphodi-esterase 1 (ENPP1) is one of the seven enzymes in the ENPP family and a type II transmembrane glycoprotein. Research has shown that ENPP1 has high hydrolysis ability and can decompose various ligands, including phosphodiester bonds and pyrophosphate bonds. ATP is also one of the main targets of ENPP1. ENPP1 not only hydrolyzes ATP into AMP and PPi, but also hydrolyzes cGAMP, inhibiting the amount of cGAMP in the human body and reducing STING activity. Therefore, the activity of ENPP1 inhibits anti-tumor and anti-infectious diseases immune response of the human immune system through STING, and inhibiting the activity of ENPP1 contributes to the synthesis of cGAMP and the increase of STING activity, thus improving the resistance of the human immune system to tumors and infectious diseases. In addition, ENPP1 has been confirmed to have a more prominent expression than usual in human breast tumors, which not only indicates that ENPP1 is a potential predictive marker of breast cancer, but also highlights the possibility and reliability of ENPP1 as an anticancer drug target.

In addition to the prominent expression of ENPP1 in tumors, studies have shown that the expression of ENPP1 is related to a variety of infectious diseases caused by bacteria or viruses. Therefore, ENPP1 can also be used for the treatment of infectious diseases.

Although cancer patients have multiple treatment options, there is still a need for effective and safe therapeutic agents and optimal application in combination therapy thereof.

### SUMMARY OF THE INVENTION

The present disclosure provides a compound of formula (I) or formula (I-A), a pharmaceutically acceptable salt thereof, a pharmaceutical composition thereof and a combination thereof. The inventor unexpectedly discovered that the phosphonic acid compound of formula (I) is a good ENPP1 inhibitor. The inventor also unexpectedly found that the compound of the present disclosure, especially a phosphonate compound, has good physical and chemical stability, good bioavailability. A phosphonic acid parent drug compound disclosed herein is a good ENPP1 inhibitor, and has good activity and high selectivity. However, it is relatively polar and not suitable for oral administration. A phosphonate or amide prodrug compound disclosed herein can be rapidly metabolized *in vivo* to produce the parent drug compound. Therefore, the phosphonate or amide prodrug compound or a pharmaceutical salt thereof disclosed herein is used as an effective ingredient in a wide variety of formulations such as an oral formulation and rapidly produce an active ingredient as a parent drug compound after absorbed *in vivo.* Therefore, the phosphonate or amide prodrug compound or the pharmaceutical salt thereof disclosed herein is useful as a prodrug of an active ingredient compound. By preparing the phosphonic acid active ingredient compound into the phosphonate or amide prodrug compound, the physicochemical properties of the parent drug are improved, the fat solubility, the oral availability and the bioavailability of the drug are improved, the side effect is reduced, and the safety is higher.

The present disclosure further provides a method for treating, preventing or improving ENPP1-mediated diseases or disorders, including administering an effective amount of ENPP1 inhibitors to an individual in need.

Specifically, the present disclosure provides a compound of formula (I) or (I-A): 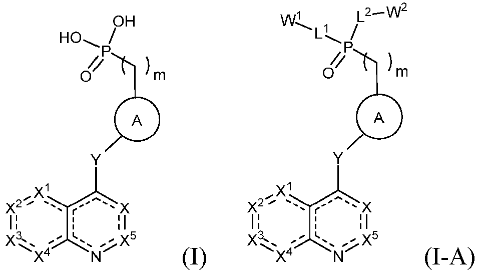 wherein, each of the variables X, X¹, X², X³, X⁴, X⁵, W¹, W², Y, L¹, L², m and rings A are defined herein, comprising a stereoisomer, a geometric isomer, a tautomer, a pharmaceutically acceptable salt, a solvate or a hydrate thereof; they can be used to treat or prevent ENPP1-mediated diseases or disorders, especially cancer or infectious diseases or disorders.

The present disclosure also provides a method for preparing the compound of the present disclosure, an intermediate for preparing the compound of the present disclosure, and a method for preparing the intermediate.

The present disclosure also provides a composition comprising at least one selected from the compound of the present disclosure, the stereoisomer, the geometric isomer, the tautomer, the pharmaceutically acceptable salt, the solvate and the hydrate thereof of the present disclosure.

The present disclosure also provides a pharmaceutical composition comprising a therapeutically effective amount of at least one selected from the compound of the present disclosure, the stereoisomer, the geometric isomer, the tautomer, the pharmaceutically acceptable salt, the solvate and the hydrate thereof of the present disclosure, as well as one or more pharmaceutically acceptable carriers, diluents or excipients.

In one embodiment, the present disclosure provides a pharmaceutical composition comprising a therapeutically effective amount of at least one selected from the compound of the present disclosure, the stereoisomer, the geometric isomer, the tautomer, the pharmaceutically acceptable salt, the solvate and the hydrate thereof of the present disclosure.

In another embodiment, the present disclosure provides a combination, especially a pharmaceutical combination, comprising a therapeutically effective amount of at least one selected from the compound of the present disclosure, the stereoisomer, the geometric isomer, the tautomer, the pharmaceutically acceptable salt, the solvate and the hydrate thereof of the present disclosure, as well as one or more other therapeutic agents.

The compound of the present disclosure may be used alone, in combination with other compounds of the present disclosure, or in combination with one or more, preferably one or two other substances simultaneously or sequentially.

The compound of the present disclosure is an ENPP1 inhibitor, which can regulate the STING activity *in vivo,* and therefore can be used to treat or prevent ENPP1-mediated diseases or obstacles, especially cancer or infectious diseases or obstacles. Therefore, the compound of the present disclosure can provide selective extracellular inhibition of ENPP1 activity to increase the extracellular level of cGAMP and activate the interferon gene stimulatory factor (STING) pathway. For example, use of the compound of the present disclosure for enhancing STING-mediated responses in a subject, and a method for using the compound of the present disclosure for regulating immune responses in a subject.

The compound of the present disclosure can be used for therapy.

The compound of the present disclosure can be used to prepare medicaments or drugs, which are used to treat or prevent ENPP1-mediated diseases or obstacles, especially cancer or infectious diseases or obstacles.

The present disclosure also involves a method for inhibiting ENPP1 receptor activity in individuals, wherein, the method includes administering a therapeutically effective amount of ENPP1 inhibitors such as the compound of the present disclosure, or the stereoisomer, the geometric isomer, the tautomer, the pharmaceutically acceptable salt, the solvate or the hydrate thereof of the present disclosure to an individual in need.

In one embodiment of the present disclosure, the present disclosure provides a method for treating or preventing ENPP1-mediated diseases or disorders, including administering an effective amount of a first therapeutic agent and an optional second therapeutic agent to patients in need, wherein the first therapeutic agent is selected at least one from the compound of the present disclosure, the stereoisomer, the geometric isomer, the tautomer, the pharmaceutically acceptable salt, the solvate and the hydrate thereof of the present disclosure, and the second therapeutic agent is one or more other therapeutic agents.

In another embodiment of the present disclosure, the present disclosure involves a method for treating or preventing ENPP1-mediated diseases or disorders, such as cancer or infectious diseases or conditions, which includes administering a therapeutically effective amount of at least one selected from the compound of the present disclosure, the stereoisomer, the geometric isomer, the tautomer, the pharmaceutically acceptable salt, the solvate and the hydrate thereof of the present disclosure to an individual.

A preferred method of the present disclosure is to treat specific types of cancer, including breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

Another preferred method of the present disclosure is to treat ENPP1-mediated infectious diseases, including herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

In addition, the present disclosure provides a product comprising a combined preparation of at least one selected from the compound of the present disclosure, the stereoisomer, the geometric isomer, the tautomer, the pharmaceutically acceptable salt, the solvate and the hydrate thereof, or the pharmaceutical composition thereof as defined above of the present disclosure, as well as one or more other active agents used simultaneously, separately, or sequentially in anti-cancer therapy.

### EMBODIMENTS

Specifically, the present disclosure provides the following embodiments:
In one aspect, the present disclosure provides a compound shown in formula (I) or formula (I-A): 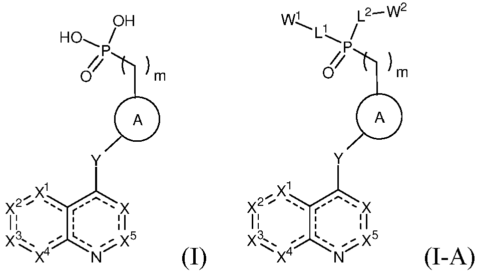
or a pharmaceutically acceptable salt, a stereoisomer, a geometric isomer, a tautomer, a pharmaceutically acceptable salt, a solvate or a hydrate thereof, wherein,
- - - -: is a single bond or a double bond;
X¹ is N, CR¹ or a bond;
X² is N, NR² or CR²;
X³ is N, NR³, -C(O) or CR³;
X⁴ is N, NR⁴, -C(O) or CR⁴;
X⁵ is N or CR⁵;
X is N or CR⁰;
provided that X and X⁵ are not N at the same time and at least one of X¹, X², X³ and X⁴ is independently N or NRⁿ, if allowed, and n = 2, 3 or 4;
R⁰, R¹, R³ and R⁵ are each independently selected from hydrogen, halogen, CN, OR^{a}, NO₂, NR^{b}R^{c}, C₁-C₆ alkyl and C₁-C₆ haloalkyl;
R² and R⁴ are each independently selected from hydrogen, halogen, CN, OR^{a}, NO₂, NR^{b}R^{c}, C₁-C₆ alkyl and C₃-C₆ cycloalkyl, wherein the alkyl and cycloalkyl are optionally substituted with halogen;
Y is O, S, NR⁶ or CR⁷R⁸, wherein R⁶, R⁷ and R⁸ are each independently selected from hydrogen, C₁-C₄ alkyl and C₃-C₆ cycloalkyl, wherein the alkyl and cycloalkyl are optionally substituted with halogen;
m is 1, 2 or 3;
ring A is selected from C6-C10 aryl and 5- to 10-membered heteroaryl, wherein ring A is optionally substituted one or more times with substituents independently selected from halogen, CN, OH, NO₂, NR^{b}R^{c}, C₁-C₄ alkyl, C₁-C₄ alkoxy, C₃-C₆ cycloalkyl, 4- to 7-membered heterocyclic alkyl, C₆-C₁₀ aryl, 5- to 10-membered heteroaryl and C₁-C₃ haloalkyl,
wherein R^{a}, R^{b} and R^{c} are each independently selected from hydrogen, C₁-C₄ alkyl and C₃-C₆ cycloalkyl, wherein the alkyl and cycloalkyl are each optionally substituted with halogen;
L¹ and L² are each independently NH or O, preferably, both L¹ and L² are O; and
   W¹ and W² are each independently selected from hydrogen, C₁-C₁₀ alkyl, C₆-C₁₀ aryl, -CH₂OC(O)R^{d}, -CH₂OC(O)OR^{d}, -CH₂C(CH₃)₂C(O)OR^{d}, -CH(CH₃)C(O)OR^{d}, and -CH₂C(CH₃)₂C(O)OR^{d}, wherein the alkyl is optionally substituted with halogen and R^{d} is C₂-C₆ alkyl.

Herein, whenever Rⁿ is mentioned, it represents R¹ for X¹, R² for X², R³ for X³ and R⁴ for X⁴.

In some specific embodiments, R⁰ and R⁵ are each independently selected from hydrogen, halogen, CN, OH, NO₂, NH₂, C₁-C₆ alkyl and C₁-C₆ alkoxy, wherein the alkyl and cycloalkyl are each optionally substituted with 0-3 halogens.

Preferably, R⁰ and R⁵ are each independently selected from hydrogen, halogen, CN, OH, NO₂, NH₂, C₁-C₄ alkyl and C₁-C₄ alkoxy, wherein the alkyl and alkoxy are each optionally substituted with 0-3 halogens;
more preferably, R⁰ and R⁵ are each independently selected from hydrogen, halogen, CN, OH, NO₂, NH₂, C₁-C₃ alkyl and C₁-C₃ alkoxy; and
most preferably, R⁰ and R⁵ are each independently selected from hydrogen, halogen, OH, CN, methyl, ethyl, propyl, methoxy and ethoxy.

In some specific embodiments, R¹ and R³ are each independently selected from hydrogen, halogen, CN, OH, NH₂, CF₃, C₁-C₄ alkyl and C₁-C₄ alkoxy;
preferably, R¹ and R³ are each independently selected from hydrogen, halogen, CN, OH, NH₂, C₁-C₃ alkyl and C₁-C₃ alkoxy; and
more preferably, R¹ and R³ are each independently selected from hydrogen, F, Cl, CN, OH, NH₂, methyl, ethyl, propyl, methoxy and ethoxy; and
most preferably, R¹ is selected from hydrogen, F, Cl; and R³ is selected from hydrogen, F, Cl, methoxy and ethoxy.

In some specific embodiments, R² and R⁴ are each independently selected from hydrogen, halogen, CN, OH, NO₂, NH₂, -NHCH₃, -N(CH₃)₂, C₁-C₄ alkyl, C₃-C₄ cycloalkyl and C₁-C₄ alkoxy, wherein the alkyl, cycloalkyl and alkoxy are each optionally substituted with 0-3 halogens.

Preferably, R² and R⁴ are each independently selected from hydrogen, halogen, CN, OH, NO₂, NH₂, C₁-C₄ alkyl, C₃-C₄ cycloalkyl, C₁-C₃ alkoxy and C₁-C₃ haloalkyl;
more preferably, R² and R⁴ are each independently selected from hydrogen, F, Cl, CN, OH, NO₂, NH₂, methyl, ethyl, propyl, isopropyl, methoxy, ethoxy, fluoromethyl, difluoromethyl, trifluoromethyl, fluoroethyl, 2,2-difluoromethyl, 1,2-difluoromethyl, 2,2,2-trifluoroethyl and cyclopropyl; and
most preferably, R² and R⁴ are each independently selected from hydrogen, F, Cl, OH, methyl and ethyl.

In some specific embodiments, R¹, R², R³ and R⁴ are each independently selected from hydrogen, halogen, CN, OH, NO₂, NH₂, C₁-C₄ alkyl and C₁-C₄ alkoxy.

Preferably, R¹ and R³ are each independently selected from hydrogen, halogen, OH and C₁-C₃ alkoxy; and R² and R⁴ are each independently selected from hydrogen, halogen, OH and C₁-C₃ alkyl.

In some specific embodiments, at least one of X¹, X², X³ and X⁴ is independently N or if allowed, in NRⁿ, n = 2, 3 or 4, for example,
X¹ is N; or X² is selected from N and NR²; or X³ is selected from N and NR³; or X⁴ is selected from N and NR⁴.

Preferably, X⁴ is N or NR⁴, or X² is N or NR²; and more preferably, X⁴ is N or X² is N.

In some specific embodiments, X⁴ is N and X³ is CR³; preferably, X⁴ is N, X³ is CR³ and X² is CR²; and further preferably, X⁴ is N, X³ is CR³, X² is CR² and X¹ is CR¹.

In some specific embodiments, X² is N and X³ is CR³; preferably, X² is N, X³ is CR³ and X¹ is CR¹; and further preferably, X² is N, X³ is CR³, X¹ is CR¹ and X⁴ is CR⁴.

In some specific embodiments, X⁴ is NR⁴, preferably, X⁴ is NR⁴, and X³ is -C(O); and more preferably, X⁴ is NR⁴, X³ is -C(O), X² is CR² and X¹ is CR¹.

In some specific embodiments, X³ is NR³, preferably, X³ is NR³, and X⁴ is -C(O); and more preferably, X³ is NR³ and X⁴ is -C(O), and X² is CR² and X¹ is CR¹.

In some specific embodiments, at least one of X¹, X², X³ and X⁴ is independently N, for example,
X¹ is N; or X² is N; or X³ is N; or X⁴ is N;
preferably, X³ is N or X⁴ is N; further preferably, X⁴ is N; and
more preferably, X³ is N and X⁴ is NR⁴; or X³ is N and X² is NR².

In some specific embodiments, only one of X¹, X², X³ and X⁴ is N, for example,
X¹ is N, X² is NR² or CR²; X³ is NR³ or CR³ and X⁴ is NR⁴ or CR⁴; or
X¹ is CR¹ or a bond, X² is N, X³ is NR³ or CR³ and X⁴ is NR⁴ or CR⁴; or
X¹ is CR¹ or a bond, X³ is N, X² is NR² or CR² and X⁴ is NR⁴ or CR⁴; or
X¹ is CR¹ or a bond; X⁴ is N, X² is NR² or CR² and X³ is NR³ or CR³.

In some specific embodiments, at least one of X¹, X², X³ and X⁴ is independently N or if allowed, in NRⁿ, n = 2, 3 or 4, for example,
X¹ is N, X² is CR², X³ is CR³ and X⁴ is CR⁴; or
X¹ is CR¹ or a bond, X² is N or NR², X³ is CR³ and X⁴ is CR⁴; or
X¹ is CR¹ or a bond, X³ is N or NR³, X² is CR² and X⁴ is CR⁴; or
X¹ is CR¹ or a bond, X⁴ is N or NR⁴, X² is CR² and X³ is CR³.

Preferably, X¹ is N, X² is CR², X³ is CR³ and X⁴ is CR⁴; or
X¹ is CR¹ or a bond, X² is N, X³ is CR³ and X⁴ is CR⁴; or
X¹ is CR¹ or a bond, X³ is N, X² is CR² and X⁴ is CR⁴; or
X¹ is CR¹ or a bond, X⁴ is N, X² is CR² and X³ is CR³.

In some specific embodiments, at most one of X and X⁵ is N, for example, X⁵ is N and X is CR⁰; or X⁵ is CR⁵ and X is N; or X⁵ is CR⁵ and X is CR⁰, for example, 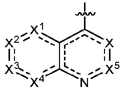 is selected from 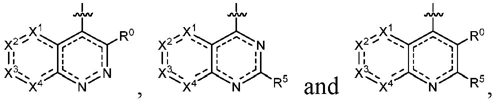 preferably, X⁵ is CR⁵ and X is CR⁰.

In some specific embodiments, at least one of X¹, X², X³ and X⁴ is independently N or if allowed, in NRⁿ, n = 2, 3 or 4; and at most one of X and X⁵ is N, for example, 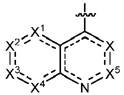 is selected from 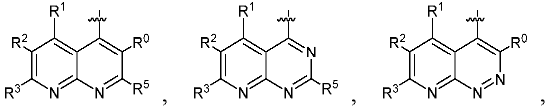 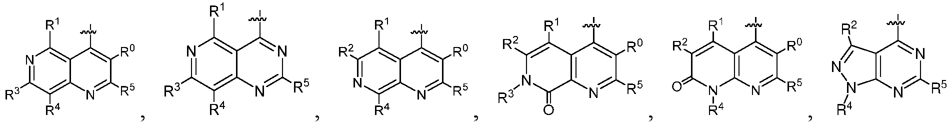 and 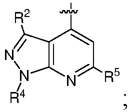 and preferably, selected from 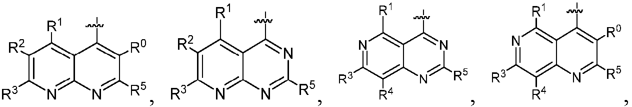 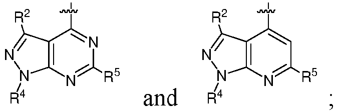 and more preferably,

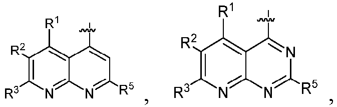

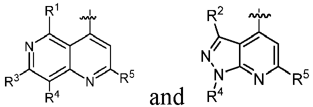

In some specific embodiments, X¹ is N or CR¹, for example, the compound shown in formula (I) or formula (I-A) is respectively a compound shown in formula (II) or formula (II-A): wherein, 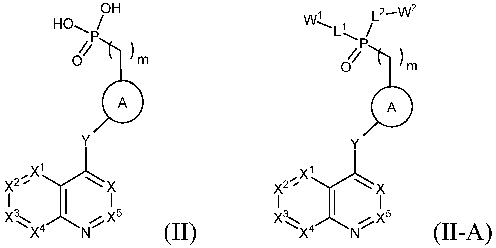
X¹ is N or CR¹; X² is N or CR²;
X³ is N, NR³, -C(O) or CR³;
X⁴ is N, NR⁴, -C(O) or CR⁴;
X⁵ is N or CR⁵; X is N or CR⁰;
provided that X and X⁵ are not N at the same time and at least one of X¹, X², X³ and X⁴ is independently N or NRⁿ, and n = 3 or 4;
Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN, OH, NO₂, NH₂, C₁-C₄ alkyl and C₁-C₄ alkoxy, wherein the alkyl is each optionally substituted with 0-3 halogens;
R¹ and R³ are each independently selected from hydrogen, halogen, CN, OH, NO₂, NH₂ and C₁-C₄ alkoxy;
R² and R⁴ are each independently selected from hydrogen, halogen, CN, OH, NO₂, NH₂ and C₁-C₄ alkyl; and
ring A, m, L¹, L², W¹ and W² are as previously defined.

In some specific embodiments, in formula (II) or formula (II-A),
R¹ and R³ are each independently selected from hydrogen, halogen and C₁-C₃ alkoxy;
R² and R⁴ are each independently selected from hydrogen, halogen, OH and C₁-C₃ alkyl; and
R⁰ and R⁵ are each independently selected from hydrogen, halogen, OH, CN, NO₂, NH₂, C₁-C₄ alkyl and C₁-C₄ alkoxy.

In some specific embodiments, in formula (II) or formula (II-A),
R¹ is hydrogen and halogen; R³ is selected from hydrogen, halogen and C₁-C₃ alkoxy;
R² is selected from hydrogen and halogen; R⁴ is selected from hydrogen, halogen and C₁-C₃ alkyl; and
R⁰ and R⁵ are each independently selected from hydrogen, halogen, CN and C₁-C₄ alkyl.

In some specific embodiments, in formula (II) or formula (II-A),
X³ is -C(O), X⁴ is NR⁴, X² is CR² and X¹ is CR¹; or
X³ is NR³ and X⁴ is -C(O); X² is CR² and X¹ is CR¹; and R³ and R⁴ are each independently selected from C₁-C₃ alkyl.

In some specific embodiments, in formula (I), X¹ is a bond, for example, the compound shown in formula (I) or formula (I-A) is respectively a compound shown in formula (III) or formula (III-A): 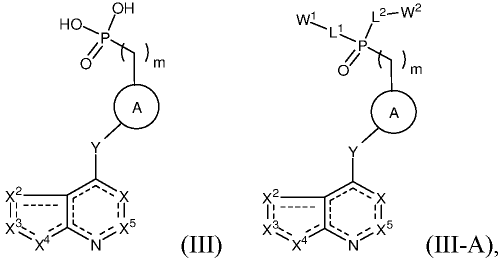
wherein - - - - : is a single bond or a double bond;
X² is N, NR² or CR²; X³ is N, NR³ or CR³; X⁴ is N, NR⁴ or CR⁴;
X⁵ is N or CR⁵; X is N or CR⁰;
provided that X and X⁵ are not N at the same time and at least one of X², X³ and X⁴ is N or NRⁿ, and n = 2, 3 or 4;
Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN, C₁-C₆ alkyl, C₁-C₄ alkoxy and C₁-C₆ haloalkyl;
R², R³ and R⁴ are each independently selected from hydrogen, halogen, CN, OH, NO₂, NH₂, C₁-C₄ alkyl and C₁-C₃ haloalkyl; and
ring A, L¹, L², m, W¹ and W² are as previously defined.

In some specific embodiments, in formula (III) or formula (III-A),
R⁰ is selected from hydrogen, halogen and C₁-C₃ alkyl; R² is selected from hydrogen, halogen and C₁-C₃ alkyl;
R³ is selected from hydrogen; R⁴ is selected from hydrogen, halogen, C₁-C₃ alkyl and C₁-C₃ haloalkyl; and
R⁵ is selected from hydrogen, halogen, CN, OH, C₁-C₄ alkoxy and C₁-C₄ alkyl.

In some specific embodiments, in formula (III) or formula (III-A),
at least one of X², X³ and X⁴ is N or NRⁿ, and n = 2, 3 or 4, for example,
X² is N or NR², or X³ is N or NR³, or X⁴ is N or NR⁴.

Preferably, X³ is N or NR³; or X⁴ is N or NR⁴.

In some specific embodiments, in formula (III) or formula (III-A), at least two of X², X³ and X⁴ are N or NRⁿ, and n = 2, 3 or 4, for example,
X³ is N and X⁴ is NR⁴, preferably, or X³ is N and X⁴ is NR⁴, and X² is CR²; or
X³ is N and X² is NR², preferably, or X³ is N and X² is NR², and X⁴ is CR⁴.

In some specific embodiments, X⁴ is N, for example, the compound shown in formula (I) or formula (I-A) is respectively a compound shown in formula (IV) or formula (IV-A): 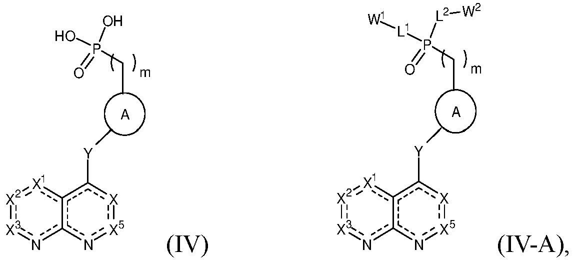
wherein - - - - : is a single bond or a double bond;
X¹ is N, CR¹ or a bond; X² is N, NR² or CR²; X³ is N, NR³ or CR³;
X⁵ is N or CR⁵; X is N or CR⁰; provided that X and X⁵ are not N at the same time;
Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN, C₁-C₆ alkyl, C₁-C₄ alkoxy and C₁-C₆ haloalkyl;
R¹ and R³ are each independently selected from hydrogen, halogen, CN, OH, NO₂, NH₂, C₁-C₄ alkyl and C₁-C₄ alkoxy;
R² is selected from hydrogen, halogen, OH and C₁-C₃ alkyl; and
ring A, L¹, L², m, W¹ and W² are as previously defined.

In some specific embodiments, X¹ is CR¹, X² is CR², X³ is CR³ and X⁴ is N, for example, the compound shown in formula (I) or formula (I-A) is respectively a compound shown in formula (V) or formula (V-A): 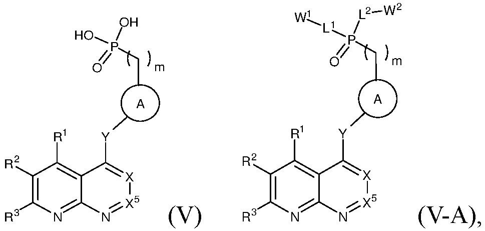
wherein X⁵ is N or CR⁵; X is N or CR⁰; and X and X⁵ are not N at the same time;
Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN and C₁-C₆ alkyl;
R¹, R² and R³ are each independently selected from hydrogen, halogen, CN, OH, NO₂, NH₂, C₁-C₄ alkyl and C₁-C₄ alkoxy; and
ring A, L¹, L², m, W¹ and W² are as previously defined.

In some specific embodiments, X¹ is CR¹, X² is N, X³ is CR³ and X⁴ is CR⁴, for example, the compound shown in formula (I) or formula (I-A) is respectively a compound shown in formula (VI) or formula (VI-A): 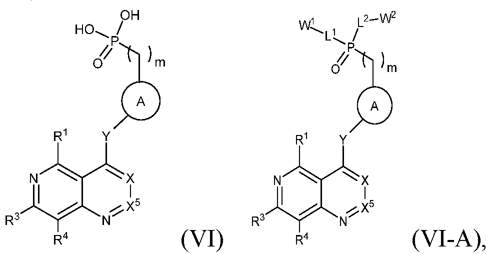
wherein X⁵ is N or CR⁵; X is N or CR⁰; and X and X⁵ are not N at the same time;
Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN and C₁-C₆ alkyl;
R¹ and R³ are each independently selected from hydrogen, halogen, CN, OH, NO₂, NH₂ and C₁-C₄ alkoxy;
R⁴ is selected from hydrogen, halogen and C₁-C₄ alkyl; and
ring A, L¹, L², m, W¹ and W² are as previously defined.

In some preferred embodiments, X¹ is CR¹, X² is CR², X³ is CR³, X⁴ is N, X⁵ is CR⁵ and X is N, for example, the compound shown in formula (I) or formula (I-A) is respectively a compound shown in formula (VII) or formula (VII-A): 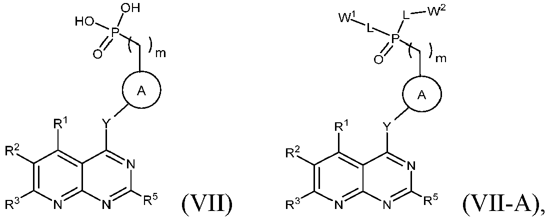
wherein Y is O, S, NR⁶ or CR⁷R⁸, and R⁶, R⁷ and R⁸ are each independently selected from hydrogen and C₁-C₄ alkyl;
R⁵ is selected from hydrogen, halogen, CN and C₁-C₆ alkyl;
R¹ and R³ are each independently selected from hydrogen, halogen, CN, OH, NO₂, NH₂ and C₁-C₄ alkoxy;
R² is selected from hydrogen, halogen and C₁-C₄ alkyl; and
ring A, L¹, L², m, W¹ and W² are as previously defined.

In some preferred embodiments, X¹ is CR¹, X² is N, X³ is CR³, X⁴ is CR⁴, X⁵ is CR⁵ and X is CR⁰, for example, the compound shown in formula (I) or formula (I-A) is respectively a compound shown in formula (VIII) or formula (VIII-A): 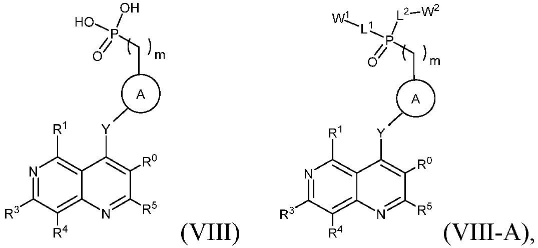
wherein Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN and C₁-C₆ alkyl;
R¹ and R³ are each independently selected from hydrogen, halogen, CN, OH, NO₂, NH₂ and C₁-C₄ alkoxy;
R² is selected from hydrogen, halogen and C₁-C₄ alkyl; and
ring A, L¹, L², m, W¹ and W² are as previously defined.

In some preferred embodiments, X¹ is CR¹, X² is CR², X³ is CR³, X⁴ is N, X⁵ is CR⁵ and X is CR⁰, for example, the compound shown in formula (I) or formula (I-A) is respectively a compound shown in formula (IX) or formula (IX-A): 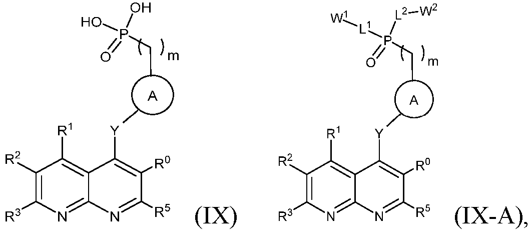
wherein Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN and C₁-C₆ alkyl;
R¹ and R³ are each independently selected from hydrogen, halogen, CN, OH, NO₂, NH₂, C₁-C₄ alkyl and C₁-C₄ alkoxy;
R² is selected from hydrogen, halogen and C₁-C₄ alkyl; and
ring A, L¹, L², m, W¹ and W² are as previously defined.

In some specific embodiments, Y is O, S, NR⁶ or CR⁷R⁸, and R⁶, R⁷ and R⁸ are each independently selected from hydrogen and C₁-C₄ alkyl; and preferably, R⁶, R⁷ and R⁸ are each independently selected from hydrogen and C₁-C₃ alkyl.

In some specific embodiments, Y is O, S, NR⁶ or CR⁷R⁸; preferably, Y is NR⁶ and R⁶ is selected from hydrogen and C₁-C₄ alkyl; and more preferably, R⁶ is selected from hydrogen and C₁-C₃ alkyl; or
preferably, Y is CR⁷R⁸, and R⁷ and R⁸ are each independently selected from hydrogen and C₁-C₄ alkyl; more preferably, R⁷ and R⁸ are each independently selected from hydrogen and C₁-C₃ alkyl; or
preferably, Y is O, S, -NH-, -N(CH₃)-, -N(ethyl)-, -N(propyl)-, -CH₂-, -CH(CH₃)- or -C(CH₃)₂-; and
more preferably, Y is O, NH, N(CH₃), CH(CH₃) or CH₂.

In some specific embodiments of the present disclosure, Y is O, S, NH or CH₂; preferably, Y is O, -NH- or -CH₂-; and more preferably, Y is O or -NH-.

In other specific embodiments of the present disclosure, Y is O.

In other specific embodiments of the present disclosure, Y is -NH-.

In other specific embodiments of the present disclosure, Y is -CH₂-.

In specific embodiments, ring A is selected from optionally substituted C₆-C₁₀ aryl and 5-to 10-membered heteroaryl; the substituent is selected from halogen, CN, OH, NO₂, NR^{b}R^{c}, C₁-C₄ alkyl, C₁-C₄ alkoxy, C₃-C₆ cycloalkyl, 4- to 7-membered heterocyclic alkyl, C₆-C₁₀ aryl, 5- to 10-membered heteroaryl and C₁-C₃ haloalkyl; R^{b} and R^{C} are each independently selected from hydrogen, C₁-C₄ alkyl and C₃-C₄ cycloalkyl, wherein the alkyl and cycloalkyl are each optionally substituted with halogen;
preferably, ring A may be optionally substituted once or more times, e.g. once or twice, independently with a substituent selected from F, Cl, CN, OH, NO₂, NH₂, C₁-C₄ alkyl, C₁-C₄ alkoxy, C₃-C₄ cycloalkyl and C₁-C₃ haloalkyl; and
further preferably, ring A may be optionally substituted once or more times, e.g. once or twice, independently with a substituent selected from F, Cl, CN, OH, NO₂, NH₂, C₁-C₃ alkyl, C₁-C₃ alkoxy and C₃-C₄ cycloalkyl.

In some specific embodiments, ring A is selected from an optionally substituted or unsubstituted pyrrole ring, furan ring, thiophene ring, pyrazole ring, thiazole ring, oxazole ring, imidazole ring, pyran ring, benzene ring, pyridine ring, pyrimidine ring, naphthalene ring, quinoline ring, isoquinoline ring, indole ring, isoindole ring, indazole ring or benzimidazole ring;
preferably, ring A is an optionally substituted or unsubstituted pyrazole ring, pyrrole ring, imidazole ring, furan ring, thiophene ring, pyran ring, benzene ring, pyridine ring, pyrimidine ring or naphthalene ring;
more preferably, ring A is an optionally substituted or unsubstituted pyrimidine ring, benzene ring, pyridine ring, imidazole ring, pyrazole ring or naphthalene ring; and
further preferably, ring A is a pyrimidine ring, benzene ring, pyridine ring, imidazole ring, pyrazole ring or naphthalene ring substituted once or twice with F, Cl, CN, OH, NO₂, NH₂, C₁-C₃ alkyl or C₁-C₃ alkoxy.

In some specific embodiments, ring A is selected from 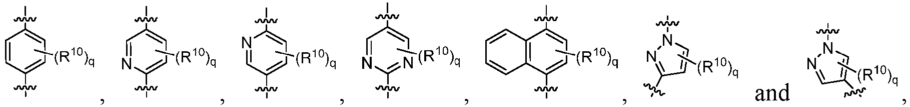
wherein each R¹⁰ may be the same or different, and independently selected from halogen, CN, OH, NO₂, NH₂, C₁-C₃ alkyl, C₁-C₃ alkoxy and C₁-C₃ haloalkyl; q is 0, 1 or 2; and
preferably, each R¹⁰ is independently selected from F, Cl, CN, C₁-C₃ alkyl and C₁-C₃ alkoxy.

In specific embodiments, m is 1, 2 or 3; and preferably, m is 1 or 2.

In specific embodiments, in formulae (I-A)-(IX-A), W¹ and W² are each independently selected from C₁-C₁₀ alkyl, C₆-C₁₀ aryl, -CH₂OC(O)R^{d}, -CH₂OC(O)OR^{d}, -CH₂C(CH₃)₂C(O)OR^{d} and -CH(CH₃)C(O)OR^{d}, wherein the alkyl is optionally substituted with halogen; R^{d} is C₂-C₆ alkyl; and preferably, R^{d} is C₂-C₄ alkyl, for example, R^{d} is ethyl, t-butyl, isobutyl or isopropyl.

Preferably, W¹ and W² are each independently selected from C₁-C₆ alkyl, C₆-C₈ aryl, -CH₂OC(O)R^{d}, -CH₂OC(O)OR^{d} and -CH(CH₃)C(O)OR^{d}, and R^{d} is C₃-C₄ alkyl, for example, R^{d} is t-butyl, isobutyl or isopropyl;
more preferably, W¹ and W² are each independently selected from phenyl, -CH₂OC(O)R^{d}, -CH₂OC(O)OR^{d} and -CH(CH₃)C(O)OR^{d}, and R^{d} is C₃-C₄ alkyl, for example, R^{d} is t-butyl, isobutyl or isopropyl;
further preferably, W¹ and W² are each independently selected from -CH₂OC(O)R^{d} and -CH₂OC(O)OR^{d};
further preferably, W¹ and W² are each independently selected from -CH₂OC(O)R^{d} and both L¹ and L² are O; and
further preferably, W¹ and W² are each independently selected from -CH₂OC(O)OR^{d} and both L¹ and L² are O.

In specific embodiments, in formulae (I-A)-(IX-A), 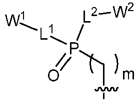 is selected from 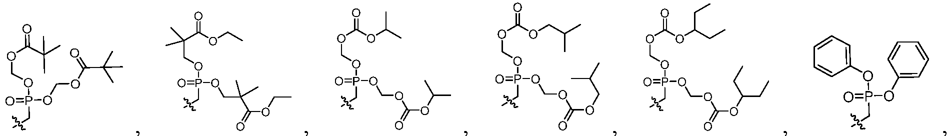 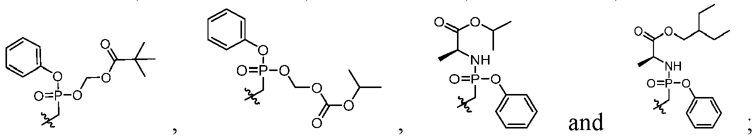 and preferably, 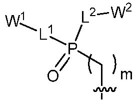 is selected from

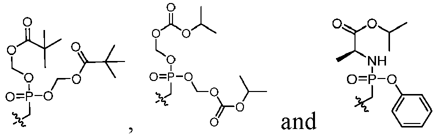

In some specific embodiments, the compound of formula (I) is selected from the following compounds:

| Compo -und No. | Structure | Compo -und No. | Structure | Compo -und No. | Structure |
|---|---|---|---|---|---|
| 1 | 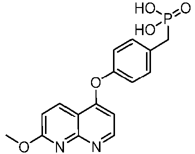 | 2 | 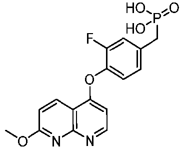 | 5 | 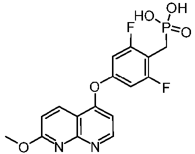 |
| 3 | 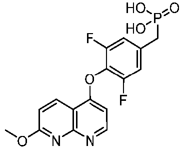 | 4 | 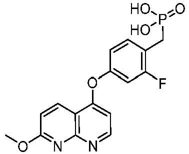 | 6 | 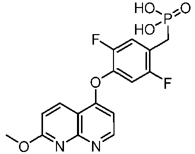 |
| 10 | 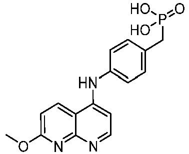 | 11 | 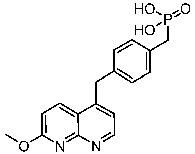 | 14 | 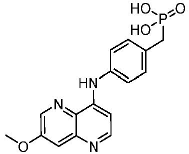 |
| 12 | 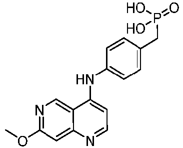 | 13 | 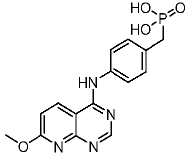 | 15 | 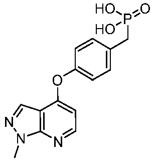 |
| 20 | 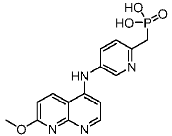 | 21 | 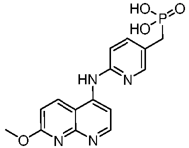 | 24 | 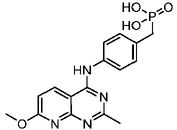 |
| 22 | 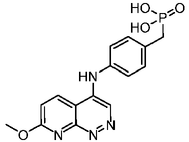 | 23 | 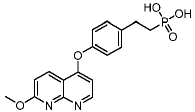 | 25 | 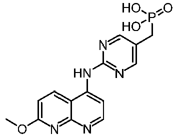 |
| 26 | 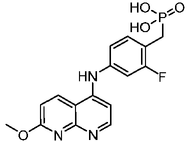 | 27 | 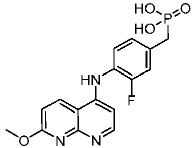 | 30 | 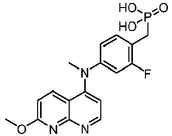 |
| 28 | 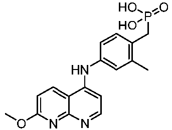 | 29 | 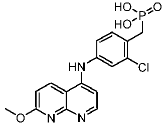 | 31 | 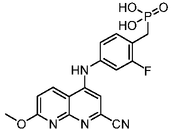 |
| 32 | 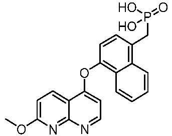 | 33 | 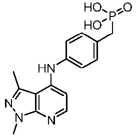 | 41 | 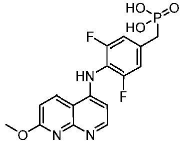 |
| 34 | 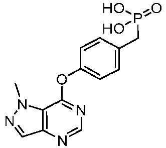 | 40 | 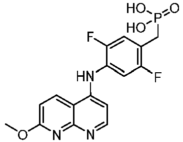 | 42 | 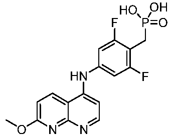 |
| 43 | 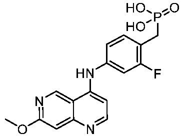 | 44 | 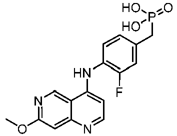 | 47 | 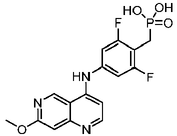 |
| 45 | 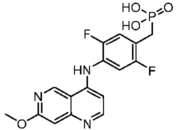 | 46 | 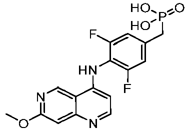 | 58 | 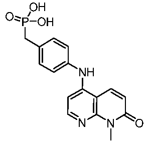 |
| 59 | 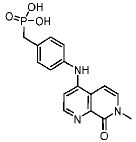 | 60 | 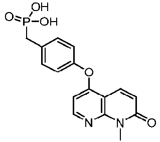 | 61 | 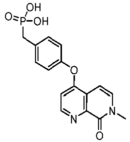 |

In other specific embodiments, the compound of formula (I-A) is selected from the following compounds:

| Compo -und No. | Structure | Compo -und No. | Structure | Compo -und No. | Structure |
|---|---|---|---|---|---|
| 7 | 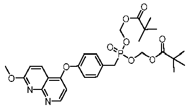 | 8 | 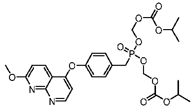 | 17 | 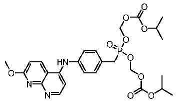 |
| 9 | 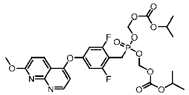 | 16 | 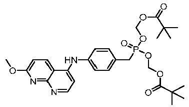 | 18 | 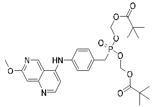 |
| 19 | 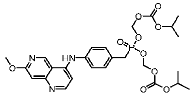 | 35 | 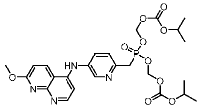 | 38 | 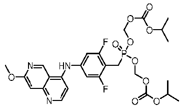 |
| 36 | 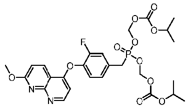 | 37 | 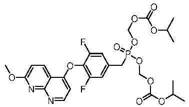 | 39 | 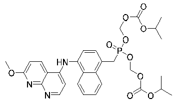 |
| 48 | 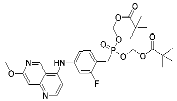 | 49 | 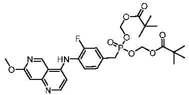 | 52 | 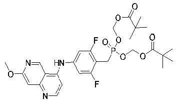 |
| 50 | 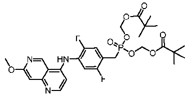 | 51 | 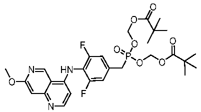 | 53 | 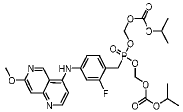 |
| 54 | 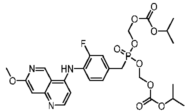 | 55 | 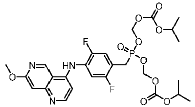 | 62 | 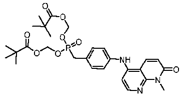 |
| 56 | 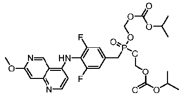 | 57 | 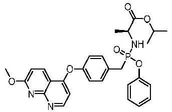 | 63 | 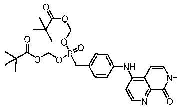 |
| 64 | 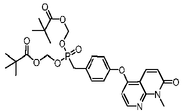 | 65 | 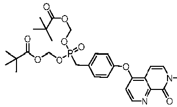 | 68 | 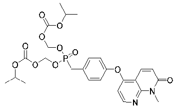 |
| 66 | 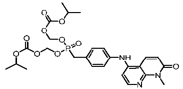 | 67 | 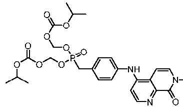 | 69 | 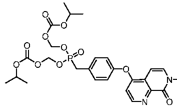 |

In another aspect, the present disclosure provides a method for preparing the compound of the present disclosure.

In specific embodiments, the compound of formula (I) of the present disclosure is prepared by the following method:
a compound of general formula A₂ is reacted with a compound of formula A₃ in the presence of cesium carbonate, Pd₂dba₃ and Xphos (when Y' in compound A₂ is a hydroxyl group) or DPBP (when Y' in compound A₂ is an amine group) to produce a compound A₄ of general formula. Then the compound A₄ of general formula is hydrolyzed under the action of TMSBr to produce the compound of formula I, as shown in the following reaction scheme: 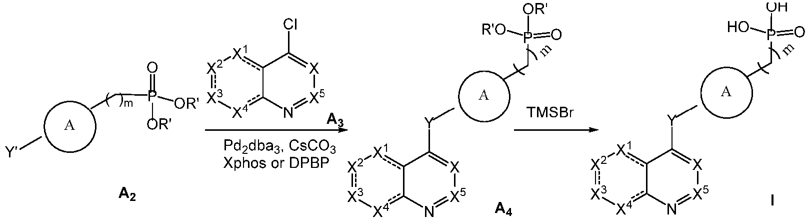
in the above reaction scheme, Y' represents hydroxyl or amino group, R' represents C₁-C₆ allkyl group, X, X¹, X², X³, X⁴, X⁵, Y, m and ring A are respectively as defined hereinbefore.

In another one aspect, the present disclosure provides an intermediate for preparing the compound of the present disclosure and a preparation method thereof.

In specific embodiments, an intermediate compound A₃ is 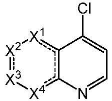 and synthesized by the following method: 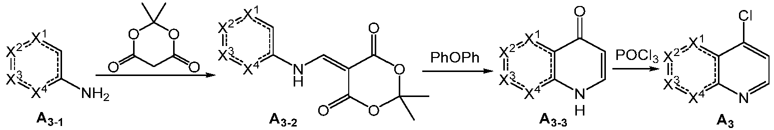
in the above reaction scheme, X¹, X², X³ and X⁴ are respectively as defined hereinbefore,
as shown in the above reaction scheme, the method comprises:
   (1) adding a compound A₃₋₁ into trimethyl orthoformate and 2,2-dimethyl-1,3-dioxan-4,6-diketone to react to obtain a compound A₃₋₂;
   (2) dissolving the compound A₃₋₂ in diphenyl ether to react to obtain a compound A₃₋₃; and
   (3) reacting the compound A₃₋₃ with phosphorus oxychloride to obtain an intermediate compound A₃.

In another one aspect, the present disclosure provides a composition comprising at least one selected from the compound of the present disclosure, the pharmaceutically acceptable salt, the stereoisomer, the geometric isomer, the tautomer, the solvate and the hydrate thereof of the present disclosure.

In an embodiment, the present disclosure provides a pharmaceutical composition comprising at least one selected from the compound of the present disclosure, the pharmaceutically acceptable salt, the stereoisomer, the geometric isomer, the tautomer, the solvate and the hydrate thereof of the present disclosure, as well as at least one pharmaceutically acceptable carrier, diluent or excipient.

In another embodiment, the present disclosure provides a pharmaceutical composition comprising a therapeutically effective amount of at least one selected from the compound of the present disclosure, the stereoisomer, the geometric isomer, the tautomer, the pharmaceutically acceptable salt, the solvate and the hydrate thereof of the present disclosure, and at least one pharmaceutically acceptable carrier, diluent or excipient. In certain embodiments of the pharmaceutical composition, the pharmaceutical composition is formulated for any suitable route of administration, for example, intravenous administration, intramuscular administration, oral administration, rectal administration, inhalation administration, nasal administration, local administration, eye administration or ear administration. In other embodiments of the pharmaceutical composition, the pharmaceutical composition is a tablet, a pill, a capsule, a liquid agent, an inhalant, a nasal spray solution agent, a suppository, a solution agent, an emulsion, an ointment, an eye drop or an ear drop.

In another one aspect, the present disclosure provides a pharmaceutical combination product comprising at least one selected from the compound of the present disclosure, the pharmaceutically acceptable salt, the stereoisomer, the geometric isomer, the tautomer, the solvate and the hydrate thereof of the present disclosure, as well as one or more other active agents.

In another one aspect, the present disclosure provides the use of a compound of formula (I) or (I-A), or a stereoisomer, a geometric isomer, a tautomer, a pharmaceutically acceptable salt, a solvate or a hydrate thereof in the preparation of a drug for preventing, treating or alleviating disorders or diseases caused by tumors or infections in a patient.

In an embodiment, the tumor or infection is caused by changes in interferon gene stimulating factor (STING).

In another embodiment, the present disclosure provides the use of a compound of formula (I) or (I-A), or a stereoisomer, a geometric isomer, a tautomer, a pharmaceutically acceptable salt, a solvate, or a hydrate thereof in the preparation of a drug for preventing, treating or alleviating diseases or obstacles mediated by ENPP1, such as cancer or infectious diseases or conditions.

In another embodiment, the present disclosure provides the compound of the present disclosure, or the stereoisomer, the geometric isomer, the tautomer, the pharmaceutically acceptable salt, the solvate or the hydrate thereof of the present disclosure, which can be used alone or optionally in combination with another compound of the present disclosure, or the stereoisomer, the geometric isomer, the tautomer, the pharmaceutically acceptable salt, the solvate or the hydrate thereof of the present disclosure, and/or at least one other therapeutic agent in therapy to treat ENPP1-mediated diseases or disorders.

In another embodiment, the present disclosure also provides the use of the compound of the present disclosure, or the stereoisomer, the geometric isomer, the tautomer, the pharmaceutically acceptable salt, the solvate or the hydrate thereof of the present disclosure in the preparation of a medicament, wherein the medicament is used alone or optionally in combination with another compound of the present disclosure, or the stereoisomer, the geometric isomer, the tautomer, the pharmaceutically acceptable salt, the solvate or the hydrate thereof of the present disclosure, and/or at least one other therapeutic agent to treat ENPP1-mediated diseases or disorders.

In another embodiment, the present disclosure provides a combined preparation of the compound of the present disclosure, or the stereoisomer, the geometric isomer, the tautomer, the pharmaceutically acceptable salt, the solvate or the hydrate thereof of the present disclosure, and one or more additional therapeutic agents for use in therapy.

In another embodiment, the present disclosure provides a combination of the compound of the present disclosure, or the stereoisomer, the geometric isomer, the tautomer, the pharmaceutically acceptable salt, the solvate or the hydrate thereof of the present disclosure, and one or more additional therapeutic agents for simultaneous or separate use in therapy.

In another embodiment, the present disclosure provides a combined preparation of the compound of the present disclosure, or the stereoisomer, the geometric isomer, the tautomer, the pharmaceutically acceptable salt, the solvate or the hydrate thereof of the present disclosure, and one or more additional therapeutic agents for simultaneous, separate or sequential use in the treatment of ENPP1-mediated diseases or disorders. The compound can be administered as a pharmaceutical composition described herein.

In another aspect, the present disclosure provides a method for treating or preventing ENPP1-mediated diseases or disorders, including administering a therapeutically effective amount of at least one selected from the compound of the present disclosure, the stereoisomer, the geometric isomer, the tautomer, the pharmaceutically acceptable salt, the solvate and the hydrate thereof of the present disclosure, or the pharmaceutical composition of the present disclosure, to an individual in need thereof.

In one embodiment, the present disclosure provides a method for treating ENPP1-mediated diseases or disorders, including administering a therapeutically effective amount of at least one selected from the compound of the present disclosure, the stereoisomer, the geometric isomer, the tautomer, or the pharmaceutically acceptable salt,the solvate or the hydrate thereof of the present disclosure alone or optionally in combination with another compound of the present disclosure, or the stereoisomer, the geometric isomer, the tautomer, the pharmaceutically acceptable salt, the solvate or the hydrate thereof of the present disclosure, and/or at least one other therapeutic agent to a patient in need of the treatment.

In another embodiment, the present disclosure provides a method for treating ENPP1-mediated diseases or disorders, including administering a first and a second therapeutic agent in a therapeutically effective amount to a patient in need thereof, wherein the first therapeutic agent is the compound of the present disclosure, or the stereoisomer, the geometric isomer, the tautomer, the pharmaceutically acceptable salt, the solvate or the hydrate thereof of the present disclosure, and the second therapeutic agent is another compound of the present disclosure, or the stereoisomer, the geometric isomer, the tautomer, the pharmaceutically acceptable salt, the solvate or the hydrate thereof of the present disclosure, and/or at least one other therapeutic agent.

In some embodiments of the present disclosure, ENPP 1-mediated diseases or disorders include, but are not limited to, cancers, infectious diseases or disorders, particularly relapsed or refractory cancers, metastatic cancers, and the like.

In another embodiment, ENPP1-mediated diseases or disorders are recurrent or refractory cancers.

In another embodiment, ENPP1-mediated diseases or disorders are metastatic cancer.

In another embodiment, the cancer are solid tumors, for example, breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

In another embodiment, the cancer are hematological malignancies; especially leukemia, lymphoma or myeloma.

In another embodiment, ENPP1-mediated diseases or disorders are infectious diseases or disorders, including but not limited to herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

The phosphonate or amide prodrug compound of formula (I-A) of the present disclosure is in the prodrug form of a biologically active compound and particularly is the active compound substituted at one or more heteroatoms (N, O) with substituents W¹ and W². The group W¹ or W² may be metabolized or otherwise converted under a certain condition so as to produce the active compound (parent drug). The specific embodiments of the present disclosure are not limited to the content of the present disclosure. The phosphonate or amide prodrug compound of the present disclosure can increase the solubility of the biologically active parent drug and thus increase oral bioavailability. It has been found that the substituents W¹ and W² can be metabolized by a phosphatase in the digestive tract, thereby forming a potentially active parent drug to be absorbed by the organism.

It has been found that when the biologically active compound of the present disclosure is formulated in the prodrug form containing the W¹ and W² groups (i.e., the phosphonate or amide compound of formula (I-A)), its lipid solubility and oral bioavailability are remarkably improved. This increase in the lipid solubility contributes to better absorption of the active ingredient in the intestinal tract.

In a specific embodiment, EED Alphascreen binding, LC-MS and/or ELISA analysis disclosed herein are used, and the phosphonic acid compound of the present disclosure, especially the naphthyridine phosphonic acid compound has a IC₅₀ value of ≤ 1µM, more preferably a IC₅₀ value of ≤ 0.5 µM, even more preferably ≤ 0.125 µM, and most preferably a IC₅₀ value of ≤ 0.1 µM..

It should be understood that within the scope of the present disclosure, the technical features defined in each of the technical solutions the present disclosure and the specific technical features described in the following (as examples) can be combined with each other to form new or preferred technical solutions that are not described one by one in the specification. It is also understood that each individual element of the embodiment is its own independent embodiment.

Other features of the present disclosure should become apparent in the course of the above descriptions of exemplary embodiments that are given for illustration of the disclosure and are not intended to be limiting thereof.

### Terminology

In the present disclosure, unless otherwise explicitly stated, the terms used in the present disclosure have the meanings defined below. Terms not explicitly defined in the present disclosure have general meanings that are generally understood by those skilled in the art.

The term "a", "an", "the" and similar terms used in the context of the present disclosure (especially in the context of the claims) are to be construed to cover both the singular and plural unless otherwise indicated herein or clearly contradicted by the context.

A dash ("-") that is not between two letters or symbols is used to indicate a connecting site for a substituent. For example, -O(C₁-C₃ alkyl) indicates that the group is connected to the rest of the molecule through an oxygen atom. However, when the connecting site of the substituent is obvious to those skilled in the art, for example, for substituents such as halogen and hydroxyl, the "-" can be omitted.

When a group has a wavy line " 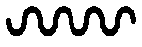 " the wavy line represents the connection position between the group and the rest of the molecule.

As used herein, "- - - - :" represents single or double bonds. Those skilled in the art can determine - - - - : whether to represent a single bond or a double bond based on the valence of the relevant ring atoms and the connected groups, which falls within the scope of their abilities. Those skilled in the art can understand that the relevant rings can be saturated, partially saturated or aromatic.

As used herein, "heteroatoms" refer to nitrogen (N), oxygen (O), or sulfur (S) atoms, particularly nitrogen or oxygen atoms, each of which can be substituted or unsubstituted, including their oxidized forms. Examples of heteroatoms include but are not limited to -O-, -N=, -NR-, -S-, -S(O)- and -S(O)₂-, wherein R is hydrogen, C₁-C₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Any heteroatom with an unsatisfied valence bond is considered to have a hydrogen atom sufficient to satisfy the valence bond, unless otherwise indicated.

As used herein, "halogen" or "halogenated" refers to fluorine, chlorine, bromine and iodine. The preferred halogen as a substituent is fluorine and chlorine.

As used herein, "alkyl" refers to a monovalent hydrocarbon group of completely saturated straight or branched chains. Alkyl preferably contains 1-10 carbon atoms, more preferably 1-8 carbon atoms, 1-6 carbon atoms, 1-4 carbon atoms or 1-3 carbon atoms. The number before alkyl represents the number of carbon atoms. For example, "C₁-C₆ alkyl" represents an alkyl with 1-6 carbon atoms, "C₁-C₄ alkyl" represents an alkyl with 1-4 carbon atoms, and "C₁-C₃ alkyl" represents an alkyl with 1-3 carbon atoms. Representative examples of alkyl include but are not limited to methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, etc. Whether the term "alkyl" appears alone or as part of other functional groups such as haloalkyl, alkoxy, etc., this definition applies.

As used herein, "alkoxy" refers to an alkyl, i.e. an alkyl-O group, connected by an oxygen bridge as defined herein. The number before alkoxy represents the number of carbon atoms. For example, "C₁-C₆ alkoxy" represents an alkoxy with 1-6 carbon atoms, i.e. -O-C₁₋₆ alkyl; "C₁-C₄ alkoxy" refers to an alkoxy with 1-4 carbon atoms, i.e. -O-C₁₋₄ alkyl; and "C₁-C₃ alkoxy" refers to an alkoxy with 1-3 carbon atoms, i.e. -O-C₁₋₃ alkyl. Representative examples of alkoxy include but are not limited to methoxy, ethoxy, propanoxy, 2-propanoxy, butoxy, tert butoxy, pentoxy, hexoxy, etc. Preferably, alkoxy contains about 1-6 or about 1-4 carbons, etc.

As used herein, "cycloalkyl" refers to saturated or partially saturated non-aromatic carbon rings, including monocyclo, bicyclo, or tricyclo, preferably with 3-12 ring carbon atoms, more preferably 3-10 ring carbon atoms, for example, 3-8, 4-10 or 4-8 ring carbon atoms. "C₃-C₈ cycloalkyl" is intended to include C₃, C₄, C₅, C₆, C₇ and C₈ cycloalkyl groups; "C₃-C₆ cycloalkyl" is intended to include C₃, C₄, C₅ and C₆ cycloalkyl; and so on. Exemplary monocyclic cycloalkyls include but are not limited to cyclopropyl, cyclobutyl, cyclopentyl, cyclopentenyl, cyclohexyl and cyclohexene. Exemplary bicyclic cycloalkyls include bornyl, tetrahydronaphthyl, decahydronaphthyl, bicyclo[2.1.1]hexyl, bicyclo[2.2.1]heptyl, bicyclo[2.2.1]heptyl, 6,6-dimethyldicyclo[3.1.1]heptyl, 2,6,6-trimethyldicyclo[3.1.1]heptyl, bicyclo[2.2.2]octyl, etc. Exemplary tricyclic cycloalkyls include diamond alkyl, etc.

As used herein, "haloalkyl" refers to an alkyl as defined herein where one or more hydrogen atoms, for example, 1, 2, 3, 4, 5, 6 or 7 hydrogen atoms, for example, 1, 2 or 3 hydrogen atoms, are replaced by a halogen, and when more than one hydrogen atom is replaced by a halogen atom, the halogen atom can be the same or different from each other. For example, "C₁-C₄ haloalkyl" is intended to include C₁, C₂, C₃ and C₄ haloalkyl groups, and "C₁-C₃ haloalkyl" is intended to include C₁, C₂ and C₃ haloalkyl groups. Examples of haloalkyl include but are not limited to fluoromethyl, difluoromethyl, trifluoromethyl, trichloromethyl, 1,1-difluoroethyl, 1,1-difluoropropyl and 1,1,1-trifluoropropyl. Examples of haloalkyl also include "fluoroalkyl", which is intended to include alkyl as defined herein where one or more hydrogen atoms are substituted by fluorine atoms. The "haloalkyl" herein is preferred that at most three hydrogen atoms in the alkyl are replaced by halogens.

As used herein, "haloalkoxy" refers to a haloalkyl as defined above with a specified number of carbon atoms connected by an oxygen bridge, where one or more hydrogen atoms, for example, 1, 2, 3, 4, 5, 6 or 7 hydrogen atoms, for example, 1, 2 or 3 hydrogen atoms, are replaced by halogens. For example, "C₁-C₆ haloalkoxy" or "C₁ to C₆ haloalkoxy" is intended to include C₁, C₂, C₃, C₄, C₅ and C₆ haloalkoxy groups. Examples of haloalkoxy include but are not limited to fluoromethoxy, difluoromethoxy, trifluoromethoxy, 2-fluoroethoxy, and 2,2,2-trifluoroethoxy.

As used herein, "aryl" refers to a monocyclic, bicyclic or tricyclic carbocyclic hydrocarbyl consisting of one or more rings fused together, with 6-20, preferably 6-14, more preferably 6-12, most preferably 6-10, such as 6-9 cyclic carbon atoms, wherein at least one ring is an aromatic ring, and the other rings (if present) can be aromatic or non-aromatic. The preferred aryl is an aryl with 6-10 cyclic carbon atoms, namely C₆-C₁₀ aryl, which includes: monocyclic aryl (such as phenyl); or a fused double ring system, where one ring is an aromatic ring and the other ring is an aromatic ring (such as in naphthalene or biphenyl) or a non-aromatic ring (such as in dihydroindene or tetrahydronaphthalene). Non-limiting examples of aryl include phenyl, biphenyl, naphthyl, tetrahydronaphthyl, indenyl, dihydroindenyl or anthryl.

As used herein, "heteroaryl" refers to a 5-14 membered, preferably 5-10 membered, more preferably 5-7 membered, or 5-6 membered aromatic ring system containing 1-8, preferably 1-4, further preferably 1-3, more preferably 1 or 2 heteroatoms selected from N, O or S, including a single ring, a double ring, or a fused multi ring, with the remaining ring atoms being carbon atoms. The preferred heteroaryl is 5-10 membered heteroaryl, more preferably 5-7 membered heteroaryl or 5-6 membered heteroaryl, each containing 1, 2 or 3 heteroatoms selected from N, O or S. Examples of heteroaryl include but are not limited to pyrrolyl, furanyl, thienyl, pyrazolyl, imidazolyl, triazolyl, thiazolyl, isothiazolyl, oxazolyl, pyridyl, pyranyl, pyrazinyl, pyridazinyl, pyrimidinyl, oxazinyl, oxadiazinyl, quinolinyl, isoquinolinyl, cinnolinyl, quinazolinyl, quinoxalinyl, benzoxazinyl, 2H-chromone, benzopyranyl, benzothienyl, indolyl, indazolyl, benzoimidazolyl, benzooxazolyl, benzothiazolyl, 7-azaindolyl, 6-azaindolyl, 5-azaindolyl, 4-azaindolyl, 1H-benzo[*d*][1,2,3]triazolyl, etc.

As used herein, "heterocycloalkyl" refers to a cycloalkyl as defined in the present application, provided that one or more cyclic carbons are replaced by heteroatoms selected from N, O or S, the heteroatoms is, for example, -O-, -N=, -NR-, -S-, -S(=O)- and -S(=O)₂-, wherein R is hydrogen, C₁₋₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Preferably, heterocycloalkyl is saturated and partially unsaturated non-aromatic rings of a single, two, or three ring with 3-20 ring atoms, such as 3-12 ring atoms, such as 3-8 ring atoms, such as 3-6 ring atoms. More preferably, heterocycloalkyl preferably contains 4- to 12-membered heterocycloalkyl containing 1, 2, or 3 heteroatoms selected from N, O and S, preferably 4- to 8-membered heterocycloalkyl, more preferably 4- to 7-membered, 4- to 6-membered, or 5- to 6-membered heterocycloalkyl, wherein the heteroatoms are substituted or unsubstituted, such as being substituted by C₁-C₄ alkyl. For example, examples of heterocycloalkyl include but are not limited to oxiranyl, aziridinyl, azetidinyl, oxetanyl, azolidinyl (pyrrolidinyl), tetrahydrofuryl, tetrahydrothienyl, tetrahydrothienyl 1,1-dioxide, pyrazolidinyl, imidazolidinyl, oxazolidinyl, thiazolidinyl, isothiazolidinyl, pyrrolidyl-2-one, imidazolonyl, piperidyl (hexahydropyridine), N-methylpiperidyl, tetrahydropyranyl, oxazinanyl, 1,3-oxazinanyl, hexahydropyrimidinyl, piperazinyl, piperidinylone, 1,4-dioxa-8-aza-spiro[4.5]decane-8-yl, morpholino, thiomorpholino, sulfanomorpholino, sulfonomorpholino, octahydropyrrolo[3,2-b]pyrrolyl, etc.

As used herein, "partially saturated heterocycles" refer to partially hydrogenated non-aromatic rings that can exist as single or double rings (including fused rings). Unless otherwise stated, the partially saturated heterocycles are typically 3- to 12-membered, preferably 5- to 10-membered, more preferably 9- to 10- membered monocyclic system or bicyclic system containing at least one heteroatom, for example, 1 to 3 heteroatoms, preferably 1 or 2 heteroatoms, wherein the heteroatoms are selected from N, O or S, and the heteroatoms are -O-, -N=, -NR- or -S-, and wherein R is hydrogen, C₁₋₄ alkyl, or nitrogen protective groups. Partially saturated heterocycles include functional groups such as dihydropyrrolyl, dihydrofuranyl, dihydrooxazolyl, dihydropyridyl, imidazolin, 1H-dihydroimidazole, 2H-pyranyl, 2H-chromenyl, dihydrooxazinyl, etc. Partially saturated heterocycles also include heterocycles with fused aromatic or heteroaromatic rings, preferably with 9-10 ring members (such asdihydrobenzofuranyl, dihydroisobenzofuranyl, dihydroindolyl (or 2,3-dihydroindolyl), dihydrobenzothiophenyl, dihydrobenzothiazolyl, dihydrobenzopyranyl, tetrahydroquinolyl, tetrahydroisoquinolyl, tetrahydropyridino[3,4-b]pyrazinyl).

As used herein, "partially or completely saturated heterocycles" refer to non-aromatic rings that have been partially or completely hydrogenated and can exist as single rings, double rings (including fused rings) or spiral rings. Unless otherwise stated, heterocycles are typically 3- to 12-membered rings containing 1 to 3, preferably 1 or 2 independently selected heteroatoms from sulfur, oxygen and/or nitrogen, and preferably 5- to 10-membered rings. When the term "partially or completely saturated heterocycles" is used, it is intended to include "heterocycloalkyl" and "partially saturated heterocycles". Examples of spiral rings include 2,6-diazaspiro[3.3]heptanyl, 3-azaspiro[5.5]undecyl, 3,9-diazaspiro[5.5]undecyl, etc.

As used herein, "heterocycles" refer to completely saturated or unsaturated, aromatic or non-aromatic cyclic groups, which are intended to include "heterocycloalkyl", "partially or completely saturated heterocycles", "partially saturated heterocycles", "completely saturated heterocycles" and "heteroaryls". For example, it is a ring system consisting of 4- to 7-membered single rings, 7- to 12-membered double rings, or 10- to 15-membered triple rings, which contain at least one heteroatom on a ring containing at least one carbon atom. A ring containing heteroatoms of the heterocycle can contain 1-6, preferably 1, 2 or 3 heteroatoms selected from nitrogen, oxygen, or sulfur atoms, wherein nitrogen and sulfur heteroatoms can also be optionally oxidized. Preferably, heterocycle is 4-to-7-membered monocyclic heterocycle.

Exemplary monocyclic heterocycles include pyrrolidine, pyrrole, pyrazole, oxocyclobutane, pyrazoline, imidazole, imidazoline, imidazolidine, triazole, thiazole, thiadiazole, thiazolidine, isothiazole, isothiazolidine, furan, tetrahydrofuran, thiophene, piperidine, piperazine, 2-oxopiperazine, 2-oxopiperidine, 2-oxypyrrolidine, 4-piperidone, pyridine, pyrazine, pyrimidine, pyridazine, tetrahydropyran, morpholine, thiomorpholine, sulfanomorpholino, sulfonomorpholino, 1,3-dioxolane and tetrahydro-1,1-dioxothiophene, 1,1,4-trioxo-1,2,5-thiadiazolidin-2-, etc.

Exemplary bicyclic heterocycles include indole, dihydroindole, benzothiazole, etc.

As used herein, "heterocyclic group" refers to a group formed by the loss of one or more hydrogen atoms from the heterocycle defined above. Heterocyclic groups can be connected at heteroatoms or carbon atoms.

As used herein, "carbon ring" refers to a saturated or unsaturated monocyclic, bicyclic or tricyclic hydrocarbon group consisting of 3-12 carbon atoms. The carbon ring is preferably composed of 3-8 cyclic carbon atoms, such as 3-7 or 4-7 cyclic carbon atoms. Exemplary monocyclic carbon rings include but are not limited to cyclopropane, cyclobutane, cyclopentane, cyclopentene, cyclohexane, cyclohexene, cycloheptane, cycloheptene, etc. Exemplary bicyclic hydrocarbons include tetrahydronaphthalene, decahydronaphthalene, bicyclic[2.1.1]hexane, bicyclic[2.2.1]heptane, etc. Exemplary tricyclic hydrocarbon groups include diamond alkyl, etc.

As used herein, the term "-C(=O)" refers to carbonyl.

Alkyl, alkenyl, alkoxy, carbon ring, cycloalkyl, heteroaryl, heterocycle, heterocyclic, carbonyl, sulfonyl, sulfinyl, and other functional groups as used herein can be substituted by substituents, and the substituents include but not limited to OH, Boc, halogen, cyanide, substituted or unsubstituted alkyl, substituted or unsubstituted alkoxy, substituted or unsubstituted cycloalkyl, substituted or unsubstituted heterocyclic; NRR', C(O)R, SO₂R, C(O)NRR' or C(O)OR, and R and R' are each independently selected from H and substituted or unsubstituted alkyl.

The term "optionally" used herein refers to the events described subsequently that may or may not occur, and this description includes both the situations in which the event occurred and the situations in which it did not occur. For example, "optionally substituted alkyl" includes "unsubstituted alkyl" and "substituted alkyl" as defined herein. "Optionally substituted by halogens" includes situations where "substituted by halogens" and situations where "not substituted by halogens", such as being substituted by 0-3 halogens. Those skilled in the art should understand that for any functional group containing one or more substituents, the group does not include any spatially impractical, chemically incorrect, synthetic infeasible, and/or inherently unstable substitution patterns.

When any variable appears more than once in any composition or formula of a compound, its definition at each occurrence is independent of its definition at each other occurrence. Therefore, for example, if a group is shown to be substituted by 0-3 Rs, the group can be either unsubstituted or substituted by up to three Rs, and R is independently selected from the definition of R each time it appears. For example, when applied to one or more substitutions in the definition of ring A, that is, when ring A is C₆-C₁₀ aryl or 5- to 6-membered heteroaryl, these groups are unsubstituted or substituted by one or more, for example, two substituent groups independently selected from the given definition each time they appear. This applies similarly to the definition of other similar situations.

When the valence bond of a substituent shows a valence bond connecting two atoms through the ring, the substituent can bond with any atom on the ring. When a substituent is listed without specifying the substituent connected to the rest of the compound of the formula, the substituent can be bonded through any atom in the substituent.

The combination of substituents and/or variables is allowed as long as such combination produces stable compounds.

When using dashed rings in a ring structure, it indicates that the ring structure can be saturated, partially saturated or unsaturated.

The term "substituted" used herein refers to one or more hydrogen atoms on a given atom or group being replaced by one or more substituents selected from a given substituent group, provided that they do not exceed the normal valence of the given atom. When the substituent is an oxo (i.e.=O), the two hydrogen atoms on a single atom are replaced by oxygen. There are no oxygen substituents present on the aromatic portion. When a ring system (such as a carbon ring or heterocycle) is replaced by a carbonyl group or double bond, it is intended that the carbonyl group or double bond is part of the ring (i.e., within the ring). Only when the combination of substituents and/or variables leads to chemically correct and stable compounds, such combinations are allowed. A chemically correct and stable compound means that it is sufficiently stable to be separated from the reaction mixture and its chemical structure can be determined, and subsequently formulated into a preparation with at least practical utility. For example, in the absence of a clear list of substituents, the terms "substituted" used herein refer to one or more hydrogen atoms on a given atom or group being independently replaced by one or more substituents, such as 1, 2, 3 or 4 substituents. When an atom or group is replaced by multiple substituents, the substituents can be the same or different.

Unless otherwise specified, the terms "the compound of the present disclosure" refer to a compound comprising one or more of the formula (I) or its subforms defined herein, such as formulas (II) and (III), or their pharmaceutically acceptable salts, and all isomers such as stereoisomers (including diastereoisomers, enantiomers and racemes), geometric isomers, conformational isomers (including rotamers and atropisomers), tautomers, internal addition products of isomers, prodrugs, and isomer labeled compounds (including deuterium substitution) and naturally formed parts (such as polymorphic forms, solvates, and/or hydrates). When there is a part that can form salt, it also includes salt, particularly a pharmaceutically acceptable salt. The presence of internal addition products of tautomers or isomers can be identified by those skilled in the art using tools such as NMR. The compound of formula (I) of the present disclosure can easily form internal addition products of tautomers and isomers as described herein.

When formula (I) is referred to herein, this designation also includes its subformulae, e.g. formulae (II), (III), (IV), (V), (VI), (VII), (VIII) and (IX), unless otherwise explained in accordance with the context. When formula (I-A) is referred to herein, this designation also includes its subformulae, e.g. formulae (II-A), (III-A), (IV-A), (V-A), (VI-A), (VII-A), (VIII-A) and (IX-A), unless otherwise explained in accordance with the context.

Those skilled in the art will recognize that the compound of the present disclosure may contain chiral centers, allowing for the existence of different isomeric forms. Therefore, the compound of the present invention can be present in the form as individual stereoisomers (e.g. enantiomers and diastereomers) and a mixture thereof in any proportion, for example, a racemate, and where appropriate, in the form as tautomers and geometric isomers thereof. As used herein, "stereoisomers" refer to different compounds with the same molecular formula but different atomic arrangements and configurations.

The stereoisomers include enantiomers, diastereomers and conformers. As used herein, the "enantiomers" are a pair of stereoisomers that are non-superimposable mirror images of each other. A mixture of a pair of the enantiomers at 1:1 is a "racemic" mixture. When appropriate, the term is used to refer to a racemate mixture.

"Diastereomers" are stereoisomers that have at least two asymmetric atoms but are not mirror images of each other. A mixture of the diastereomers may be separated by a high-resolution analytical method such as electrophoresis and chromatography, e.g., HPLC. As used herein, the "racemate mixture" and the "racemate" refer to an equimolar mixture of two enantiomers without the optical activity. The racemate mixture can be used as such or resolved into individual isomers. The resolution may result in a stereochemically pure compound or in a mixture enriched in one or more isomers.

As used herein, the "tautomer" or "tautomeric form" refers to structural isomers of different energies that are interconvertible via a low-energy barrier. For example, proton tautomers (also known as proton transfer tautomers) include interconversion by proton migration, such as keto-enol and imine-enamine isomerizations. Valence tautomers include interconversions through recombination of some bonding electrons.

As used herein, the "geometric isomers" are isomers resulting from the inability of a free rotation of a double bond or a single bond of a cyclic carbon atom and also known as cis-trans isomers. Cis-isomers are substituent groups on the same side of the plane and trans-isomers are substituent groups on the opposite side of the plane. If the compound contains a double bond, the substituent may be in the E or Z conformation.

Some of the compounds described herein contain one or more asymmetric centers or axes, thus producing enantiomers, diastereomers, and other stereoisomers that can be defined as (R)- or (S)- in absolute stereochemistry.

The "conformational isomers" are isomers that differs by the rotation of one or more valence bonds. "Rotamers" are conformational isomers that differs by the rotation of only a single valence bond. "Atropisomers" refers to a structural isomer based on axial or planar chirality generated by rotational confinement within a molecule.

Unless otherwise stated, the compound of the present disclosure is intended to include all such possible isomers, including racemic mixtures, optically active forms, and intermediate mixtures. (R)- and (S)- isomers with optical activity can be prepared using chiral synthons or chiral reagents, or split using conventional techniques.

The compound of the present disclosure can be separated into optically active or racemic forms. The compound in the optically active form can be split through racemic separation or synthesis from materials with optical activity. All methods for preparing the compound of the present disclosure and the intermediates prepared therein are considered to be part of the present disclosure. When preparing enantiomer or diastereomer products, they can be separated by conventional methods such as chromatography or stepwise crystallization.

According to the method conditions, the end product of the present disclosure is obtained in the form of free (neutral) or salt. The free and salt forms of these end products are within the scope of the present disclosure. If desired, one form of the compound can be transformed into another form. Free bases or acids can be converted into salts; salts can be converted into free compounds or other salts; the mixture of isomeric compounds of the present disclosure can be separated into individual isomers.

Pharmaceutically acceptable salts are preferred. However, other salts can be useful, for example, in separation or purification steps, which can be used during preparation and are therefore considered within the scope of the present disclosure.

As used herein, "pharmaceutically acceptable" and "medical" are interchangeable, including substances or compositions that must be chemically/toxicologically compatible with other components, including formulations, and/or mammals treated with them.

As used herein, the "pharmaceutical salt" and "pharmaceutically acceptable salts" refer to salts that maintain the biological effects and properties of the compound of the present disclosure, and such salts are not unexpected in terms of biology or other aspects. Non-limiting examples of the salt include non-toxic, inorganic or organic base or acid addition salts of the compound of the present disclosure. The salts include, but not limited to an acid addition salt formed by the compound of the present disclosure with an inorganic acid, including, for example, hydrochloric acid, hydrobromic acid, sulfuric acid, sulfites, carbonates, bicarbonates, nitric acid, phosphoric acid and the like, as well as an acid addition salt formed by the compound of the present disclosure with an organic acid, including, for example, acetic acid, propionic acid, hydroxyacetic acid, pyruvate, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, etc.; and also include a pharmaceutically acceptable base addition salt formed by the compound of the present disclosure with inorganic and organic bases from which salts can be derived include, for example, sodium, potassium, lithium, ammonium, calcium, magnesium, iron, zinc, copper, manganese, aluminum, etc.; particularly preferred are ammonium, potassium, sodium, calcium and magnesium salts. Organic bases from which salts can be derived include, for example, primary amines, secondary amines and tertiary amines, substituted amines (including naturally occurring substituted amines), cyclic amines, alkaline ion exchange resins, etc., especially such as isopropylamine, trimethylamine, diethylamine, triethylamine, tripropylamine and ethanolamine. By conventional chemical methods, the pharmaceutically acceptable salt of the present disclosure can be synthesized from the parent compound (alkaline portion or acidic portion). In general, the salt can be prepared by reacting the free acid form of the compound with a stoichiometric quantity of an appropriate base (such as hydroxide of Na, Ca, Mg or K, carbonate, bicarbonate, etc.) or by reacting the free base form of the compound with a stoichiometric quantity of an appropriate acid. This type of reaction is usually carried out in water, organic solvents or a mixture of both. In general, non-aqueous media such as ether, ethyl acetate, ethanol, isopropanol or acetonitrile are preferred when feasible.

As used herein, "pharmaceutical composition" refers to the compound which can be administered through oral, rectal, may optionally comprise one or more pharmaceutically acceptable excipients.

As used herein, "pharmaceutically acceptable excipients" include any and all solvents, dispersants, coatings, surfactants, antioxidants, preservatives (such as antibacterial agents, antifungal agents), isotopes, absorption retardants, salts, drugs, drug stabilizers, adhesives, excipients, disintegrants, lubricants, sweeteners, correctors, dyes, similar substances and their combinations, which is well-known to those skilled in the art (see, for example, Remington's Pharmaceutical Sciences, 18th edition, Mack Printing Company, 1990, pp 1289-1329, which was introduced herein as a reference). Unless any conventional carrier cannot coexist with the active ingredient, it can be considered for use in therapeutic or pharmaceutical compositions.

As used herein, "solvate" refers to the physical combination of the compound of the present disclosure with one or more organic or inorganic solvent molecules. This physical combination includes hydrogen bonds. In some cases, solvates will be able to separate, for example, when one or more solvent molecules are incorporated into the lattice of a crystalline solid. The solvent molecules in the solvate can exist in a regular and/or unordered arrangement. The solvate can contain solvent molecules with a stoichiometric quantity or a non-stoichiometric quantity. "Solvates" include solution phases and separable solvates. Exemplary solvates include but are not limited to hydrates, ethanolamides, methanolides and isopropanolides. The method of solvation is well-known in the art.

As used herein, "polymorphic form" refers to crystalline forms with the same chemical structure/composition, but with different spatial arrangements of molecules and/or ions that form crystals. The compound of the present disclosure can be provided as amorphous or crystalline solids. The freeze-drying method can be used to provide the solid compound of the present disclosure.

As used herein, "prodrug" refers to a chemically modified active or inactive compound that, after administration to an individual, undergoes physiological processes in the body (such as hydrolysis, neometabolism, etc.) to become the compound of the present disclosure. The adaptability and technology related to the manufacturing and use of prodrugs are well-known to those skilled in the art.

As used herein, the "therapeutic effective amount" of the compound of the present disclosure refers to the amount of the compound of the present disclosure that can cause individual biological or medical reactions, improve symptoms, slow or delay disease progression, or prevent disease. The "therapeutic effective amount" can be determined by participating physicians or veterinary practitioners, and will vary depending on factors such as the compound, the disease status being treated, the severity of the disease being treated, the individual's age and relevant health status, the route and form of administration, and the judgment of the attending physician or veterinary practitioner.

As used herein, "individual" refers to animals. Preferably, animals are mammals. Individuals also refer to primates (for example, humans), cows, sheep, goats, horses, dogs, cats, rabbits, rats, mice, fish, birds, etc. In a preferred embodiment, the individual is a human.

As used herein, "inhibition" refers to the alleviation or inhibition of a specific patient, symptom or disease, or a significant decrease in biological activity or process baseline activity.

As used herein, the term "treatment" or any disease or condition in an embodiment refers to the improvement of diseases or conditions (i.e., preventing or slowing down the development of the disease or at least one clinical symptom). In another embodiment, "treatment" refers to improving at least one physical parameter that may not be perceived by the patient. In another embodiment, "treatment" refers to the regulation of diseases or conditions on the body (such as stable and perceptible symptoms) or physiology (such as stable body parameters) or both.

As used herein, "prevention" refers to the administration of one or more pharmaceutical substances, particularly the compound, and/or the pharmaceutically acceptable salt thereof of the present disclosure, to individuals with a predisposition to the disease, in order to prevent them from developing the disease.

When it comes to chemical reactions, "treatment", "contact" and "reaction" refer to adding or mixing two or more reagents under appropriate conditions to produce the shown and/or desired products. It should be understood that the reaction that produces the shown and/or desired product may not necessarily come directly from a combination of the two reagents initially added, i.e., there may be one or more intermediates generated in the mixture that ultimately lead to the formation of the shown and/or desired product.

In general, the term "about" is used herein to adjust the given value to be 20% higher or lower than that value, such as 10%, such as 5%.

The arbitrary formula given herein is also intended to represent the unlabeled form of the compound and the isotopic labeled form. Isotopic labeled compounds have the structure described in the formula provided herein, except for one or more atoms replaced by atoms with the selected atomic mass or mass number. Examples of isotopes that can be incorporated into the compound of the present disclosure include isotopes of hydrogen, carbon, nitrogen, oxygen, phosphorus, fluorine and chlorine, such as ²H (i.e. D), ³H (i.e. T), ¹¹C, ¹³C, ¹⁴C, ¹⁵N, ¹⁸F, ³¹P, ³²P, ³⁵S, ³⁶Cl and ¹²⁵I, respectively. The present disclosure includes different isotopic labeled compounds as defined herein, such as those containing radioactive isotopes such as ³H, ¹³C and ¹⁴C. These isotopic labeled compounds can be used for metabolic studies (using ¹⁴C), reaction kinetics studies (such as using ²H or ³H), detection or imaging techniques, such as positron emission tomography (PET) or single photon emission computed tomography (SPECT), including drug or substrate tissue distribution measurements, or can be used for patient radiation therapy. Particularly, ¹⁸F or labeled compounds can be particularly desired for use in PET or SPECT studies. Typically, isotopic labeled compounds of the present disclosure can be prepared by using readily available isotope labeled reagents instead of non-isotope labeled reagents, as described in the following processes or examples and preparation examples.

Moreover, being replaced by heavier isotopes, particularly deuterium (i.e. ²H or D), can also yield certain therapeutic benefits due to greater metabolic stability, such as prolonging in vivo half-life, reducing dose requirements, or improving treatment indices. It can be understood that deuterium in the context can be regarded as a substituent of the compound of the present disclosure. The concentration of these heavier isotopes, particularly deuterium, can be defined by isotopic enrichment factors. "Isotope enrichment factor" represents the ratio between the isotopic abundance of a specified isotope and its natural abundance.

The isotope-labeled compound of the present disclosure can typically be prepared using conventional techniques known to those skilled in the art or by performing methods similar to those described herein, using appropriate isotope-labeled reagents instead of other unlabeled reagents used. These compounds have various potential applications, such as serving as standards and reagents for determining the ability of potential drug compounds to bind to target proteins or receptors, or for imaging the compound of the present disclosure that binds to biological receptors in vivo or in vitro.

The undefined technical and scientific terms used herein have the meanings commonly understood by those skilled in the art to which the present disclosure belongs.

### Efficacy

The hydroxamic acid compound of the present disclosure is an ENPP1 inhibitor compounds with high selectivity and inhibitory activity towards ENPP1, fewer side effects, high resistance, high bioavailability, and high clinical application value. The compound of the present disclosure can be used in various applications that require inhibition of ENPP1.

The compound of the present disclosure can be used to treat, prevent or improve ENPP1-mediated diseases or obstacles, for example, ENPP1-mediated tumors (such as cancer) or infectious diseases or obstacles, especially recurrent or refractory cancers or metastatic cancers.

Particularly, the compound of the present disclosure can be used for treating, preventing or improving solid tumors, for example, selected from breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

The compound of the present disclosure can also be used for treating, preventing or improving hematopoietic malignancies, for example, selected from leukemia, lymphoma or myeloma.

Moreover, the compound of the present disclosure can also be used to treat, prevent or improve infectious diseases or conditions, particularly ENPP1-mediated infectious diseases or conditions, for example, selected from herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

### Pharmaceutical Composition and Administration

The compound of the present disclosure can be administered to individuals in the form of pharmaceutical compositions, which may optionally comprise one or more pharmaceutically acceptable excipients.

The compound of the present disclosure can be administered through various known routes, including oral, rectal, gastric, intracranial and parenteral administration, for example, intravenous, intramuscular, nasal, dermal, subcutaneous and similar administration routes. Particularly preferred are oral, intranasal and parenteral administration. Depending on the administration route, different pharmaceutical preparations are required, and some of these administration routes may require the application of protective coatings to the pharmaceutical preparations to prevent degradation of the compound of the present disclosure, for example, in the digestive tract.

The compound of the present disclosure can be prepared into syrup, infusion or injection, spray, tablet, capsule, pastille, liposome or suppository, etc.

The particularly preferred drug form for administering the compound of the present disclosure is a form suitable for injection, including sterile aqueous solutions or dispersions and sterile powders for immediate preparation of sterile injection solutions or dispersions. In all cases, the final solution or dispersion form must be sterile and fluid. Typically, such solutions or dispersions will contain solvents or dispersion medium that contain, for example, water-buffered aqueous solutions such as biocompatible buffering agents, ethanol or polyols such as glycerol, propylene glycol, polyethylene glycol, suitable mixtures thereof, surfactants or vegetable oils. The compound of the present disclosure can also be formulated into liposomes, particularly liposomes for parenteral administration. Liposomes provide the advantage of increased half-life in circulation (if compared to free drugs) and longer and more uniform release of encapsulated drugs.

The sterilization of infusion and injection can be achieved through recognized techniques in this field, including but not limited to the addition of preservatives such as antibacterial or antifungal agents, such as nipagin esters, tert butanol trichloride, phenol, sorbic acid or thiomersal. In addition, isotonic agents such as sugar or salt, particularly sodium chloride, can be added to infusion and injection.

The production of sterile injections containing one or more compounds of the present disclosure is completed by introducing the required amount of each compound into a suitable solvent with the various components listed above as appropriate, and then sterilizing. To obtain sterile powder, vacuum dry or freeze dry the above solution as needed. The preferred diluents of the present disclosure are water, physiologically acceptable buffering agents, physiologically acceptable buffer salt solutions or salt solutions. The preferred carriers are cocoa butter and vitebesole.

The excipients that can be used in conjunction with various drug forms of the compound of the present disclosure can be selected from the following non-limiting list: a) adhesives, such as lactose, mannitol, crystalline sorbitol, hydrogen phosphate, sugars, microcrystalline cellulose, carboxymethyl cellulose, hydroxyethyl cellulose, and/or polyvinylpyrrolidone; b) lubricants, such as magnesium stearate, talc powder, calcium stearate, zinc stearate, stearic acid, hydrogenated vegetable oil, leucine, glyceride and sodium stearoyl fumarate; and c) disrupting agents, such as starch, cross-linked carboxymethyl cellulose, sodium methylcellulose, agar, bentonite, alginate, carboxymethyl cellulose, and/or polyvinylpyrrolidone. Other suitable pharmaceutical carriers and their formulations are well-known in this field.

In one embodiment, the preparation is used for oral administration and comprises one or more or all of the following ingredients: pre-gelatinized starch, talc powder, polyvinylpyrrolidone K30, cross-linked carboxymethyl cellulose sodium, sodium stearyl fumarate, gelatin, titanium dioxide, sorbitol, monosodium citrate, xanthan gum, titanium dioxide, flavoring agent, sodium benzoate and saccharin sodium.

In an embodiment, the compound of the present disclosure is administered intranasally, which can be administered in a dry powder inhaler or a spray in a pressurized container, pump, spray or atomizer using a suitable propellant such as dichlorodifluoromethane, trichlorofluoromethane, dichlorotetrafluoroethane, hydrofluorane such as 1,1,1,2-tetrafluoroethane (HFA 134A^{™}) or 1,1,1,2,3,3,3-hexafluoropropane (HFA 227EA^{™}), carbon dioxide, or other suitable gases. The pressurized container, pump, spray or atomizer can contain a solution or suspension of the compound of the present disclosure, such as a solution or suspension using ethanol and a propellant as the solvent, and it can also contain a lubricant, such as sorbitane trioleate.

The typical dosage range of the compound of the present disclosure is 0.001-1000 mg of active ingredient/kg body weight/day. The dose can be administered once a day or in multiple doses. The determination of appropriate dosage is determined by the attending physician based on the type and severity of the disease to be treated, the individual's health status and past medical history, shared medications, specific compounds to be administered, and routes of administration. As needed, the dosage of the compound of the present disclosure may exceed this dosage range.

### Pharmaceutical Combination

The compound of the present disclosure may be used alone or in combination with one or more other active agents or therapies for the purposes described herein, which may have or produce the same or different pharmacological effects. The compound of the present disclosure may be administered simultaneously, before, or after other active agents or therapies.

When the compound of the present disclosure is administered in combination with other active agents, the dosage of the other active agents administered in combination will naturally vary depending on factors such as the shared drug, the disease to be treated, the general health condition of the patient, and the judgment of a physician or veterinarian. The compound of the present disclosure can be administered simultaneously, separately or sequentially with other active agents in combination through the same or different administration routes. They can be comprised in the same pharmaceutical composition, or they can be a combination product in a separate form, for example, in the form of a medicine box. They can be prepared and/or formulated by the same or different manufacturers. Moreover, the compound of the present disclosure and other active agents may be added in combination therapy (i) before sending the combination product to physicians (for example, in the case of a medicine box containing the compound of the present disclosure and other drugs); (ii) prior to administration, by the physician themselves (or under the guidance of the physicians); (iii) by the patients themselves, for example, during the sequential administration of the compound of the present disclosure and other active agents.

In an embodiment, the present disclosure provides a pharmaceutical composition comprising the compound or the pharmaceutically acceptable salt thereof of the present disclosure, as well as one or more other active agents. Optionally, the pharmaceutical composition may comprise a pharmaceutically active excipient as described above.

In an embodiment, the present disclosure provides a medicine box comprising two or more separate pharmaceutical compositions, wherein at least one comprises a compound of formula (I) or a pharmaceutically acceptable salt thereof. In an embodiment, the medicine box comprises utensils for separately retaining the composition, such as containers, separate bottles, or separate foil bags. An example of this type of medicine box is a blister bag, which is usually used for packaging tablets, capsules, etc.

The medicine box of the present disclosure can be used for administering different dosage forms, such as oral and parenteral dosage forms, for administering individual compositions at different dose intervals, or for gradually increasing individual compositions relative to another. To aid compliance, the medicine box of the present disclosure typically includes an administration instruction.

In the combination therapy of the present disclosure, the compound of the present disclosure and other therapeutic agents may be prepared and/or formulated by the same or different manufacturers. Moreover, the compound of the present disclosure and other therapeutic agents can be introduced together into combination therapy, which can be done (i) before sending the combination product to physicians (for example, for a medicine box comprising the compound of the present disclosure and other therapeutic agents); (ii) prior to administration, by the physician themselves (or under the guidance of the physicians); (iii) by the patients themselves, for example, during the sequential administration of the compound of the present disclosure and other therapeutic agents.

### General Synthesis Methods

The compound of the present disclosure can be prepared by various methods, including the methods described below, the methods described in examples, or similar methods. The following text describes a suitable general synthesis scheme. The appropriate reaction conditions for each reaction step are known to those skilled in the art. The raw materials can be purchased and obtained, or can be prepared through the methods described below, similar methods to those provided below, or methods known in the art. The variables in the general formula have the same meanings as in the previous text, unless otherwise stated.

In an embodiment, the compound of the present disclosure can be synthesized through the following general synthesis scheme, where each variable is defined herein, and the specific reaction conditions are the same as in the example.

### Process 1

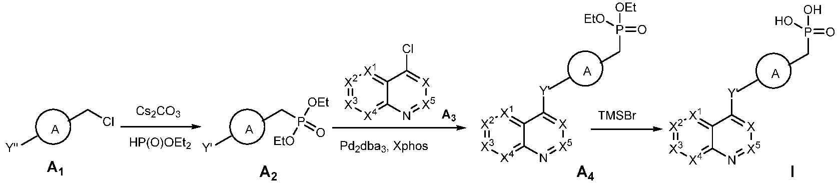

In the above reaction scheme, Y' is hydroxyl, Y" is C₁-C₄ alkoxy, and X¹, X², X³, X⁴, Y and ring A are respectively as previously defined.

The compound of general formula A₂ may be prepared by the following synthetic method: adding a starting material A₁, diethyl phosphate and cesium carbonate into dimethylformamide, reacting same at room temperature overnight, adding water into reaction solution for quenching, extracting same by dichloromethane, washing the organic phase using a saturated sodium chloride solution, concentrating same under reduced pressure, and purifying the residue through a silica gel column to obtain the compound of general formula A₂.

The compound of general formula A₄ may be prepared by the following synthetic method: adding starting materials A2 and A3, cesium carbonate, Pd₂dba₃ and Xphos into dioxane and reacting same while stirring overnight at 100°C under nitrogen protection; and cooling the reaction solution to room temperature, filtering and concentrating same, and purifying the residue using a high-performance liquid phase reverse-phase preparative column to obtain the compound of general formula A₄.

A compound of formula A₅ may be prepared by the following synthetic method: adding the starting material A₄ and trimethyl bromosilane into acetonitrile, reacting same at room temperature for 16 h, concentrating same, and separating and purifying same using a high-performance liquid phase reverse-phase preparative column to obtain the compound of general formula I.

In another embodiment, the compound A₃ of the present disclosure may be synthesized using the following general synthetic scheme:

### Process 2

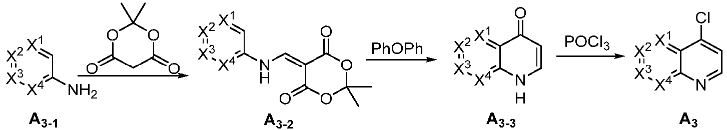

A compound of general formula A₃ may be prepared by the following synthetic method: adding trimethyl orthoformate and 2,2-dimethyl-1,3-dioxan-4,6-dione, reacting same at 100°C for 2 h, adding a compound A₃₋₁, and continuously reacting same at the temperature for 2 h; cooling the reaction solution to room temperature to precipitate a solid, filtering, and then collecting the solid to obtain a compound A₃₋₂; dissolving the compound A₃₋₂ in diphenyl ether, reacting same at 230°C for 1 h, cooling the reaction solution to room temperature, adding petroleum ether to precipitate a solid, filtering, and then collecting the solid to obtain a compound A₃₋₃; and reacting the compound A₃₋₃ with phosphorus oxychloride at 100°C for 1 h, cooling the reaction solution to room temperature, removing excessive phosphorus oxychloride, adding iced water into the residue, adjusting the pH to 8 with ammonium hydroxide, extracting same with dichloromethane, successively washing the organic phase with water and a saturated sodium chloride solution, and drying, filtering, concentrating, and purifying same with a silica gel column to obtain the compound of general formula A₃.

### Brief Description of the Drawings

FIG. 1 is a pharmacokinetics curve graph of an active ingredient compound 1 in plasma of mice after oral administration of a prodrug compound 7 (50 mg/kg); and
FIG. 2 is a pharmacokinetics curve graph of an active ingredient compound 1 in plasma of mice after oral administration of a prodrug compound 8 (50 mg/kg).

### DETAILED DESCRIPTION OF THE INVENTION

In the present application, when the chemical name and structural formula are inconsistent, the one shown in the structural formula shall prevail, unless it can be inferred from the context that the chemical name rather than the structural formula is correct.

As those skilled in the art can clearly understand, for the sake of simplicity, not all hydrogen atoms are clearly labeled in the structural formulas of some compounds provided in the present application. When there is a vacant valence of carbon or nitrogen atoms in a compound, it indicates the presence of unmarked hydrogen.

The compound of the present disclosure can be prepared using various methods known to technicians in the field of organic synthesis according to the methods, reaction processes and examples provided herein. The compound of the present disclosure can be synthesized using the methods described below in combination with known synthesis methods in the field of organic chemistry, or through alternative methods that can be understood by those skilled in the art. The preferred methods include but are not limited to those described below. The reaction is carried out in a solvent or solvent mixture suitable for the reagents and materials used and suitable for the transformation being carried out. Technicians in the field of organic synthesis can understand that the functional groups present on the molecule should be consistent with the planned conversion. This sometimes requires judgment to change the sequence of synthesis steps or to choose a specific operational process relative to others to obtain the expected compound of the present disclosure.

The present disclosure will be further described in combination with specific examples. It should be understood that these examples are only used to illustrate the present disclosure and not to limit the scope of the present disclosure. The experimental methods without specific conditions in the following embodiments are usually under conventional conditions, or the conditions recommended by the manufacturer. Unless otherwise stated, percentages and parts are weight percentages, weight parts or volume percentages (when liquid).

For the purpose of explanation, the reaction process described below provides potential pathways and key intermediates for synthesizing the compound of the present disclosure. For a more detailed description of individual reaction steps, please refer to the following example section. Those skilled in the art will understand that other synthetic pathways can be used to synthesize the compound of the present disclosure. Although specific raw materials and reagents are described in the process and discussed below, other raw materials and reagents can be easily replaced to provide various derivatives and/or reaction conditions. In addition, most of the compounds prepared by the following methods can be further modified using conventional chemistry familiar to those skilled in the art according to the present disclosure.

In the preparation of the compound of the present disclosure, protection of the distal functional group of the intermediate may be necessary. The demand for such protection will vary depending on the properties of the remote functional groups and the conditions of the preparation method. The need for such protection is easily determined by those skilled in the art.

### Examples

The experimental materials and reagents used in the following examples can be obtained from commercial channels unless otherwise stated. Raw materials can usually be obtained from commercial sources or easily prepared using methods known to those skilled in the art.

In each example, the experimental instrument description (for example, ¹H NMR was recorded by Varian Mercury-300 or Varian Mercury-400 nuclear magnetic resonance instrument, ¹³C NMR was recorded by Varian Mercury-400 or Varian Mercury-500 or Varian Mercury-600 nuclear magnetic resonance instrument, chemical shift was represented by δ (ppm); the mass spectrometry was recorded by Finnigan/MAT-95 (EI) and Finnigan LCQ/DECA and Micromass Ultra Q-TOF (ESI) mass spectrometers; reverse phase preparation of silica gel for HPLC separation was 200-300 meshes).

### Abbreviations

| | | | |
|---|---|---|---|
| Cs₂CO₃ | Cesium carbonate | Xphos | 2-dicyclohexylphosphino-2',4',6'-triisop ropylbiphenyl |
| PhOPh | Diphenyl ether | Pd₂dba₃ | Tris(dibenzylideneacetone)dipalladium |
| HC(OMe)₃ | Trimethyl orthoformate | TMSBr | Trimethyl bromosilane |
| HP(O)(OEt)2 | Triethyl phosphate | DIEA | N,N-diisopropyl ethylamine |
| DMAP | 4-dimethylaminopyrid ine | PE | Petroleum ether |
| EA | Ethyl acetate | DPBP | 1,1' -biphenyl-2,2' -bisdiphenylphosphine |

### Synthesis of Key Intermediates

### Intermediate 1a: 5-chloro-2-methoxy-1,8-naphthyridine

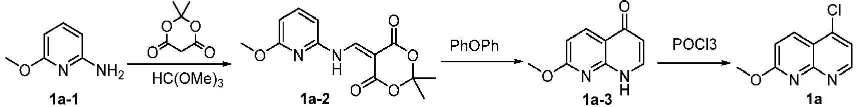

### Step 1: Synthesis of 5-(((6-methoxypyridin-2-yl)amino)methylene)-2,2-dimethyl-1,3-dioxane-4,6-dione

Compound 2,2-dimethyl-1,3-dioxan-4,6-dione (12.7 g, 88.61 mmol) was added to a single-necked bottle (250 mL) containing trimethyl orthoformate (100 mL). The reaction solution was stirred at 100°C for 2 hours, then compound **1a-1** (5.0 g, 40.28 mmol) was added to continue the reaction at this temperature for 2 hours. After cooling the reaction solution to room temperature, the solid was precipitated, filtered, and collected to obtain compound **1a-2** (10 g, yellow solid) with a yield of 89%. ¹H NMR (400 MHz, CDCl₃-*d*): δ 9.30 (d, *J* = 13.6 Hz, 1H), 7.63-7.59 (m, 1H), 6.63-6.57 (m, 2H), 3.99 (s, 3H), 1.76 (s, 6H).

### Step 2: Synthesis of 7-methoxy-1,8-naphthyridin-4(1H)-one

**1a-2** (5.0 g, 17.98 mmol) was added to a single-necked bottle (250 mL) containing 50 mL of diphenyl ether, stirred at 230°C for 1 hour. The reaction solution was cooled to room temperature, petroleum ether was added, and the solid was precipitated, filtered and collected to obtain compound **1a-3** (2.9 g, yellow solid), with a yield of 94%. ¹H NMR (400 MHz, CDCl₃-*d*): δ 11.97 (s, 1H), 8.30 (d, *J =* 8.8 Hz, 1H), 7.78-7.75 (m, 1H), 6.79 (d, *J =* 8.8 Hz, 1H), 6.05-6.03 (m, 1H), 3.96 (s, 3H).

### Step 3: Synthesis of 5-chloro-2-methoxy-1,8-naphthyridine

Compound **1a-3** (500 mg, 2.84 mmol) and 10 mL of phosphorus oxychloride were added sequentially to a single-necked bottle (100 mL). After a reaction at 100°C for 1 hour, the reaction solution was cooled to room temperature to remove excess phosphorus oxychloride. Ice water was added to the residue, and the pH was adjusted to 8 with ammonia water. The reaction solution was extracted with dichloromethane. The organic phase was washed sequentially with water and saturated salt water, dried with anhydrous sodium sulfate, filtered, and concentrated to obtain the crude product. The crude product was purified on a silica gel column (PE: EA = 5:1) to obtain compound **1a** (400 mg, white solid) with a yield of 73%. LCMS(ESI): *m*/*z* 195.1 [M+H]⁺; RT = 1.137 min (6.00 min).

### Intermediate 2a: 4-chloro-1,3-dimethyl-1H-pyrazolo[3,4-b]pyridine

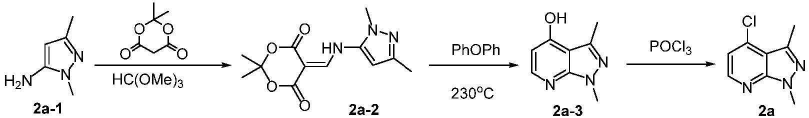

### Step 1: Synthesis of 5-(((1,3-dimethyl-1H-pyrazole-5-yl)amino)methylene)-2,2-dimethyl-1,3-dioxane-4,6-dione

2,2-dimethyl-1,3-dioxan-4,6-dione (2.33 g, 16.19 mmol) was added to a single-necked bottle (100 mL) containing 30 mL of trimethyl orthoformate. After reacting at 100°C for 2 hours, compound **2a-1** (1.5 g, 13.5 mmol) was added and stirred at 100°C for another 2 hours. After cooling the reaction solution to room temperature, the reaction solution was diluted with petroleum ether, filtered, and the solid was collected to obtain compound **2a-2** (2.6 g, yellow solid) with a yield of 74%; LCMS(ESI): *m*/*z* 264.1 [M-H]⁺; RT=1.004 min (2.50 min).

### Step 2: Synthesis of 1,3-dimethyl-1H-pyrazolo[3,4-b]pyridin-4-ol

Compound **2a-2** (2.6 g, 9.81 mmol) was added to a single-necked bottle (100 mL) containing 50 mL of diphenyl ether, stirred at 230°C for 2 hours, the reaction solution was cooled to room temperature, petroleum ether (100 mL) was added, filtered, the solid was collected and purified using a silica gel column (DCM : methanol = 15:1) to obtain compound **2a-3** (0.8 g, white solid); yield: 50%; LCMS(ESI): *m*/*z* 164.2 [M+H]⁺; RT=0.680 min (2.50 min).

### Step 3: Synthesis of 4-chloro-1,3-dimethyl-1H-pyrazolo[3,4-b]pyridine

**2a-3** (0.7 g, 4.30 mmol) was added to a single-necked bottle (100 mL) containing 10 mL of POCl₃, and the reaction solution was stirred at 100°C for 2 hours. After concentrating the reaction solution, the pH was adjusted to 8 with saturated sodium bicarbonate aqueous solution in an ice bath, the reaction solution was extracted with dichloromethane, and the organic phase was washed sequentially with water and saturated salt water, dried with anhydrous sodium sulfate, filtered and concentrated to obtain compound **2a** (700 g, gray solid) with a yield of 91%; LCMS(ESI): *m*/*z* 182.1 [M+H]⁺; RT=1.540 min (2.50 min).

### Intermediate 3a: 4-chloro-7-methoxy-pyrido[2,3-d]pyrimidine

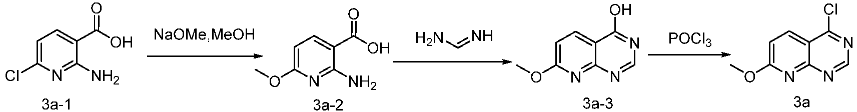

### Step 1: synthesis of 2-amino-6-methoxynicotinic acid

A compound **3a-1** (5.0 g, 29.0 mmol), sodium methoxide (15.7 g, 290 mmol) and methanol (50 mL) were successively added in a dry single-neck flask (100 mL) at room temperature. The mixture was heated to 80 °C and reacted for 48 h. The reaction solution was cooled to room temperature and filtered under reduced pressure, and the filter cake was washed with methanol and dried in vacuum to obtain a compound **3a-2** (2.0 g, white solid) with the yield of 40%. LCMS (ESI): *m*/*z* 169.0 [M+H]⁺; RT = 1.10 min (3 min).

### Step 2: synthesis of 7-methoxypyrido[2,3-d]pyrimidin-4-ol

A compound **3a-2** (1.0 g, 6.0 mmol), formamidine acetate (624 mg, 17.8 mmol) and ethylene glycol methyl ether (10 mL) were successively added to a dry single-neck flask (50 mL) at room temperature. The mixture was heated to 100°C and reacted for 18 h. The reaction solution was concentrated and purified (DCM:methanol = 10:1) to obtain a compound **3a-3** (200 mg, white solid) with the yield of 20%. LCMS (ESI): *m*/*z* 177.9 [M+H]⁺; RT = 1.502 min (3 min).

### Step 3: synthesis of 4-chloro-7-methoxypyrido[2,3-d]pyrimidine

A compound **3a-3** (200 mg, 1.13 mmol) and phosphorus oxychloride (10 mL) were successively added to a dry single-neck flask (50 mL) at room temperature. The mixture was heated to 100°C and reacted for 16 h. The reaction solution was concentrated, the residue was alkalified with a sodium bicarbonate solution to a pH of 9 and extracted with dichloromethane, and the organic phase was dried over sodium sulfate and concentrated to obtain a compound **3a** (200 mg, white solid) with the yield of 90%. LCMS (ESI): *m*/*z* 339.7 [M+H]⁺; RT = 1.521 min (3 min).

### Intermediate 4a: 4-chloro-7-methoxy-2-methylpyridino[2,3-d]pyrimidine

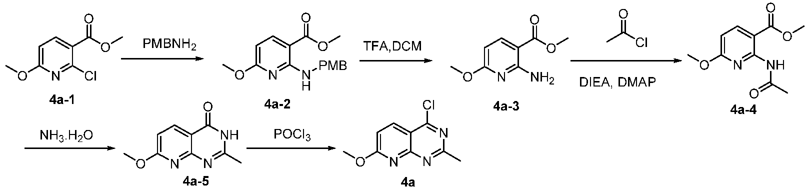

### Step 1: Synthesis of methyl 6-methoxy-2-((4-methoxybenzyl)amino)nicotinate

At room temperature, **4a-1** (5.0 g, 24.9 mmol), PMBNH₂ (4.1 g, 29.9 mmol), potassium carbonate (6.87 g, 49.8 mmol), and DMF (20 mL) were added sequentially to a dry single-necked bottle (100 mL). The reaction solution was heated to 80°C and reacted for 16 hours. After cooling to room temperature, the reaction solution was extracted with ethyl acetate and water, the organic phase was washed with salt water, concentrated, and purified (PE/EA = 2/1) to obtain compound **4a-2** (7g, colorless liquid) with a yield of 92%. LCMS (ESI): *m*/*z* 303.1[M+H]⁺; RT = 1.98 min (3 min).

### Step 2: Synthesis of methyl-2-amino-6-methoxynicotinate

At room temperature, compound **4a-2** (7.0 g, 24.0 mmol), dichloromethane (5 mL), and TFA (5 mL) were added sequentially to a dry single-necked bottle (100 mL) and reacted at room temperature for 16 hours. Concentration was carried out, the residue was diluted with dichloromethane, washed with sodium bicarbonate aqueous solution, the organic phase was dried with anhydrous sodium sulfate, filtered and concentrated, purified to obtain compound **4a-3** (3.5 g, colorless liquid) with a yield of 80%. LCMS (ESI): *m*/*z* 183.1[M+H]⁺; RT = 1.42 min (3 min).

### Step 3: Synthesis of methyl 2-acetamino-6-methoxynicotinate

At room temperature, compound **4a-3** (2.0 g, 11.0 mmol), DIEA (7.1 g, 55.0 mmol), DMAP (134 mg, 1.1 mmol), dichloromethane (20 mL), and acetyl chloride (1.7 g, 22.0 mmol) were added sequentially to a dry single-necked bottle (50 mL). The reaction solution was reacted at room temperature for 16 hours. Concentration was carried out, the residue was extracted with ethyl acetate and water, the organic phase was concentrated and purified (PE/EA = 10/1-1/1) to obtain compound **4a-4** (1.0 g, white solid). Yield: 45%. LCMS (ESI): *m*/*z* 224.8[M+H]⁺; RT = 1.541 min (3 min).

### Step 4: Synthesis of 7-methoxy-2-methylpyridino[2,3-J]pyrimidin-4(3H)-one

At room temperature, compound **4a-4** (1.0 g, 4.46 mmol), anhydrous methanol (10 mL), and ammonia solution (20 mL) were added sequentially to a dry single-necked bottle (50 mL). The reaction solution was reacted at room temperature for 16 hours. Concentration was carried out, the residue was extracted with ethyl acetate and water, the organic phase was concentrated, and then the residue was beaten with a small amount of ethyl acetate, filtered to obtain compound **4a-5** (200 mg, white solid) with a yield of 21%. LCMS (ESI): *m*/*z* 191.8[M+H]⁺; RT = 1.715 min (3 min).

### Step 5: Synthesis of 4-chloro-7-methoxy-2-methylpyridino[2,3-d]pyrimidine

At room temperature, compound **4a-5** (200 mg, 1.05 mmol) and phosphorus oxychloride (10 mL) were added sequentially to a dry single-necked bottle (50 mL). The reaction solution was heated to 100°C and reacted for 16 hours. Concentration was carried out, the residue was adjusted to pH=9 with sodium bicarbonate aqueous solution, extracted with ethyl acetate, the organic phase was dried, concentrated, and filtered to obtain compound **4a** (210 mg, white solid) with a yield of 90%. LCMS (ESI): *m*/*z* 209.8[M+H]⁺; RT = 2.44 min (5 min).

### Intermediate 5a: 7-chloro-1-methyl-1H-pyrazolo[4,3-d]pyrimidine

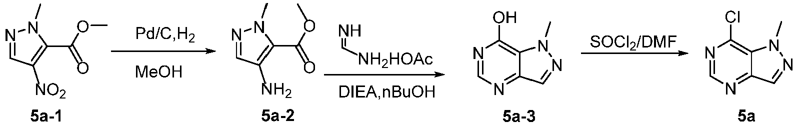

### Step 1: Synthesis of methyl 4-amino-1-methyl-1H-pyrazole-5-carboxylate

Compound **Sa-1** (2.00 g, 10.8 mmol) and palladium carbon (400 mg) were added to a single-necked bottle containing 30 mL of methanol, and stirred at room temperature under hydrogen pressure (15 psi) for 3 hours. After filtering, the filtrate was concentrated to obtain compound **5a-2** (1.60 g, white solid) with a yield of 95.6%. LCMS(ESI): *m*/*z* 156.1 [M+H]⁺, RT = 0.94 min (2.00 min).

### Step 2: Synthesis of 1-methyl-1H-pyrazolo[4,3-d]pyrimidin-7-ol

Compound **5a-2** (1.60 g, 10.3 mmol) and acetamidine acetate (1.29 g, 12.4 mmol) were added to a single-necked bottle containing n-butanol (15 mL) and DIEA (15 mL), and stirred at 110°C for 6 hours. The reaction solution was cooled to room temperature, filtered, and the solid was washed with ether to obtain compound **5a-3** (1.30 g, white solid) with a yield of 84.1%; LCMS(ESI): *m*/*z* 151.2 [M+H]⁺; RT=0.623 min (2.00 min).

### Step 3: Synthesis of 7-chloro-1-methyl-1H-pyrazolo[4,3-d]pyrimidine

Compound **5a-3** (1.30 g, 8.67 mmol) and DMF (0.5 mL) were added to a single-necked bottle containing 20 mL of dichlorosulfoxide, and stirred at 90°C for 2 hours. Dichlorosulfoxide was removed, dichloromethane and water were added to separate the solution. The aqueous phase was extracted with dichloromethane, the organic phase was washed with saturated salt water, dried with anhydrous sodium sulfate, and concentrated to obtain compound **5a** (1.20 g, white solid) with a yield of 82.4%; LCMS(ESI): *m*/*z* 169.0 [M+H]⁺; RT = 1.20 min (2.00 min).

### Intermediate 6a: 4-chloro-7-methoxy-1,6-naphthyridine

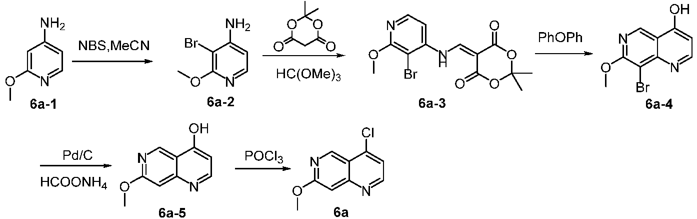

### Step 1: Synthesis of 3-bromo-2-methoxypyridin-4-amine

Compound **6a-1** (1.0 g, 8.06 mmol) was added to a single-necked bottle (100 mL) containing 10 mL of anhydrous acetonitrile. After cooling to 0°C, NBS (1.44 g, 8.06 mmol) was slowly added and stirred overnight at room temperature. The reaction solution was diluted with water, and extracted with ethyl acetate, and the organic phase was merged, washed sequentially with water and saturated salt water, dried with anhydrous sodium sulfate, filtered, concentrated and purified using a column (PE: EA = 8:1) to obtain compound **6a-2** (1.4 g, yellow solid) with a yield of 88%. LCMS(ESI): *m*/*z.* 203.0 [M+H]⁺; RT = 0.967 min (2.50 min).

### Step 2: Synthesis of 5-(((3-bromo-2-methoxypyridin-4-yl)amino)methylene)-2,2-dimethyl-1,3-dioxane-4,6-dione

2,2-dimethyl-1,3-dioxan-4,6-dione (709 mg, 4.92 mmol) was added to a single-necked bottle (100 mL) containing HC(OMe)₃ (10 mL), stirred at 100°C for 2 hours, compound **6a-2** (500 mg, 2.46 mmol) was added, and the reaction was continued for 2 hours. After cooling the reaction solution to room temperature, petroleum ether was added, the solid was precipitated, filtered, and collected to obtain compound **6a-3** (679 mg, white solid) with a yield of 77%. LCMS(ESI): *m*/*z* 357.0 359.0 [M+H]⁺; RT = 1.517 min (2.50 min).

### Step 3: Synthesis of 8-bromo-7-methoxy-1,6-naphthyridin-4-ol

Compound **6a-3** (3.0 g, 8.40 mmol) was added to a single-necked bottle (100 mL) containing 20 mL of diphenyl ether, stirred at 230°C for 1 hour, the reaction solution was cooled to room temperature, diluted with petroleum ether, filtered, and filter cake was collected to obtain compound **6a-4** (2.27 g, brown solid) with a yield of 100%. LCMS(ESI): *m*/*z* 255.0 257.0 [M+H]⁺; RT = 0.957 min (2.50 min).

### Step 4: Synthesis of 7-methoxy-1,6-naphthyridin-4-ol

Compound **6a-4** (2.0 g, 7.84 mmol), ammonium formate (980 mg, 15.68 mmol), and palladium carbon (300 mg, wt%: 10%) were added sequentially to a single-necked bottle (50 mL) containing 10 mL of methanol, and stirred at 60°C for 1 hour. The reaction solution was filtered, the filtrate was diluted with ethyl acetate, washed with water and saturated salt water in sequence, dried with anhydrous sodium sulfate, filtered and concentrated to obtain compound **6a-5** (1.4 g, yellow solid) with a yield of 100%. LCMS(ESI): *m*/*z* 177.1 [M+H]⁺, RT = 0.757 min (2.50 min).

### Step 5: Synthesis of 4-chloro-7-methoxy-1,6-naphthyridine

Compound **6a-5** (900 mg, 0.644 mmol) and DIEA (1.7 mL, 5.62 mmol) were added sequentially to a three-necked bottle (100 mL) containing anhydrous acetonitrile (10 mL). The reaction solution was cooled to 0°C under nitrogen protection, and then phosphorus oxychloride (0.9 mL, 5.62 mmol) was slowly added dropwise. After stirring at 70°C for 30 minutes, the reaction solution was cooled to room temperature. Saturated sodium bicarbonate was used to adjust the pH to 8. The reaction solution was extracted with ethyl acetate, and the organic phase was combined. The organic phase was washed sequentially with water and saturated salt water, dried with anhydrous sodium sulfate, filtered, concentrated, and purified on a column (PE: EA = 10:1) to obtain compound **6a** (1.0 g, yellow solid) with a yield of 100%. LCMS(ESI): *m*/*z* 195.1 [M+H]⁺; RT=1.287 min (2.50 min).

### Intermediate 7a: 8-chloro-3-methoxy-1,5-naphthyridine

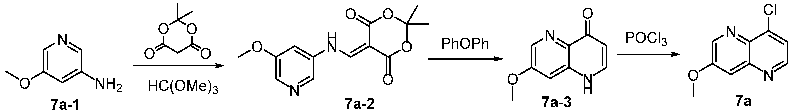

### Step 1: Synthesis of 5-((5-methoxypyridin-3-yl)amino)methylene)-2,2-dimethyl-1,3-dioxo-4,6-dione

2,2-dimethyl-1,3-dioxo-4,6-dione (4.64 g, 16.11 mmol) was added to a single-necked bottle (250 mL), and then trimethyl orthoformate (100 mL) was added. The reaction solution was stirred at 105°C for 2 hours. Compound **7a-1** (4.00 g, 16.11 mmol) was added and stirred overnight at 105°C. The reaction solution was diluted with petroleum ether at room temperature, filtered, and the filter cake was washed with petroleum ether. The filter cake is crude **7a-2** (7.0 g, yellow solid). LCMS (ESI): *m*/*z* 279.1[M-H]⁻; RT = 0.891 min (2.50 min).

### Step 2: Synthesis of 7-methoxy-1,5-naphthylidin-4(1H)-one

Compound **7a-2** (5.00 g, 17.97 mmol) was loaded into a three-necked bottle (250 mL), diphenyl ether (100 mL) was added, and stirred under nitrogen protection for 1 hour. A large amount of n-hexane was added at room temperature to dilute the reaction solution, which was filtered, and the filter cake was washed with n-hexane. The filter cake is crude **7a-3** (2.5 g, gray solid).

### Step 3: Synthesis of 8-chloro-3-methoxy-1,5-naphthyridine

Compound **7a-3** (3.50 g, 19.87 mmol) was loaded into a three-necked bottle (250 mL), phosphorus oxychloride (70 mL) was added, and stirred at 110°C for 12 hours. After cooling to room temperature, solvent was removed, then dichloromethane and a small amount of water were added. The pH was adjusted to 9-10 using sodium bicarbonate, and the reaction solution was extracted with ethyl acetate. The organic phase was washed with salt water, dried with anhydrous sodium sulfate, and the solvent was removed. The crude product was purified using a normal phase column (PE/EA = 3:1) to obtain compound **7a** (2.70 g, yellow solid). Yield: 70%. LCMS (ESI): *m*/*z* 195.1 [M+H]⁺; RT = 1.271 min (2.50 min).

### Intermediate 8a: 5-chloro-1-methyl-1,8-naphthyridin-2(1H)-one

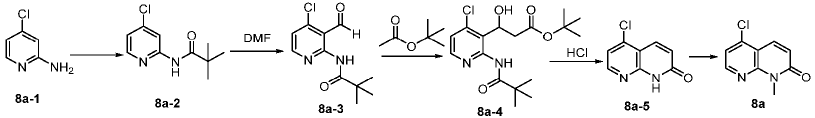

### Step 1: synthesis of N-(4-chloropyridin-2-yl)pivaloylindandione amide

A compound **8a-1** (1.28 g, 10.0 mmol), triethylamine (1.31 g, 13.0 mmol), pyridine (15 mL) and dichloromethane (15 mL) were successively added at room temperature in a dry 100-mL single-neck flask; and then tert-valeryl chloride (1.32 g, 11.0 mmol) was dropwise added under ice bath and reacted while stirring overnight. The reaction solution was concentrated under reduced pressure and the residue was purified by a silica gel column (PE:EA = 10:1) to obtain a product **8a-2** (1.85 g, pink solid) with the yield of 87.00%. ¹H-NMR(400 MHz, CDCl₃): 8.36-8.35 (m, 1H), 8.16-8.14 (m, 1H), 8.10 (s, 1H), 7.05-7.03 (m, 1H), 1.30 (s, 9H).

### Step 2: synthesis of N-(4-chloro-3-formylpyridin-2-yl)pivaloylindandione amide

A compound **8a-2** (5.7 g, 26.9 mmol) and tetrahydrofuran (70 mL) were successively added in a dry 250-mL three-neck flask at room temperature. N-butyllithium (27 mL, 2.5 M tetrahydrofuran) was dropwise added at -78°C under nitrogen protection and the reaction solution was stirred at -78°C for 0.5 h. A solution of N,N-dimethylformamide in tetrahydrofuran (30 mL) was added and continuously stirred at -78°C for 1 h. After the mixture was heated to room temperature, a saturated ammonium chloride solution (200 mL) was added, and the mixture was reacted at room temperature for 30 min. The reaction solution was extracted three times with EA (100 mL), and the organic phase was washed once with a saturated salt solution (50 mL), dried over anhydrous sodium sulfate, filtered, and concentrated under reduced pressure. The residue was purified by a silica gel column (PE:EA = 3:1) to obtain a product **8a-3** (2 g, yellow oil) with the yield of 31.00%. LCMS (ESI): *m*/*z* 240.8 [M+H]⁺; RT = 1.768 min (3.00 min).

### Step 3: synthesis of tert-butyl 3-(4-chloro-2-pivaloylindandione amidopyri din-3-yl)-3 -hydroxypropionate

A compound **8a-3** (2.28 g, 9.5 mmol) and tetrahydrofuran (40 mL) were successively added in a dry 250-mL three-neck flask at room temperature. Lithium diisopropylamide (10.5 mL, 2 M tetrahydrofuran) was dropwise added at -78°C under nitrogen protection, the reaction solution was stirred at -78°C for 0.5 h; and then tert-butyl acetate in tetrahydrofuran (20 mL) was added and continuously stirred at -78°C for 1 h. The reaction solution was heated to room temperature, water (40 mL) was added, the reaction solution was extracted with EA (50 mL), and the organic phase was washed once with a saturated salt solution (20 mL), dried over anhydrous sodium sulfate, filtered, and concentrated under reduced pressure. The residue was purified by a silica gel column (PE: EA = 2: 1) to obtain a product **8a-4** (2.2 g, yellow solid) with the yield of 65.00%. LCMS (ESI): *m*/*z* 356.7 [M+H]⁺; RT = 1.871 min (3.00 min).

### Step 4: synthesis of 5-chloro-1,8-naphthyridin-2(1H)-one

A compound **8a-4** (1.9 g, 5.34 mmol) and 3 M hydrochloric acid (20 mL) were successively added in a dry 50-mL single-neck flask at room temperature, and reacted at 100°C for 8 h, and the reaction solution was cooled and filtered, successively washed with a saturated sodium bicarbonate solution (5 mL) and water (5mL), and dried to obtain a product **8a-5** (440 mg, white solid) with the yield of 46.00%. LCMS (ESI): *m*/*z* 181.0 [M+H]⁺; RT = 1.20 min (3.00 min).

### Step 5: 5-chloro-1-methyl-1,8-naphthyridin-2(1H)-one

A compound **8a-5** (100 mg, 0.56 mmol), methyl iodide (102 mg, 0.72 mmol), potassium carbonate (154 mg, 1.12 mmol) and DMF (3 mL) were successively added in a dry 25-mL single-neck flask at room temperature. The reaction solution was stirred overnight at room temperature. Water (10 mL) was added in the reaction solution, the reaction solution was extracted with EA (20 mL), and the organic phase was washed with a saturated salt solution (10 mL), dried over anhydrous sodium sulfate, filtered, and concentrated under reduced pressure. The residue was purified by a silica gel column (PE:EA= 2: 1) to obtain a product **8a** (100 mg, white solid) with the yield of 92.60 %. LCMS (ESI): *m*/*z* 194.8 [M+H]⁺; RT = 1.621 min (3.00 min).

### Intermediate 9a: Synthesis of 4-chloro-7-methyl-1,7-naphthyridin-8-(7H)-one

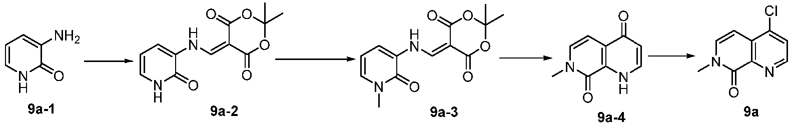

### Step 1: synthesis of 2,2-dimethyl-5-(((2-oxo-1,2-dihydropyridin-3-yl)amino)methylene)-1,3-dioxa-4,6-dione

2,2-dimethyl-1,3-dioxan-4,6-dione (2.36 g, 16.35 mmol) was added to a 100-mL single neck flask containing 20 mL of trimethyl orthoformate and stirred at 105 °C for 2 h under nitrogen protection. Then a compound **9a-1** (1.5 g, 13.62 mmol) was added and continuously reacted at the temperature for 2 h. After the reaction solution was cooled to room temperature, a solid was precipitated, the reaction solution was filtered, and a solid was collected to obtain a product **9a-2** (3.9 g, light grey white solid) with the yield of 100%; LCMS (ESI): *m*/*z* 263.1 [M-H]⁻; RT = 0.931 min (2.50 min).

### Step 2: synthesis of 2,2-dimethyl-5-(((1-methyl-2-oxo-1,2-dihydropyridin-3-yl)amino)methylene)-1,3-dioxa-4,6-d ione

**9a-2** (1.5 g, 5.68 mmol) and anhydrous potassium carbonate (4.32 g, 31.22 mmol) were successively added at room temperature in a 100-mL three-neck flask containing 30 mL of DMF, and methyl iodide (0.35 mL, 5.68 mmol) was slowly dropwise added thereto after cooling to 0°C. The reaction solution was stirred overnight at room temperature, filtered and concentrated under reduced pressure. The crude product was purified by column chromatography (dichloromethane:methanol = 20:1) to obtain **9a-3** (700 mg, yellow solid). LCMS (ESI): *m*/*z* 277.1 [M-H]⁺; RT = 0.831 min (2.50 min).

### Step 3: synthesis of 7-methyl-1,7-dihydro-1,7-naphthyridine-4,8-dione

**9a-3** (6.0 g, 21.58 mmol) was added to a 250-mL single neck flask containing 100 mL of diphenyl ether and stirred at 230°C for 2 h. After cooled to room temperature, the reaction solution was filtered and a filter cake was collected to obtain **9a-4** (3.1 g, brown solid) with the yield of 82%. LCMS (ESI): *m*/*z* 177.1 [M+H]⁺; RT = 0.617 min (2.50 min).

### Step 4: synthesis of 4-chloro-7-methyl-1,7-naphthyridin-8-(7H)-one

**9a-4** (3.1 g, 17.6 mmol) was added to a 100-mL single neck flask containing 30 mL of phosphorus oxychloride and stirred at 100°C for 1 h under nitrogen protection. After the reaction solution was cooled, the phosphorus oxychloride was removed, crushed ice was added at 0°C and the pH was adjusted to 8 with a saturated sodium bicarbonate, the reaction solution was extracted with dichloromethane, and the organic phase was washed successively with water and saturated sodium chloride, dried over anhydrous sodium sulfate, filtered and concentrated to obtain a product **9a** (2.1 g, brown solid). LCMS (ESI): *m*/*z* 195.1 [M+H]⁻; RT= 0.937 min (2.50 min).

### Example 1: Synthesis of compound 1

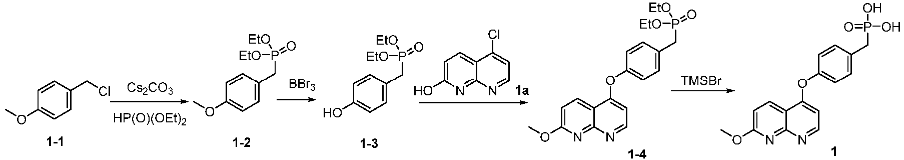

### Step 1: synthesis of diethyl(4-methoxybenzyl)phosphonate

A compound **1-1** (2.0 g, 12.8 mmol), triethyl phosphate (2.7 g, 19.2 mmol), cesium carbonate (8.4 g, 25.6 mmol) and dimethylformamide (60 mL) were successively added in a single neck flask containing 100 mL of methanol. The reaction was performed overnight at a room temperature. The reaction solution was quenched with water and extracted with dichloromethane, the organic phase was washed with a saturated sodium chloride solution and concentrated under reduced pressure, and the residue was purified by a silica gel column (PE:EA = 5:1) to obtain a compound **1-2** (2.5 g, oil). LCMS(ESI): *m*/*z* 259.0 [M+H]⁺; RT = 1.452 min (2.50 min).

### Step 2: synthesis of diethyl(4-hydroxybenzyl)phosphonate

The compound **1-2** (200 mg, 0.8 mmol) was added to a three-neck flask containing dry dichloromethane (3 mL) and under argon protection, 1.0 M of boron tribromide in dichloromethane (3.2 mL, 3.2 mmol) was dropwise added at -78°C. After the dropwise addition was complete, the reactant was heated to 0°C and stirred for 1 h. The reaction solution was quenched with saturated ammonium chloride and extracted with dichloromethane. The organic phase was washed with a saturated salt solution. The washed organic phase was concentrated under reduced pressure and the residue was purified by a silica gel column (PE:EA = 1:1) to obtain a compound **1-3** (100 mg, white solid). ¹H-NMR(400 MHz, CDCl₃-*d*): δ 7.05-7.02 (m, 2H), 6.63 (d, *J* = 8.4 Hz, 2H), 4.06-4.00 (m, 4H), 3.05 (d, *J* = 21.2 Hz, 2H), 1.28-1.25 (m, 6H).

### Step 3: synthesis of diethyl(4-((7-methoxy-1,8-naphthyridin-4-yl)oxy)benzyl)phosphonate

The compound **1-3** (100 mg, 0.4 mmol), a compound **1a** (78 mg, 0.4 mmol), cesium carbonate (261 mg, 0.8 mmol), Pd₂dba₃ (catalytic amount) and Xphos (catalytic amount) were added to a single neck flask containing 3 mL of dioxane. The reaction was performed while stirring overnight at 100°C under nitrogen protection. The reaction solution was cooled to room temperature, filtered and concentrated, and the residue was purified by Prep-HPLC to obtain a compound **1-4** (21 mg, off-white solid) with the yield of 13.0%. LCMS(ESI): *m*/*z* 403.15 [M+H]⁺; RT = 2.323 min (6.00 min). ¹H-NMR(400 MHz, MeOD-*d*₄): δ 8.62 (d, *J =* 8.8 Hz, 2H), 7.48 (dd, *J* = 8.4, 2.4 Hz, 2H), 7.22 (d, *J* = 8.4 Hz, 2H), 7.11 (d, *J* = 8.8 Hz, 1H), 6.59 (d, *J=* 6.4 Hz, 1H), 4.20-4.00 (m, 7H), 3.35 (s, 2H), 1.29 (t, *J=* 7.2 Hz, 6H).

### Step 4: synthesis of (4-((7-methoxy-1,8-naphthyridin-4-yl)oxy)benzyl)phosphonate

The compound **1-4** (75 mg, 0.19 mmol) and trimethyl bromosilane (0.12 mL, 0.93 mmol) were added to a single neck flask containing acetonitrile (5 mL) and reacted while stirring at room temperature for 16 h. The reaction solution was concentrated and separated and purified by a high-performance solution phase reverse-phase preparative column to obtain a compound 1 (5.15 mg, white solid) with the yield of 7.98%. LCMS(ESI): *m*/*z* 347.00 [M+H]⁺; RT = 3.262 min (6.00 min). ¹H-NMR(400 MHz, MeOD-*d₄*): δ 8.83-8.77 (m, 2H), 7.56 (d, *J* =5.6 Hz, 2H), 7.39 (d, *J* =9.2 Hz, 1H), 7.30 (d, *J* = 6.0 Hz, 2H), 6.99 (d, *J* = 9.2 Hz, 1H), 4.22 (s, 3H), 3.24-3.16 (m, 2H).

### Examples 2-6: Synthesis of compounds 2-6

The synthetic method was the same as that of the compound 1 in example 1, except that 4-chloromethyl-2-fluoro-1-methoxybenzene, 4-chloromethyl-2,6-difluoro-1-methoxybenzene, 4-chloromethyl-5-fluoro-1-methoxybenzene, 4-chloromethyl-3,5-difluoro-1-methoxybenzene, and 4-chloromethyl-2,6-difluoro-1-methoxybenzene were used to replace the starting compound 1-1 in example 1 respectively. The structural formulae of the compounds of each example referred to the previous table. Data of the compounds of examples 2-6 was as follows.

| Example | LCMS (ESI): [M+H]⁺ | ¹H NMR (400 MHz) |
|---|---|---|
| 2 | *m*/*z* 364.9 | (MeOD-d₄): δ 8.76-8.72 (m, 2H), 7.40-7.27 (m, 4H), 6.87 (d, *J* = 6.4 Hz, 1H), 4.18 (s, 3H), 3.14 (d, *J* = 21.2 Hz, 2H). |
| 3 | *m*/*z* 382.9 | (DMSO-d₆): δ 8.81 (d, *J* = 6.0 Hz, 1H), 8.71 (d, *J* = 9.2 Hz, 1H), 7.32-7.26 (m, 3H), 6.74 (d, *J* = 5.6 Hz, 1H), 4.08 (s, 3H), 3.12 (d, *J* = 21.2 Hz, 2H). |
| 4 | *m*/*z* 364.9 | (MeOD-d₄): δ 8.77-8.75 (m, 2H), 7.66-7.61 (m, 1H), 7.33 (d, *J* = 5.2 Hz, 1H), 7.20-7.12 (m, 2H), 6.99 (d, *J* = 6.4 Hz, 1H), 4.20 (s, 3H), 3.19 (d, *J* = 21.2 Hz, 2H). |
| 5 | *m*/*z* 382.9 | (DMSO-d₆): δ 8.77 (d, *J* = 5.2 Hz, 1H), 8.53 (d, *J* = 8.8 Hz, 1H), 7.18-7.15 (m, 3H), 6.75 (d, *J* = 5.6 Hz, 1H), 4.04 (s, 3H), 2.99 (d, *J* = 20.4 Hz, 2H). |
| 6 | *m*/*z* 382.9 | (DMSO-d₆): δ 8.75 (d, J = 5.6 Hz, 1H), 8.61 (d, *J* = 8.8 Hz, 1H), 7.56-7.47 (m, 2H), 7.19 (d, *J* = 9.2 Hz, 1H), 6.64 (d, *J* = 5.2 Hz, 1H), 4.04 (s, 3H), 3.05 (d, *J* = 21.2 Hz, 2H). |

### Example 7: Synthesis of compound 7

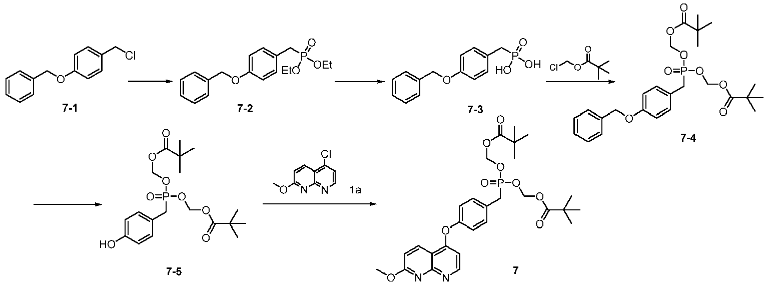

### Step 1: synthesis of diethyl(4-(benzyloxy)benzyl)phosphonate

A compound **7-1** (3.0 g, 12.92 mmol) was added to a 100-mL single neck flask and triethyl phosphite (20 mL) was added at room temperature. The reaction was performed while stirring at 150°C for 6 h under nitrogen protection. The reaction solution was concentrated. The residue was purified by a silica gel column (PE:EA = 1:1) to obtain a compound **7-2** (4.18 g, yellow oil) with the yield of 97%. LCMS (ESI): *m*/*z* 357.1 [M+Na]⁺; RT = 1.441 min (2.50 min).

### Step 2: synthesis of (4-(benzyloxy)benzyl)phosphonate

The compound **7-2** (4.80 g, 14.35 mmol) was added to a 250-mL single neck flask containing chloroform (50 mL). Trimethylsilyl bromide (20 mL) was added at room temperature and stirred overnight at room temperature, and the reaction solution was quenched with ethanol, diluted with dichloromethane, and concentrated under reduced pressure. The residue was pulped with PE:EA = 5:1 (50 mL) and filtered, and the filter cake was washed (PE:EA = 4:1, 10 mL) and purified to obtain a compound **7-3** (2.80 g, white solid) with the yield of 70%. ¹H NMR (400 MHz, DMSO-*d₆*): δ 7.44-7.30 (m, 5H), 7.15-7.12 (m, 2H), 6.91-6.90 (d, *J* = 8.4 Hz, 2H), 5.06 (s, 2H), 2.86 (d, *J* = 21.6 Hz, 2H).

### Step 3: synthesis of ((4-(benzyloxy)benzyl)(methylene)-l5 phosphinodioyl)bis(oxy)bis(methylene)bis(2,2-dimethylpropanoic acid)

The compound **7-3** (200 mg, 0.72 mmol), N-methylpyrrolidinone (5 mL), chloromethyl tervalerate (0.62 mL, 4.31 mmol) and N,N-diisopropylethylamine (0.71 mL, 4.31 mmol) were successively added at room temperature in a dry 25-mL single neck flask. The reaction was performed at 75°C for 16 h. After the reaction was completed, the reaction solution was poured into 20 mL and extracted with ethyl acetate. The organic phases were combined, washed with a saturated salt solution, dried over anhydrous sodium sulfate, and filtered. The filtrate was concentrated under reduced pressure. The crude product was purified using a silica gel chromatographic column (PE:EA = 1:1) to obtain a compound **7-4** (320 mg, yellow oil) with the yield of 87.89%. ¹H-NMR(400 MHz, CDCl₃): δ 7.43-7.30 (m, 5H), 7.20 (dd, *J =* 2.8 Hz, 8.8 Hz, 2H), 6.92 (d, *J* = 8.4 Hz, 2H), 5.62-5.56 (m, 4H), 5.04 (s, 2H), 3.17 (d, *J* = 21.6 Hz, 2H), 1.21 (s, 18H).

### Step 4: synthesis of ((4-(hydroxybenzyl)methylene)-l5-phosphinodioyl)bis(oxy)bis(methylene)bis(2,2-dimethylpr opanoic acid)

The compound **7-4** (480 mg, 0.95 mmol), ethyl acetate (30 mL) and a palladium/carbon catalyst (48 mg) were successively added in a dry 100-mL single neck flask at room temperature. The reaction was performed at room temperature for 1.5 h. The reaction solution was filtered and the filtrate was concentrated under reduce pressure to obtain **7-5** (390 mg and colorless oil) with the yield of 98.84%. ¹H-NMR(400 MHz, CDCl₃-d ): δ 7.05 (dd, *J* = 2.8 Hz, 8.4 Hz, 2H), 6.96 (s, 1H), 6.65 (d, *J* = 8.0 Hz, 2H), 5.64-5.58 (m, 4H), 3.15 (d, *J =* 21.6 Hz, 2H), 1.22 (s, 18H).

### Step 5: synthesis of (((4-((7-methoxy-1,8-naphthyridin-4-yl)oxy)benzyl)(methylene)-l5-phosphinodioyl)bis(oxy)b is(methylene)bis(2,2-dimethylpropanoic acid)

The compound **7-5** (86 mg, 0.21 mmol), toluene (5 mL), the compound **1a** (40 mg, 0.21 mmol), potassium phosphate (87 mg, 0.41 mmol), Xphos (9 mg, 0.02 mmol) and Pd₂dba₃ (19 mg, 0.02 mmol) were successively added at room temperature in a dry 25-mL single neck flask. The mixture was heated to 100°C and the reaction was performed for 16 h under nitrogen protection. After the reaction solution was filtered and concentrated, the residue was purified by prep-HPLC to obtain a compound 7 (8.5 mg, colorless oil) with the yield of 7.20%. LCMS (ESI): m/z 575.25 [M+H]⁺; RT = 4.181 min (6.00 min). ¹H-NMR(400 MHz, DMSO-*d₆*): δ 8.71 (d, *J=* 5.6 Hz, 1H), 8.58 (d, *J=* 8.8 Hz, 1H), 7.42 (d, *J* = 7.2 Hz, 2H), 7.26 (d, *J =* 8.0 Hz, 2H), 7.17 (d, *J* =8.8 Hz, 1H), 6.57 (d, *J* = 5.6 Hz, 1H), 5.64-5.57 (m, 4H), 4.05 (s, 3H), 3.45 (d, *J* = 21.6 Hz, 2H), 1.17 (s, 18H).

### Examples 8 and 9: Synthesis of compounds 8 and 9

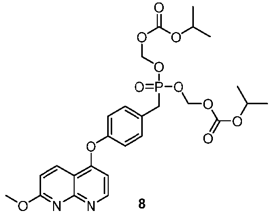

Compounds **8** and **9** were synthesized referring to the synthetic method of example 7.

Compound **8:** LCMS(ESI): *m*/*z* 579.0 [M+H]⁺; RT = 3.957 min (6.00 min). ¹H-NMR (400 MHz, DMSO-*d₆*): δ 8.70 (d, J= 5.6 Hz, 1H), 8.57 (d, *J* = 8.8 Hz, 1H), 7.42-7.40 (m, 2H), 7.26 (d, *J* = 8.8 Hz, 2H), 7.17 (d, *J* = 8.8 Hz, 1H), 6.57 (d, *J* = 5.6 Hz, 1H), 5.61-5.56 (m, 4H), 4.85-4.82 (m, 2H), 4.05 (s, 3H), 3.50 (d, *J* = 22.4 Hz, 2H), 1.25 (d, *J* = 6.4 Hz, 12H).

Compound **9:** LCMS(ESI): *m*/*z* 615.5 [M+H]⁺.

### Example 10: Synthesis of compound 10

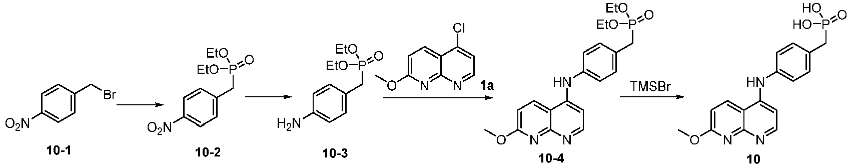

### Step 1: synthesis of diethyl(4-nitrobenzyl)phosphonate

P-nitrobenzyl bromide (2.5 g, 11.6 mmol), triethyl phosphite (4.8 g, 34.9 mmol), and toluene (30 mL) were successively added to a dry 100-mL single neck flask at room temperature, and heated to 100°C overnight. The reaction solution was concentrated under reduced pressure. The residue was purified by a silica gel column (PE:EA = 1:1) to obtain a compound **10-2** (2.5 g, yellow solid) with the yield of 81.00%. ¹H-NMR(600 MHz, CDCl₃): 8.19-8.18 (m, 2H), 7.48-7.46 (m, 2H), 4.07-4.04 (m, 4H), 3.25 (d, *J* = 22.2 Hz, 2H), 1.27 (t, *J* = 7.2 Hz, 6H).

### Step 2: synthesis of diethyl(4-amidobenzyl)phosphonate

The compound **10-2** (4 g, 14.6 mmol), 10% palladium on carbon (400 mg) and ethanol (30 mL) were successively added in a dry 100-mL single neck flask at room temperature, and stirred overnight under hydrogen protection. The reaction solution was concentrated under reduced pressure. The residue was purified by a silica gel column (EA) to obtain a product **10-3** (3 g, yellow solid) with the yield of 84.00%. LCMS (ESI): *m*/*z* 244.1 [M+H]⁺; RT = 1.10 min (3.00 min).

### Step 3: synthesis of diethyl(4-((7-methoxy-1,8-naphthyridin-4-yl)amino)benzyl)phosphonate

The compound **10-3** (130 mg, 0.53 mmol), the compound **1a** (104 mg, 0.53 mmol), Pd₂dba₃ (48 mg, 0.053 mmol), Xphos (33 mg, 0.053 mmol), potassium carbonate (345 mg, 1.06 mmol) and dioxane (10 mL) were successively added at room temperature in a dry 50-mL single neck flask. The mixture was heated to 100°C and stirred overnight under nitrogen protection. The reaction solution was concentrated under reduced pressure. The residue was purified by a silica gel column (dichloromethane:methanol = 30:1) to obtain a product **10-4** (150 mg, yellow solid) with the yield of 7.0%. LCMS (ESI): *m*/*z* 402.1 [M+H]⁺; RT = 1.19 min (3.00 min).

### Step 4: synthesis of (4-((7-methoxy-1,8-naphthyridin-4-yl)amino)benzyl)phosphonate

The compound **10-4** (150 mg, 0.37 mmol) and chloroform (5 mL) were added to a 25-mL three-neck flask. Under nitrogen protection, trimethyl bromosilane (1 M dichloromethane solution) (0.37 mL, 0.37 mmol) was dropwise added under ice bath and the reaction was performed while stirring at room temperature for 2 h. The reaction solution was concentrated and separated and purified by a high-performance solution phase reverse-phase preparative column to obtain a compound **10** (30 mg, white solid). LCMS (ESI): m/z 345.9 [M+H]⁺; RT = 3.26 min (5.00 min). ¹H-NMR(400 MHz, DMSO-*d₆*): 9.02 (d, J *=* 9.2 Hz, 1H), 8.32 (d, *J* = 7.2 Hz, 1H), 7.46-7.38 (m, 4H), 7.30 (d, J *=* 9.2 Hz, 1H), 6.79 (d, *J* = 7.2 Hz, 1H), 4.10 (s, 3H), 3.10 (d, *J* = 21.6 Hz, 2H).

### Example 11: Synthesis of compound 11

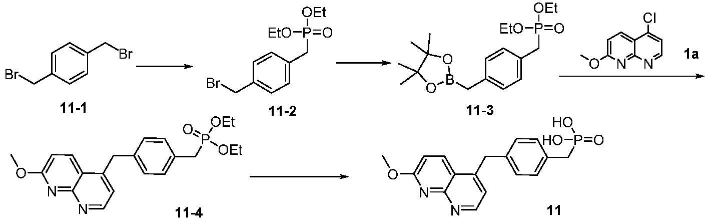

### Step 1: synthesis of diethyl 4-bromomethylbenzylphosphite

1,4-bis(bromomethyl)benzene (1 g, 3.8 mmol), triethyl phosphite (318 mg, 1.9 mmol), and N,N-dimethylformamide (2 mL) were successively added in a dry 50-mL single neck flask at room temperature. The reaction was performed at 150°C for 2 min under microwave. The reaction solution was extracted three times with water (10 mL) and ethyl acetate (20 mL), the organic phases were combined and washed with water (10 mL) and a salt solution (10 mL), and the combined organic phase was dried and concentrated under reduced pressure. The residue was purified by a silica gel column (PE:EA = 20:1) to obtain a product 11-2 (800 mg, yellow solid) with the yield of 66%. LCMS (ESI): m/z 322.9 [M+H]+; RT = 1.55 min (3.00 min).

### Step 2: synthesis of diethyl(4-((4,4,5,5-tetramethyl-1,3,2-dioxabenzaldehyde-2-yl)methyl)benzyl)phosphonate

The compound **11-2** (1.1 g, 3.44 mmol), bis(pinacolato)diboron (1.31 g, 5.16 mmol), potassium acetate (842 mg, 8.59 mmol) and 1,1-bis(diphenylphosphino)ferrocene palladium dichloride (catalytic amount) were successively added in a 100-mL three-neck flask containing 15 mL of dioxane at room temperature. The reaction was performed at 100°C for 10 h under the nitrogen environment. The reaction solution was filtered and the filtrate was concentrated under reduced pressure. The residue was purified by a column (PE:EA = 3:1) to obtain a compound **11-3** as a crude product (520 mg, yellow oil) with the yield of 38.1%. LCMS(ESI): m/z 369.2 [M+H]⁺; RT = 1.047 min (2.50 min).

### Step 3: synthesis of diethyl(4-((7-methoxy-1,8-naphthyridin-4-yl)methyl)benzyl)phosphonate

The compound **11-3** (420 mg, 1.14 mmol), 5-chloro-2-methoxy-1,8-naphthidine (221 mg, 1.14 mmol), potassium carbonate (394 mg, 2.85 mmol) and 1,1-bis(diphenylphosphino)ferrocene palladium dichloride (catalytic amount) were added in a 50-mL three-neck flask containing 9 mL of dioxane and 3 mL of water. The reaction was performed at 100°C for 3 h under nitrogen protection. After cooled, the reaction solution was diluted with water and extracted 3 times with ethyl acetate, and the organic phase was washed twice with saturated sodium chloride, dried and filtered. After the organic phase was concentrated under reduced pressure, the residue was purified using prep-TLC (PE/EA = 2/1) to obtain a compound **11-4** (430 mg, yellow oil) with the yield of 76.1%. LCMS (ESI): *m*/*z* 401.0 [M+H]⁺; RT = 1.024 min (2.50 min).

### Step 4: synthesis of (4-((7-methoxy-1,8-naphthyridin-4-yl)methyl)benzyl)phosphonate

The compound **11-4** (100 mg, 0.25 mmol) and trimethyl bromosilane (191 mg, 1.25 mmol) were successively added in a 50-mL single neck flask containing 6 mL of chloroform at room temperature, and reacted overnight at room temperature. The reaction solution was quenched with methanol, concentrated and purified by a prep-HPLC (trifluoroacetic acid system) to obtain a compound **11** (45.0 mg, white solid) with the yield of 52.3%. LCMS (ESI): *m*/*z* 344.9 [M+H]⁺; RT = 3.305 min (6.00 min). ¹H NMR (400 MHz, DMSO-*d₆*): δ 8.92 (d, *J* = 5.2 Hz, 1H), 8.69 (d, *J* = 9.2 Hz, 1H), 7.51 (d, *J* = 4.8 Hz, 1H), 7.27 (d, *J* = 9.2 Hz, 1H), 7.18 (s, 4H), 4.52 (s, 2H), 4.06 (s, 3H), 2.90 (d, *J* = 21.6 Hz, 2H).

### Examples 12-56 and 58-69: Synthesis of compounds 12-56 and 58-69

Compounds **12-56** and **58-69** were synthesized with reference to the synthetic methods of the compounds **1 7, 10** and **11** respectively. The data of each compound was as follows:

| Example | LCMS (ESI): [M+H]⁺ | ¹H NMR (400 MHz) |
|---|---|---|
| 12 | *m*/*z* 345.9 | (MeOD-d4): δ 9.61 (s, 1H), 8.27 (d, J = 7.6 Hz, 1H), 7.55-7.53 (m, 2H), 7.43 (d, J = 8.0 Hz, 2H), 7.03 (s, 1H), 6.76 (d, J = 7.2 Hz, 1H), 4.13 (s, 3H), 3.23 (d, J = 21.6 Hz, 2H). |
| 13 | m/*z 347.0* | (DMSO-d₆): δ 10.41 (s, 1H), 8.89 (d, J = 8.8 Hz, 1H), 8.72 (s, 1H), 7.62 (d, J = 8.4 Hz, 2H), 7.32-7.30 (m, 2H), 7.24 (d, J = 9.2 Hz, 1H), 4.05 (s, 3H), 3.02-2.97 (m, 2H). |
| 15 | *m*/*z* 319.9 | (DMSO-d₆): δ 8.38 (d, *J* = 5.2 Hz, 1H), 7.70 (s, 1H), 7.39 (d, *J* = 6.8 Hz, 2H), 7.22 (d, *J* = 8.0 Hz, 2H), 6.46 (d, *J* = 5.2 Hz, 1H), 4.03 (s, 3H), 3.06-3.00 (m, 2H). |
| 20 | *m*/*z* 347.0 | (DMSO-d₆): δ 9.05 (d, *J* = 9.2 Hz, 1H), 8.75 (d, *J* = 2.4 Hz, 1H), 8.44 (d, *J* = 7.2 Hz, 1H), 8.15-8.13 (m, 1H), 7.75-7.72 (m, 1H), 7.38 (d, *J* = 9.2 Hz, 4H), 6.91 (d, *J* = 7.2 Hz, 1H), 4.11 (s, 3H), 3.45-3.39 (m, 2H). |
| 21 | *m*/*z* 347.1 | (DMSO-d₆): δ 8.16-8.13 (m, 2H), 7.95 (s, 2H), 7.42 (d, *J* = 9.2 Hz, 1H), 6.99 (d, *J* = 6.8 Hz, 1H), 6.76 (d, *J* = 8.8 Hz, 1H), 3.58 (s, 3H), 2.90 (d, J = 21.2 Hz, 2H). |
| 23 | *m*/*z* 361.1 | (MeOD-d₄): δ 8.83 (d, *J* = 9.2 Hz, 1H), 8.77 (d, *J* = 6.8 Hz, 1H), 7.51 (d, *J* = 8.8 Hz, 2H), 7.41 (d, *J* = 9.2 Hz, 1H), 7.32-7.30 (m, 2H), 6.97 (d, *J* = 6.8 Hz, 1H), 4.23 (s, 3H), 3.04-2.98 (m, 2H), 2.12-2.03 (m, 2H). |
| 24 | *m*/*z* 360.9 | (DMSO-d₆): δ 9.61 (s, 1H), 8.76 (d, *J* = 8.8 Hz, 1H), 7.68-7.67 (m, 2H), 7.24 (s, 2H), 6.98 (d, *J* = 9.2 Hz, 1H), 3.97 (s, 3H), 2.88-2.82 (m, 2H), 2.47 (s, 3H). |

| Example | LCMS (ESI): [M+H]⁺ | Example | LCMS (ESI): [M+H]⁺ | Example | LCMS (ESI): [M+H]⁺ |
|---|---|---|---|---|---|
| 13 | *m*/*z* 347.1 | 14 | *m*/*z* 346.1 | 16 | *m*/*z* 574.2 |
| 17 | *m*/*z* 578.2 | 18 | *m*/*z* 574.2 | 19 | *m*/*z* 578.2 |
| 21 | *m*/*z* 347.1 | 22 | *m*/*z* 347.1 | 23 | *m*/*z* 361.1 |
| 25 | *m*/*z* 348.1 | 26 | *m*/*z* 364.1 | 27 | *m*/*z* 364.1 |
| 28 | *m*/*z* 360.1 | 29 | *m*/*z* 380.7 | 30 | *m*/*z* 378.3 |
| 31 | *m*/*z* 389.3 | 32 | *m*/*z* 397.3 | 33 | *m*/*z* 333.3 |
| 34 | *m*/*z* 321.1 | 35 | *m*/*z* 579.5 | 36 | *m*/*z* 597.5 |
| 37 | *m*/*z* 615.5 | 38 | *m*/*z* 614.2 | 39 | *m*/*z* 628.2 |
| 40 | *m*/*z* 382.1 | 41 | *m*/*z* 382.1 | 42 | *m*/*z* 382.1 |
| 43 | *m*/*z* 364.1 | 44 | *m*/*z* 364.1 | 45 | *m*/*z* 382.1 |
| 46 | *m*/*z* 382.1 | 47 | *m*/*z* 382.1 | 48 | *m*/*z* 592.2 |
| 49 | *m*/*z* 592.2 | 50 | *m*/*z* 610.2 | 51 | *m*/*z* 610.2 |
| 52 | *m*/*z* 610.2 | 53 | *m*/*z* 596.2 | 54 | *m*/*z* 596.2 |
| 55 | *m*/*z* 614.2 | 56 | *m*/*z* 614.2 | 58 | *m*/*z* 346.1 |
| 59 | *m*/*z* 346.1 | 60 | *m*/*z* 347.1 | 61 | *m*/*z* 347.1 |
| 62 | *m*/*z* 574.2 | 63 | *m*/*z* 574.2 | 64 | *m*/*z* 575.2 |
| 65 | *m*/*z* 575.2 | 66 | *m*/*z* 578.2 | 67 | *m*/*z* 578.2 |
| 68 | *m*/*z* 579.2 | 69 | *m*/*z* 579.2 | | |

### Example 57: Synthesis of compound 57

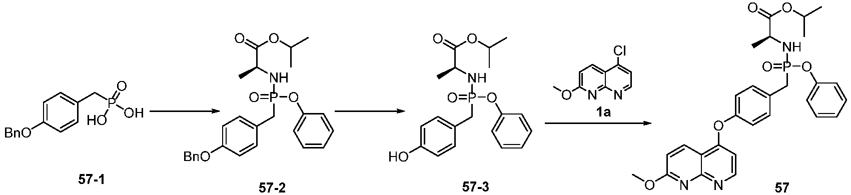

### Step 1: synthesis of isopropyl((4-(benzyloxy)benzyl)(phenoxy)phosphoryl)-L-alanine ester

A compound **57-1** (700 mg, 2.52 mmol) and N,N-dimethylformamide (catalytic amount) were added in a 50-mL three-neck flask containing 10 mL of anhydrous dichloromethane and heated to 40°C, and a solution of oxalyl chloride (0.47 mL, 5.54 mmol) in anhydrous dichloromethane was slowly dropwise added under nitrogen protection. The reaction was performed while stirring at the temperature for 3 h, the temperature was reduced to 30°C and the reaction was performed overnight. The prepared acyl chloride solution was slowly added to a dry tetrahydrofuran solution containing phenol (592 mg, 6.3 mmol) and triethylamine (636 mg, 6.3 mmol) in an ice bath and stirred at room temperature for 2 h. The previous step was repeated. The reaction solution was continuously dropwise added to a dry tetrahydrofuran solution containing isopropyl L-alaninate (464 mg, 2.77 mmol) and triethylamine (390 mg, 3.02 mmol) and reacted overnight at room temperature. The reaction solution was filtered and concentrated to obtain a crude product which was purified by a column (PE:EA = 1:1) to obtain a compound **57-2** (140 mg, yellow solid) with the yield of 11.9%. LCMS(ESI): m/z 468.2 [M+H]⁺; RT = 1.296 min (2.50 min).

### Step 2: synthesis of isopropyl((4-hydroxybenzyl(phenoxy)phosphoryl)-L-alanine ester

The compound **57-2** (0.14 g, 0.30 mmol) and 10% wet palladium on carbon (14 mg) were successively added in a 50-mL single neck flask containing 10 mL of ethyl acetate and reacted at room temperature for 2 h under hydrogen protection. The reaction solution was filtered and the filtrate was concentrated to obtain a compound **57-3** (130 mg, yellow oil) with the purity of 60%. LCMS(ESI): m/z 378.1 [M+H]⁺; RT = 1.179 min (2.50 min).

### Step 3: synthesis of isopropyl((4-((7-methoxy-1,8-naphthyridin-4-yl)oxy)benzyl)(phenoxy)phosphoryl)-L-alanine ester

The compound **57-3** (65 mg, 0.17 mmol), **1a** (35 mg, 0.17 mmol), anhydrous potassium phosphate (73 mg, 0.34 mmol), Pd₂dba₃ (catalytic amount) and 2-di-tert-butylphosphino-2',4',6'-triisopropylbiphenyl (catalytic amount) were successively added in a 50-mL three-neck flask containing 6 mL of anhydrous toluene, and reacted overnight at 100°C under argon protection. The reaction solution was filtered, and the filtrate was concentrated and purified by Prep-HPLC (formic acid system) to obtain a compound 57 (9.75 mg, white solid) with the yield of 5.3%. LCMS(ESI): m/z 536.1 [M+H]⁺; RT = 3.821 min (6.00 min). ¹H-NMR(400 MHz, MeOH-*d₄*): δ 8.61 (d, *J =* 5.6 Hz, 2H), 7.56-7.53 (m, 2H), 7.34-7.31 (m, 2H), 7.24-7.09 (m, 6H), 6.63-6.59 (m, 1H), 4.95-4.90 (m, 1H), 4.12 (s, 3H), 3.88-3.86 (m, 1H), 3.51-3.40 (m, 2H), 1.22-1.15 (m, 9H).

### Biological Example

### Experimental Example 1: Evaluation of ENPP1 Inhibitory Activity of Compounds

The ENPP1 inhibitory activity of the compound of the present disclosure was tested in substrate 2',3'-cGAMP detection.

**Experimental objective:** based on the established experimental method, the inhibitory IC₅₀ value of the compound in the present application on ENPP1 was detected using 2',3'-cGAMP as the substrate. STF-32 was used as a positive control compound (STF-32 is derived from literature: Cell Chemical Biology 2020 (27), 1347-1358).

**Experimental reagent:** hENPP1-ECD-His (ChemPartner, cat. 202103121201); AMP-Glo^{™} Assay Kit (Promega, cat: V5011); DMSO (Sigma, cat. D8418-1L); 384-well white color board (PerkinElmer, cat. 6007290), 2'3'- cGAMP (MCE, cat. HY-100564A).

### Experimental method:

1. Preparation of 1 × reaction solution: 50 mM Tris-HCl (pH 7.5), 10 mM NaCl, 0.5 mM CaCl₂, 1 µM ZnCl₂, 0.01% Tween-20, 0.01% BSA.
2. Preparation of compound concentration gradient: the tested compound was dissolved in DMSO (dimethyl sulfoxide) to an initial concentration of 10 µM, diluted at 3 times, with 10 concentrations, single well or multi-well testing was set. The initial concentration in the positive control compound STF-32 test was 1 µM, diluted at 3 times, with 10 concentrations, and multi-well testing was set for each concentration. The compound was diluted by gradient in a 384 well plate to a solution with the final concentration of 1000 times, and then 5 nL of the solution was transferred to a 384 well reaction plate for testing using Echo550. 5 nL of 100% DMSO was transferred from the smallest well and the largest well.
3. 20 nM of 2 × protein solution was prepared using 1 × reaction solution.
4. 40 µM of 2 × substrate solution was prepared using 1 × reaction solution.
5. 2.5 µL of 2 × protein solution was added to each compound well and the maximum pore of the reaction plate, and 2.5 µL of 1 × reaction solution was added to the smallest well.
6. The reaction solution was centrifuged at 1000 rpm for 1 minute and incubated at room temperature for 15 minutes.
7. 2.5 µL 2 × substrate solution was added to each well of the reaction plate, centrifuged at 1000 rpm for 1 minute, and incubated at room temperature for 60 minutes.
8. 5 µL of R1 solution (from AMP-Glo^{™} Kit) was added to each well of the reaction plate, centrifuged at 1000 rpm for 1 minute and incubated at room temperature for 120 minutes.
9. 10 µL of R2 solution (from AMP-Glo^{™} Kit) was added to each well of the reaction plate, centrifuged at 1000 rpm for 1 minute and incubated at room temperature for 30 minutes.
10. A multifunctional enzyme-linked immunosorbent assay reader (2104EnVision) was used to detect and the test results were recorded.

### Data analysis

The inhibition rate is calculated using the following formula: inhibition rate% = (maximum signal - compound signal)/(maximum signal - minimum signal) × 100 wherein the "minimum signal" represents the mean value of the negative control well, and the "maximum signal" represents the mean value of the positive control well.

### Fitting the dose-response curve:

Using the log value of concentration as the X-axis and the percentage inhibition rate as the Y-axis, the dose-response curve was fitted using the log(inhibitor) vs. response-variable slope of analysis software GraphPadPrism5 to obtain the inhibitory IC₅₀ value of the compound of the present disclosure on enzyme activity.

The fitting formula is: Y = bottom + (top - bottom) / (1 + 10^((logIC₅₀ - X) * HillSlope)),

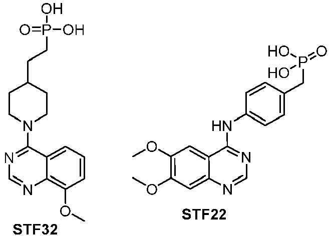

**Table 1 Inhibitory effect IC₅₀ of compounds of the present disclosure on ENPP1 kinase**

| Example | | c-GAMP (IC₅₀, nM) | Example | | c-GAMP (IC₅₀, nM) | | Example | | c-GAMP (IC₅₀, nM) | |
|---|---|---|---|---|---|---|---|---|---|---|
| | 1 | 1.2 | | 2 | | 6.7 | | 3 | | 18 |
| | 4 | 8.2 | | 5 | | 11 | | 6 | | 11 |
| | 10 | 1.1 | | 15 | | 115 | | 24 | | 1.2 |
| | 11 | 1.4 | | 12 | | 2.2 | | 13 | | 0.94 |
| | 20 | 2.1 | | 21 | | 5.0 | | 23 | | 37 |
| STF22 | | 63 | STF32 | | | 6.2 | | 58 | | 38 |

It can be seen from Table 1 that the activity of the phosphonic acid compound of the present disclosure in inhibiting an ENPP1 kinase was equivalent to that of a positive compound STF32 and significantly better than that of a compound STF22 from the literature (STF22 was derived from Cell Chemical Biology 2020 (27), 1347-1358), especially a naphthyridine phosphonic acid compound. It was reported by the literature that in-vivo metabolism of STF32 in mice was unstable, the half-life of injection administration was relatively short and T_{1/2} = 14 min. The *in-vivo* metabolic stability of the phosphonic acid compound of the present disclosure was remarkably improved and the T_{1/2} was 3-5 h. Besides, the prodrug had relatively high oral bioavailability and thus can overcome the defect that the phosphonic acid compound cannot be taken orally.

### Experimental example 2: Pharmacokinetic test experiment of mice

1. In each administration mode, 9 healthy ICR mice male, weighing 30-35 g, were randomly divided into 3 groups (M1, M2, M3) with 3 mice in each group. The tested compound was administered by gavage (50 mg/kg) and intravenous injection (1 mg/kg), respectively:
   fasting for 12 hours before the experiment and drinking water freely. Feeding uniformly 4 hours after administration.
2. Blood collection time and sample processing

Intragastric administration: 0.25 h, 0.5 h, 1.0 h, 2.0 h, 3.0 h, 4.0 h, 6.0 h, 8.0 h and 24 h after administration.

Intravenous administration: 5 min, 0.25 h, 0.5 h, 1.0 h, 2.0 h, 4.0 h, 6.0 h, 8.0 h and 24 h after administration.

Blood was collected continuously, with 3 animals collected at each time point. Plasma collection and processing: at the above set time points, 30-40 µL of venous blood was collected from the posterior venous plexus of the mouse eyeball, placed in EDTA-K2 test tube, centrifuged at 3500 rpm for 10 min, plasma was separated, and frozen at -20°C in a refrigerator.

### 3. Sample testing and data analysis

LC/MS/MS method was used to determine the concentration of compounds in mice plasma. The pharmacokinetic parameters after administration were calculated using a non-compartmental model using Phoenix 8.3 software (Pharsight, USA).

### 4. Experimental results

In the oral administration experiment of prodrug compounds 7 and 8 in mice, the plasma concentrations of the prodrug compounds 7 and 8, and an active ingredient thereof (parent drug compound 1) were respectively detected by LC-MS/MS. The results found that the plasma concentrations of the active ingredient (parent compound 1) in the mice after 5 min were 2,941 ng/mL and 2,432 ng/mL respectively, indicating that the prodrug compounds 7 and 8 were converted to the parent drug compound 1 in the mice plasma at a faster rate (within 5 min) after the oral administration of the prodrug compounds 7 and 8.

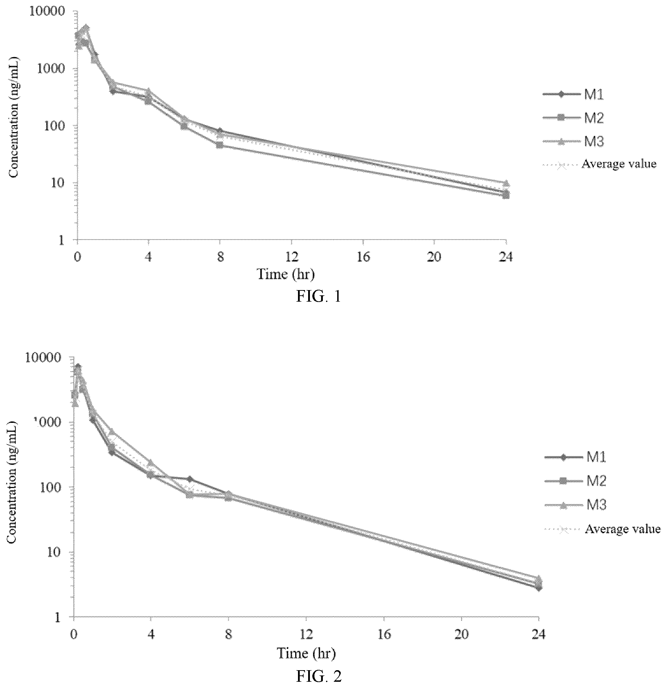

Specifically, the pharmacokinetics of the active ingredient 1 detected in the plasma of the mice after the oral administration of the prodrug compounds 7 and 8 (50 mg/kg) in the mice respectively were shown in FIGs. 1 and 2, wherein M1, M2, and M3 were the number of animal grouping respectively. It can be known from FIGs. 1 and 2, the compounds 7 and 8 and compound 1 of the present disclosure all had good pharmacokinetic characteristics. After the compounds 7 and 8 were administrated in the mice, the pharmacokinetic characteristics such as clearance, AUC and bioavailability of the compound 1 were monitored to be similar to those of the administration of the compound 1 monomer, indicating that the compounds 7 and 8 can be converted into the parent drug compound 1 in the mice and the compound 1 exerted the pharmacological activity. After the compound 1 was prepared into the prodrug compound 7 or 8, the pharmacological activity of the compound was not influenced, indicating that the compound 1 of the present disclosure had a remarkable inhibitory effect on diseases related to ENPP1 expression, such as tumors or disorders or diseases caused by infection.

**Table 2 Pharmacokinetics* of active ingredient in plasma of mice after oral administration of a phosphonate prodrug (50 mg/kg).**

| | **Compound** | | **T_{1/2} (hr)** | **AUC₀₋ₜ (ng·hr/mL)** | | **Bioavailability%** | |
|---|---|---|---|---|---|---|---|
| | 7 | | 4.7 | | 6210 | | 17 |
| | 8 | | 3.7 | | 5940 | | 13 |

| | | | | | | | |
|---|---|---|---|---|---|---|---|
| *After the oral administration of the compounds in example 7 and example 8, the active ingredient compound 1 converted by the compounds 7 and 8 in plasma was detected. | | | | | | | |

It can be seen from Table 2, after the phosphonate or amide prodrug compound of the present disclosure, such as those in example 7 and example 8, was orally taken by the mice for 5 min, only the active ingredient compound 1 thereof was detected in the plasma. Compared with the prodrug compounds 7 and 8, the parent drug compound 1 of the present disclosure showed a significant improvement in AUC₀₋ₜ. Thus, it is demonstrated that the phosphonate or amide prodrug of the present disclosure can be used for oral absorption. Since the phosphonic acid parent drug compound containing a free phosphate group had too large polarity, it had poor lipid solubility and low oral bioavailability and was not conductive for absorption in the gastrointestinal tract and the oral administration. The problem can be overcome after the phosphate group was converted into a phosphonic diester or amide derivative. For example, the ester obtained by esterifying the compound 1, such as the compounds 7 and 8, had enhanced lipid solubility and was hydrolyzed into the compound 1 *in vivo* to play a pharmacological action, and the oral bioavailability can reach 10%-17%. Compared with the compound 1, the phosphonate form improved the lipid solubility, the oral taking property and the bioavailability of the drug, reduced the side effect and had higher safety.

### Experimental example 3: Pharmacokinetic test experiment for rats

1. Six SD rats of 6-8 weeks old, male, were taken in each administration mode, and the tested compound was administered by gavage (5 mg/kg) and intravenous injection (1 mg/kg), respectively: fasting for 12 hours before the experiment and drinking water freely. Feeding uniformly 4 hours after administration.
2. Blood collection time and sample processingIntragastric administration: 0.25 h, 0.5 h, 1.0 h, 2.0 h, 3.0 h, 4.0 h, 6.0 h, 8.0 h and 24 h after administration.

Intravenous administration: 5 min, 0.25 h, 0.5 h, 1.0 h, 2.0 h, 4.0 h, 6.0 h, 8.0 h and 24 h after administration.

Plasma collection and processing: at the above set time points, 150-200 µL of blood was collected via the plexus jugularis or a suitable part, placed in EDTA-K2 test tube, centrifuged at 3500 rpm for 10 min, plasma was separated, and frozen at -20°C in a refrigerator.

### 3. Sample testing and data analysis

LC/MS/MS method was used to determine the concentration of compounds in rats plasma. The pharmacokinetic parameters after administration were calculated using a non-compartmental model using Phoenix 8.3 software (Pharsight, USA).

### 4. Experimental results

In the intravenous injection and oral administration experiment of the prodrug compound 7 in the rats, the plasma concentration of the parent drug compound 1 thereof (active ingredient) was detected by LC-MS/MS. The results were shown in Table 3.

### Experimental Example 4: Pharmacokinetic test experiment for dogs

1. In each administration mode, 6 beagle dogs of 0.5-1.5 old were administered by gavage (2 mg/kg) and intravenous injection (1 mg/kg), respectively: fasting for 12 hours before the experiment and drinking water freely. Feeding uniformly 4 hours after administration.
2. Blood collection time and sample processing

Intravenous and Intragastric administration: 0.083, 0.25, 0.5, 1, 2, 4, 6, 8 and 24 h after administration. Blood was collected continuously, with 3 animals collected at each time point. Plasma collection and processing: at the above set time points, 1,000 µL of blood was collected via the plexus jugularis or a suitable part, placed in EDTA-K2 test tube, centrifuged at 3500 rpm for 10 min, plasma was separated, and frozen at -20°C in a refrigerator.

### 3. Sample testing and data analysis

LC/MS/MS method was used to determine the concentration of compounds in the beagle dogs plasma. The pharmacokinetic parameters after administration were calculated using a non-compartmental model using Phoenix 8.3 software (Pharsight, USA).

### 4. Experimental results

In the intravenous injection and oral administration experiment of the prodrug compound 7 in the beagle dogs, the plasma concentration of the parent drug compound 1 thereof (active ingredient) was detected by LC-MS/MS. The results were shown in Table 3.

**Table 3 Pharmacokinetics* of phosphonate prodrug compound 7 in rats and dogs**

| **Pharmacokinetic parameters** | **Pharmacokinetics of rats** | | **Pharmacokinetics of dogs** | |
|---|---|---|---|---|
| Clearance CL (ml/min-kg) i.v. | | 3.36 | | 3.40 |
| Apparent volume of distribution Vss (ml/kg) i.v. | | 278 | | 217 |
| Half-life T_{1/2} (h) i.v. | | 4.48 | | 1.2 |
| Area under the curve AUC₀₋ₜ (ng/ml-h) i.v. | | 5,014 | | 4,890 |
| Area under the curve AUC₀₋ₜ (ng/ml-h) p.o. | | 5,984 | | 741 |
| Plasma concentration Cₘₐₓ (ng/ml) p.o. | | 4,420 | | 488 |
| Time to peak Tₘₐₓ (h) p.o. | | 0.25 | | 0.33 |
| Bioavailability Oral BA (%F) | | 40 | | 21 |

| | | | | |
|---|---|---|---|---|
| *After the administration of the compound in example 7, the active ingredient compound '1 converted by the compound 7 in the plasma was detected. The rats were orally administrated with the prodrug compound 7 (5 mg/kg) and intravenously injected with the active compound 1 (1 mg/kg). The beagle dogs were orally administrated with the prodrug compound 7 (2 mg/kg) and intravenously injected with the active compound 1(1 mg/kg). | | | | |

The results of the pharmacological test in the SD rats and beagle dogs showed that the intravenous injection of the active compound 1 had the lower clearance and longer half-life *in vivo.* The orally administrated prodrug compound 7 can be rapidly metabolized to the active compound 1 *in vivo* and had the relatively high oral bioavailability.

In conclusion, although the phosphonate or amide prodrug compound disclosed by the disclosure did not have the biological activity, it can be changed into an active phosphonic acid compound through *in-vivo* metabolism after IV or PO administration. The phosphonic acid parent drug compound had the strong inhibitory activity of ENPP1, but was not beneficial to oral administration due to high polarity. However, the phosphonate or amide prodrug compound can increase the drug solubility, improve the administration route, also increase the drug stability and improve the drug oral bioavailability.

The phosphonate or amide compound (I-A) of the present disclosure can be used as a prodrug of the phosphonate compound (I). The compound of formula (I) was shown to be an ENPP1 inhibitor, and therefore, the compound of the present disclosure can be used in the treatment of ENPP 1-related diseases and used as a therapeutic agent for the ENPP 1-related diseases.

## Claims

1. A compound shown in formula (I) or formula (I-A), or a pharmaceutical salt, a stereoisomer, a geometrical isomer, a tautomer, a solvate or a hydrate thereof: 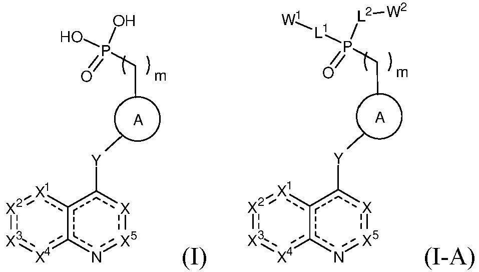 wherein,
- - - -: is a single bond or a double bond;
X¹ is N, CR¹ or a bond;
X² is N, NR² or CR²;
X³ is N, NR³, -C(O) or CR³;
X⁴ is N, NR⁴, -C(O) or CR⁴;
X⁵ is N or CR⁵;
X is N or CR⁰;
provided that X and X⁵ are not N at the same time and at least one of X¹, X², X³ and X⁴ is independently N or NRⁿ; and n = 2, 3 or 4;
R⁰, R¹, R³ and R⁵ are each independently selected from hydrogen, halogen, CN, OR^{a}, NO₂, NR^{b}R^{c}, C₁-C₆ alkyl and C₁-C₆ haloalkyl;
R² and R⁴ are each independently selected from hydrogen, halogen, CN, OR^{a}, NO₂, NR^{b}R^{c} and C₁-C₆ alkyl, wherein the alkyl is optionally substituted with halogen;
Y is O, S, NR⁶ or CR⁷R⁸, wherein R⁶, R⁷ and R⁸ are each independently selected from hydrogen and C₁-C₄ alkyl;
m is 1, 2 or 3;
ring A is selected from C₆-C₁₀ aryl and 5- to 10-membered heteroaryl, wherein ring A is optionally substituted one or more times with substituents independently selected from halogen, CN, OH, NO₂, NR^{b}R^{c}, C₁-C₄ alkyl, C₁-C₄ alkoxy and C₁-C₃ haloalkyl,
wherein R^{a}, R^{b} and R^{c} are each independently selected from hydrogen and C₁-C₄ alkyl optionally substituted with halogen;
L¹ and L² are each independently NH or O, preferably, both L¹ and L² are O; and
W¹ and W² are each independently selected from C₆-C₁₀ aryl unsubstituted or substituted with halogen, -CH₂OC(O)R^{d}, -CH₂OC(O)OR^{d}, -CH₂C(CH₃)₂C(O)OR^{d}, -CH(CH₃)C(O)OR^{d} and -CH₂C(CH₃)₂C(O)OR^{d}, wherein R^{d} is C₂-C₆ alkyl.

2. The compound shown in formula (I) or formula (I-A), or the pharmaceutical salt, the stereoisomer, the geometrical isomer, the tautomer, the solvate or the hydrate thereof according to claim 1, wherein 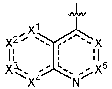 is selected from 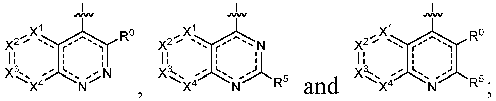
preferably, 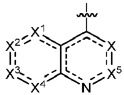
is selected from 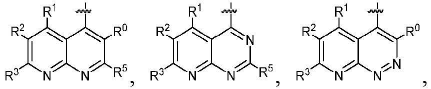 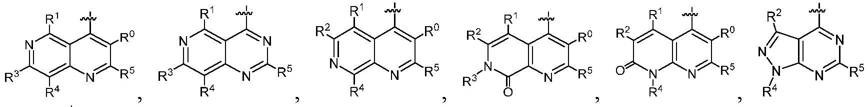
and 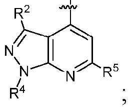
more preferably, 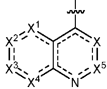
is selected from 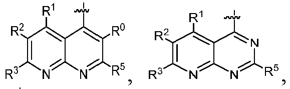 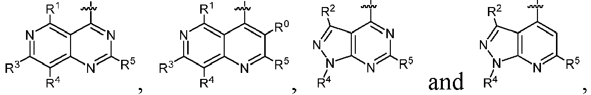
further preferably, 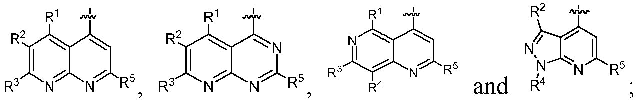 and/or 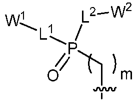
is selected from 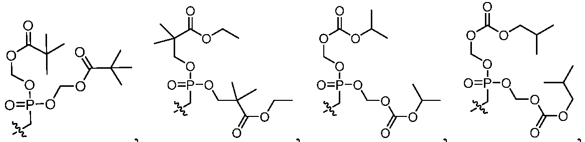 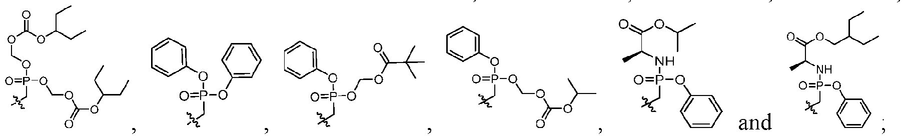
preferably, 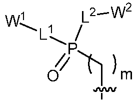
is selected from 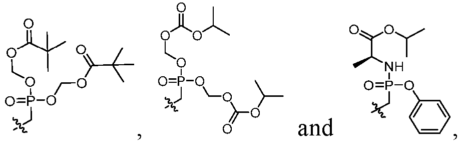 wherein each substituent is as defined in claim 1.

3. The compound shown in formula (I) or formula (I-A), or the pharmaceutical salt, the stereoisomer, the geometrical isomer, the tautomer, the solvate or the hydrate thereof according to claim 1 or 2, wherein
the compound shown in formula (I) or formula (I-A) is respectively a compound shown in formula (II) or formula (II-A): 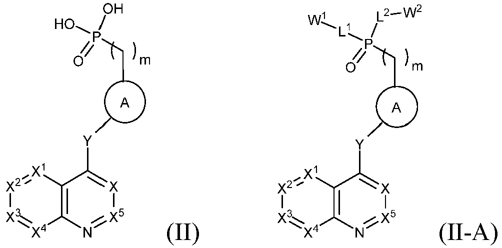
wherein in formula (II) or formula (II-A),
X¹ is N or CR¹;
X² is N or CR²;
X³ is N, NR³, -C(O) or CR³;
X⁴ is N, NR⁴, -C(O) or CR⁴;
X⁵ is N or CR⁵; X is N or CR⁰;
provided that X and X⁵ are not N at the same time and at least one of X¹, X², X³ and X⁴ is independently N or NRⁿ, and n = 3 or 4;
R⁰ and R⁵ are each independently selected from hydrogen, halogen, CN, OH, NO₂, NH₂, C₁-C₆ alkyl and C₁-C₄ alkoxy, wherein the alkyl is each optionally substituted with 0-3 halogens;
R¹ and R³ are each independently selected from hydrogen, halogen, CN, OH, NO₂, NH₂ and C₁-C₄ alkoxy;
R² and R⁴ are each independently selected from hydrogen, halogen, CN, OH, NO₂, NH₂ and C₁-C₄ alkyl;
Y is O, S, NR⁶ or CR⁷R⁸, wherein R⁶, R⁷ and R⁸ are each independently selected from hydrogen and C₁-C₄ alkyl; and
ring A, m, L¹, L², W¹ and W² are as defined in claim 1.

4. The compound shown in formula (I) or formula (I-A), or the pharmaceutical salt, the stereoisomer, the geometrical isomer, the tautomer, the solvate or the hydrate thereof according to claim 1 or 2, wherein
the compound shown in formula (I) or formula (I-A) is respectively a compound shown in formula (III) or formula (III-A): 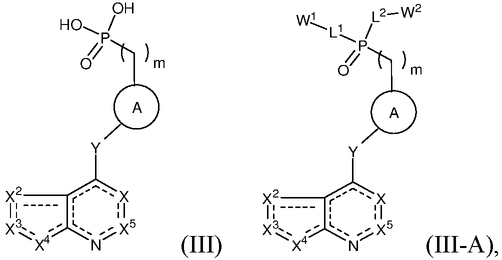
wherein in formula (III) or formula (III-A),
- - - -: is a single bond or a double bond;
X² is N, NR² or CR²; X³ is N, NR³ or CR³; X⁴ is N, NR⁴ or CR⁴;
X⁵ is N or CR⁵; X is N or CR⁰;
provided that X and X⁵ are not N at the same time and at least one of X², X³ and X⁴ is independently N or NRⁿ; and n = 2, 3 or 4;
R⁰ and R⁵ are each independently selected from hydrogen, halogen, CN, C₁-C₆ alkyl, C₁-C₄ alkoxy and C₁-C₆ haloalkyl;
R², R³ and R⁴ are each independently selected from hydrogen, halogen, CN, OH, NO₂, NH₂, C₁-C₄ alkyl and C₁-C₃ haloalkyl;
Y is O, S, NR⁶ or CR⁷R⁸, wherein R⁶, R⁷ and R⁸ are each independently selected from hydrogen and C₁-C₄ alkyl; and
ring A, m, L¹, L², W¹ and W² are as defined in claim 1.

5. The compound shown in formula (I) or formula (I-A), or the pharmaceutical salt, the stereoisomer, the geometrical isomer, the tautomer, the solvate or the hydrate thereof according to claim 1 or 2, wherein
the compound shown in formula (I) or formula (I-A) is respectively a compound shown in formula (IV) or formula (IV-A): 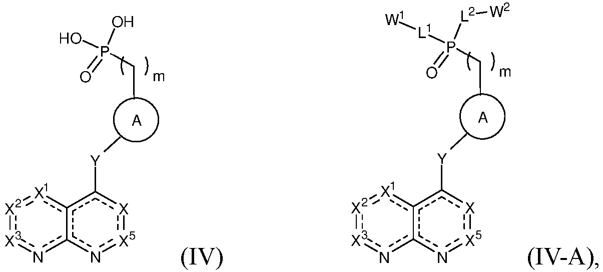
wherein in formula (IV) or formula (IV-A),
- - - -: is a single bond or a double bond;
X¹ is N, CR¹ or a bond; X² is N, NR² or CR²; X³ is N, NR³ or CR³;
X⁵ is N or CR⁵; X is N or CR⁰; provided that X and X⁵ are not N at the same time;
R⁰ and R⁵ are each independently selected from hydrogen, halogen, CN, C₁-C₆ alkyl, C₁-C₄ alkoxy and C₁-C₆ haloalkyl;
R¹ and R³ are each independently selected from hydrogen, halogen, CN, OH, NO₂, NH₂, C₁-C₄ alkyl and C₁-C₄ alkoxy;
R² is selected from hydrogen, halogen, OH and C₁-C₃ alkyl;
Y is O, S, NR⁶ or CR⁷R⁸, wherein R⁶, R⁷ and R⁸ are each independently selected from hydrogen and C₁-C₄ alkyl; and
ring A, m, L¹, L², W¹ and W² are as defined in claim 1.

6. The compound shown in formula (I) or formula (I-A), or the pharmaceutical salt, the stereoisomer, the geometrical isomer, the tautomer, the solvate or the hydrate thereof according to claim 1 or 2, wherein
the compound shown in formula (I) or formula (I-A) is respectively a compound shown in formula (V) or formula (V-A): 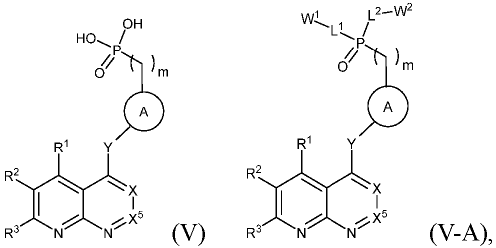
wherein in formula (V) or formula (V-A),
X⁵ is N or CR⁵; X is N or CR⁰; and X and X⁵ are not N at the same time;
R⁰ and R⁵ are each independently selected from hydrogen, halogen, CN and C₁-C₆ alkyl; R¹, R² and R³ are each independently selected from hydrogen, halogen, CN, OH, NO₂, NH₂, C₁-C₄ alkyl and C₁-C₄ alkoxy; and
Y is O, S, NR⁶ or CR⁷R⁸, wherein R⁶, R⁷ and R⁸ are each independently selected from hydrogen and C₁-C₄ alkyl; and
ring A, L¹, L², m, W¹ and W² are as defined in claim 1;
or
the compound shown in formula (I) or formula (I-A) is respectively a compound shown in formula (VI) or formula (VI-A): 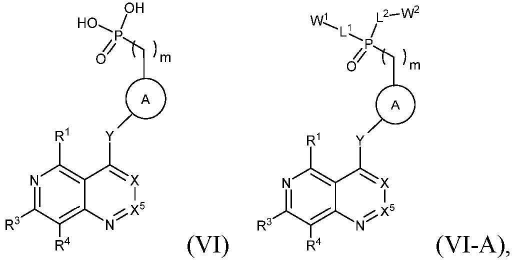
wherein in formula (VI) or formula (VI-A),
X⁵ is N or CR⁵; X is N or CR⁰; X and X⁵ are not N at the same time;
R⁰ and R⁵ are each independently selected from hydrogen, halogen, CN and C₁-C₆ alkyl;
R¹ and R³ are each independently selected from hydrogen, halogen, CN, OH, NO₂, NH₂ and C₁-C₄ alkoxy;
R⁴ is selected from hydrogen, halogen and C₁-C₄ alkyl;
Y is O, S, NR⁶ or CR⁷R⁸, wherein R⁶, R⁷ and R⁸ are each independently selected from hydrogen and C₁-C₄ alkyl;
ring A, L¹, L², m, W¹ and W² are as defined in claim 1;
or
the compound shown in formula (I) or formula (I-A) is respectively a compound shown in formula (VII) or formula (VII-A): 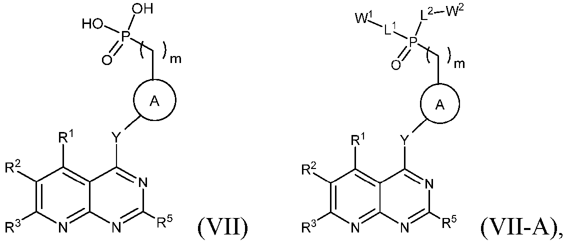
wherein in formula (VII) or formula (VII-A),
Y is O, S, NR⁶ or CR⁷R⁸, wherein R⁶, R⁷ and R⁸ are each independently selected from hydrogen and C₁-C₄ alkyl;
R⁵ is selected from hydrogen, halogen, CN and C₁-C₆ alkyl;
R¹ and R³ are each independently selected from hydrogen, halogen, CN, OH, NO₂, NH₂ and C₁-C₄ alkoxy;
R² is selected from hydrogen, halogen and C₁-C₄ alkyl;
ring A, L¹, L², m, W¹ and W² are as defined in claim 1;
or
the compound shown in formula (I) or formula (I-A) is respectively a compound shown in formula (VIII) or formula (VIII-A): 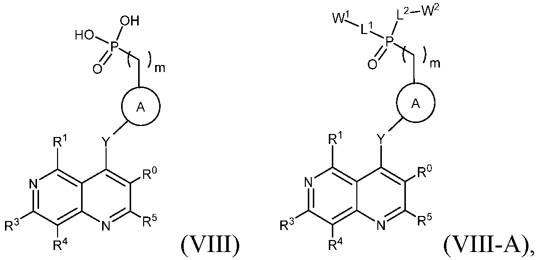
wherein in formula (VIII) or formula (VIII-A),
Y is O, S, NR⁶ or CR⁷R⁸, wherein R⁶, R⁷ and R⁸ are each independently selected from hydrogen and C₁-C₄ alkyl;
R⁰ and R⁵ are each independently selected from hydrogen, halogen, CN and C₁-C₆ alkyl; R¹ and R³ are each independently selected from hydrogen, halogen, CN, OH, NO₂, NH₂ and C₁-C₄ alkoxy;
R² is selected from hydrogen, halogen and C₁-C₄ alkyl;
ring A, L¹, L², m, W¹ and W² are as defined in claim 1;
or
the compound shown in formula (I) or formula (I-A) is respectively a compound shown in formula (IX) or formula (IX-A): 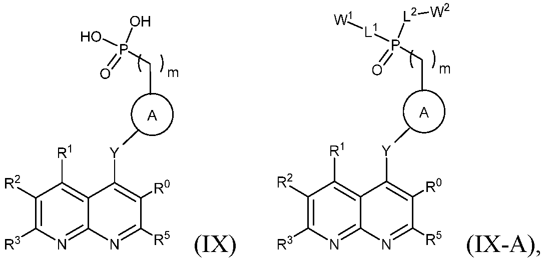
wherein in formula (IX) or formula (IX-A),
Y is O, S, NR⁶ or CR⁷R⁸, and R⁶, R⁷ and R⁸ are each independently selected from hydrogen and C₁-C₄ alkyl;
R⁰ and R⁵ are each independently selected from hydrogen, halogen, CN and C₁-C₆ alkyl; R¹ and R³ are each independently selected from hydrogen, halogen, CN, OH, NO₂, NH₂, C₁-C₄ alkyl and C₁-C₄ alkoxy;
R² is selected from hydrogen, halogen and C₁-C₄ alkyl; and
ring A, L¹, L², m, W¹ and W² are as defined in claim 1.

7. The compound shown in formula (I) or formula (I-A), or the pharmaceutical salt, the stereoisomer, the geometrical isomer, the tautomer, the solvate or the hydrate thereof according to claim 1, wherein
the compound of formula (I) is selected from the following compounds:
| Compo -und No. | Structure | Compo -und No. | Structure | Compo -und No. | Structure |
|---|---|---|---|---|---|
| 1 | 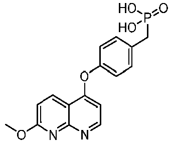 | 2 | 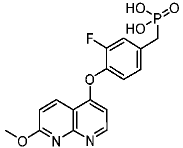 | 5 | 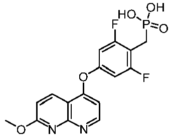 |
| 3 | 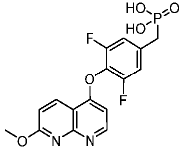 | 4 | 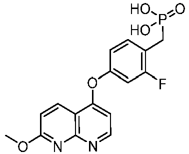 | 6 | 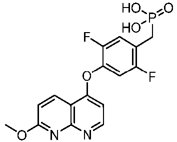 |
| 10 | 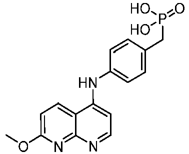 | 11 | 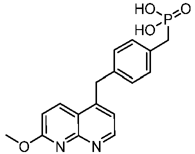 | 14 | 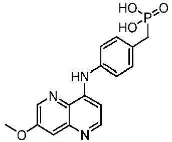 |
| 12 | 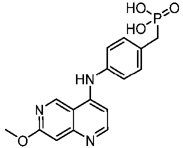 | 13 | 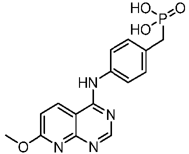 | 15 | 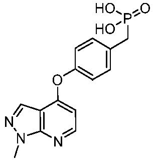 |
| 20 | 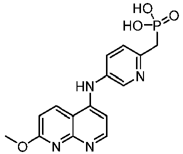 | 21 | 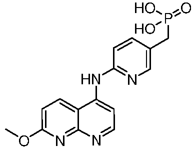 | 24 | 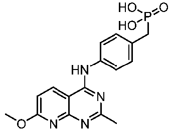 |
| 22 | 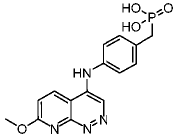 | 23 | 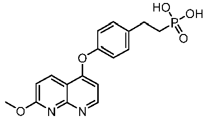 | 25 | 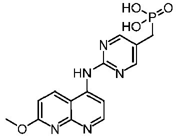 |
| 26 | 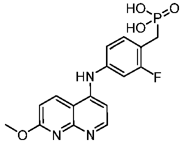 | 27 | 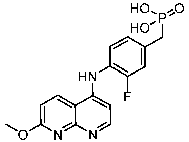 | 30 | 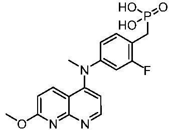 |
| 28 | 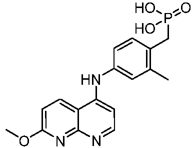 | 29 | 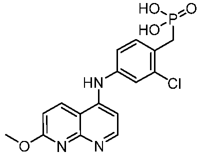 | 31 | 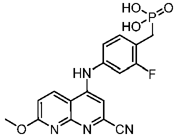 |
| 32 | 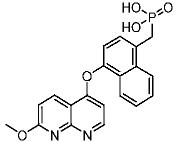 | 33 | 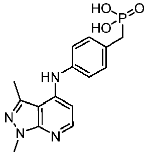 | 41 | 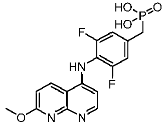 |
| 34 | 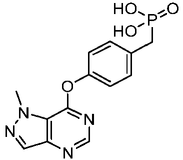 | 40 | 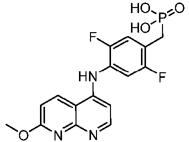 | 42 | 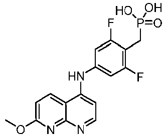 |
| 43 | 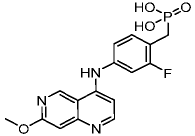 | 44 | 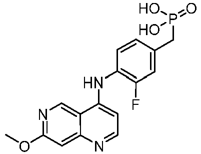 | 47 | 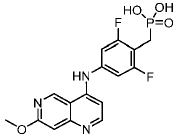 |
| 45 | 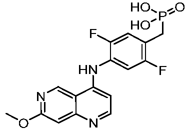 | 46 | 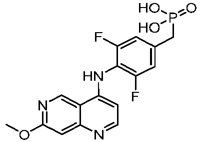 | 58 | 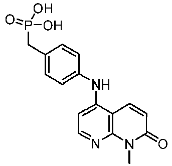 |
| 59 | 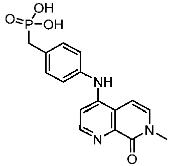 | 60 | 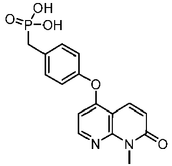 | 61 | 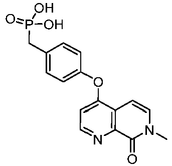 |
and
the compound of formula (I-A) is selected from the following compounds:
| Compo -und No. | Structure | Compo -und No. | Structure | Compo -und No. | Structure |
|---|---|---|---|---|---|
| 7 | 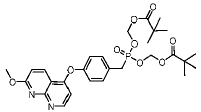 | 8 | 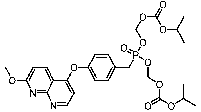 | 17 | 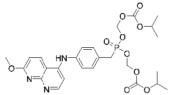 |
| 9 | 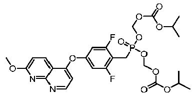 | 16 | 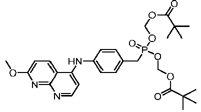 | 18 | 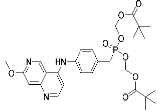 |
| 19 | 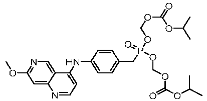 | 35 | 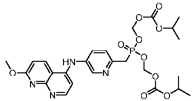 | 38 | 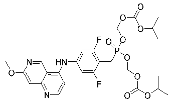 |
| 36 | 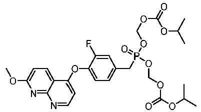 | 37 | 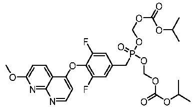 | 39 | 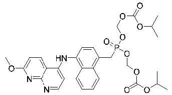 |
| 48 | 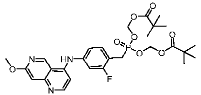 | 49 | 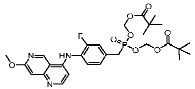 | 52 | 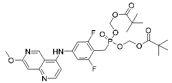 |
| 50 | 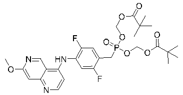 | 51 | 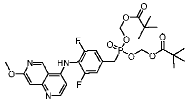 | 53 | 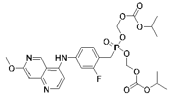 |
| 54 | 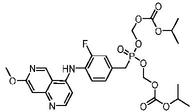 | 55 | 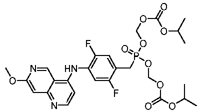 | 62 | 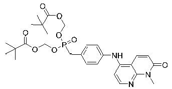 |
| 56 | 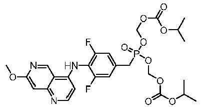 | 57 | 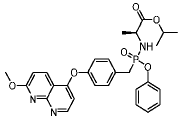 | 63 | 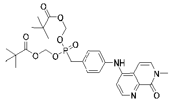 |
| 64 | 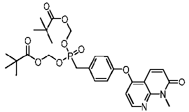 | 65 | 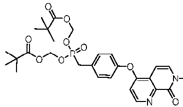 | 68 | 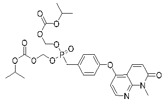 |
| 66 | 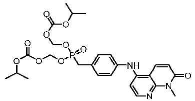 | 67 | 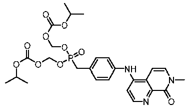 | 69 | 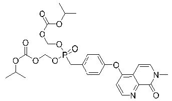 |

8. A pharmaceutical composition, comprising one or more selected from the compound of formula (I) or formula (I-A), the pharmaceutically acceptable salt, the stereoisomer, the geometrical isomer, the tautomer, the solvate or the hydrate thereof according to any one of the claims 1-7 used as an active ingredient, and optionally pharmaceutically acceptable carrier, diluent or excipient.

9. Use of the compound of formula (I) or formula (I-A), the pharmaceutically acceptable salt, the stereoisomer, the geometrical isomer, the tautomer, the solvate or the hydrate thereof according to any one of the claims 1-7, or the pharmaceutical composition of claim 8 in preparing a drug for preventing, treating or alleviating of ENPP1-mediated diseases or disorders in patients.

10. A method for treating or preventing ENPP1-mediated diseases or disorders, including administering a therapeutically effective amount of one or more selected from the compound of formula (I) or formula (I-A), the pharmaceutically acceptable salt, the stereoisomer, the geometrical isomer, the tautomer, the solvate or the hydrate thereof according to any one of the claims 1-7, or the pharmaceutical composition of claim 8 to an individual in need thereof.

11. The use of claim 9 or the method of claim 10, wherein,
the ENPP1-mediated diseases or disorders are cancer or infectious diseases or disorders;
further preferably, the cancer is selected from recurrent or refractory cancer, metastatic cancer, for example, selected from solid tumor of breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer, or selected from hematopoietic malignancies, preferably, leukemia, lymphoma and myeloma.;
further preferably, the infectious diseases or disorders are selected from herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.
